# EUROPEAN PATENT APPLICATION

(11) **EP 4 289 849 A1**
(43) Date of publication of application: **13.12.2023**
(21) Application number: 22749827.6
(22) Date of filing: 04.02.2022
(51) Int. Cl.: C07D 487/06, C09K 11/06, H01L 51/50

(54) **COMPOUND, LIGHT-EMITTING MATERIAL, AND ORGANIC LIGHT-EMITTING ELEMENT**

(30) Priority: 04.02.2021 WO PCT/JP2021/004053; 28.07.2021 JP 2021123021
(71) Applicant: Kyulux, Inc., Fukuoka-shi, Fukuoka 819-0388 (JP)
(72) Inventor: BALIJAPALLI Umamahesh, Fukuoka-shi, Fukuoka 819-0388 (JP); SUZUKI Yoshitake, Fukuoka-shi, Fukuoka 819-0388 (JP); YAMANE Yu, Fukuoka-shi, Fukuoka 819-0388 (JP); IBRAYIM Saidalimu, Fukuoka-shi, Fukuoka 819-0388 (JP); YAMASHITA Masataka, Fukuoka-shi, Fukuoka 819-0388 (JP); CHOI Shinhyung, Fukuoka-shi, Fukuoka 819-0388 (JP); HIGA Takuya, Fukuoka-shi, Fukuoka 819-0388 (JP)
(74) Representative: Zimmermann & Partner Patentanwälte mbB
(86) International application number: PCT/JP2022/004517
(87) International publication number: WO 2022/168956

(57) **Abstract**

A compound represented by the following general formula has excellent light emission characteristics. Ar¹ represents a benzene ring, a naphthalene ring, a phenanthrene ring, etc.; D represents SH-indolo[3,2,1-de]phenazin-5-yl group, etc.; A represents a cyano group, a phenyl group, a pyrimidyl group, a triazyl group, etc.; m is 1 or 2;, n is 0 to 2; R¹ to R⁴ each represent H, an aryl group, a cyano group, etc.

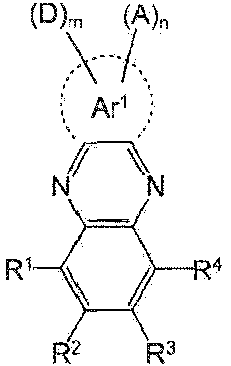

## Description

### Technical Field

The present invention relates to a compound having good emission characteristics. Also, the present invention relates to a light-emitting material and an organic light-emitting device using the compound.

### Background Art

An organic light-emitting device is a light-emitting device using an organic material, which can be produced by coating and which does not use a rare element, and therefore, attention has recently been paid to the organic light-emitting device. Above all, an organic electroluminescent device (organic EL device) emits self-luminous light and does not require a backlight, and is therefore advantageous in that it can be a lightweight and flexible device. In addition, it has features of high responsiveness and high visibility, and is expected as a next generation light source. Consequently, studies relating to materials useful for organic light-emitting devices such as typically organic electroluminescent devices have been promoted actively. In particular, studies relating to light-emitting materials have been carried out actively (for example, NPL 1).

### Citation List

### Non-Patent Literature

NPL 1: Chem. Soc. Rev., 2017, 46, 915

### Summary of Invention

### Technical Problem

On the other hand, there is still room for improvement in the emission characteristics of organic light-emitting devices, and further enhancement of emission properties is desired.

Accordingly, the present inventor have promoted assiduous studies for the purpose of developing a novel compound capable of contributing toward improvement of emission characteristics of organic light-emitting devices.

### Solution to Problem

As a result of assiduous studies, the present inventors have found that a compound having a specific skeleton with groups each having a characteristic structure bonding to the skeleton is a compound useful for light-emitting devices. The present invention has been proposed on the basis of such findings, and has the following constitution.
[1] A compound represented by the following general formula (1). 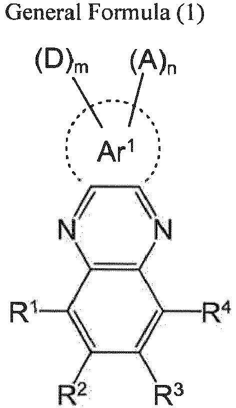 In the general formula (1), Ar¹ represents a cyclic structure, and represents a benzene ring, a naphthalene ring, an anthracene ring, or a phenanthrene ring. D represents a group represented by the following general formula (2). A represents one or a combination of two or more groups selected from the group consisting of a cyano group, a phenyl group, a pyrimidyl group, a triazyl group and an alkyl group (except for a substituted alkyl group). m represents 1 or 2, n represents 0, 1 or 2. When m is 2, two D's can be the same or different. When n is 2, two A's can be the same or different. R¹ to R⁴ each independently represent a hydrogen atom, a deuterium atom, or one or a combination of two or more groups selected from the group consisting of an alkyl group, an aryl group, a heteroaryl group and a cyano group. R¹ and R², and R³ and R⁴ each can bond to each other to form a cyclic structure selected from the group consisting of a benzene ring, a naphthalene ring and a pyridine ring, and the formed cyclic structure can be substituted with one or a combination of two or more groups selected from the group consisting of an alkyl group, an aryl group, a heteroaryl group and a cyano group. 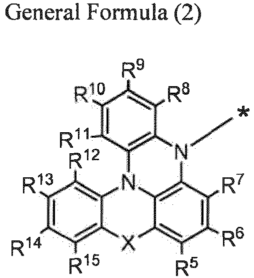 In the general formula (2), R⁵ to R¹⁵ each independently represent a hydrogen atom, a deuterium atom or a substituent. R⁵ and R⁶, R⁶ and R⁷, R⁸ and R⁹, R⁹ and R¹⁰, R¹⁰ and R¹¹, R¹¹ and R¹², R¹² and R¹³, R¹³ and R¹⁴, and R¹⁴ and R¹⁵ each can bond to each other to form a cyclic structure. X represents a single bond, an oxygen atom or a sulfur atom. * indicates a bonding position.
[2] The compound according to [1], represented by the following general formula (3). 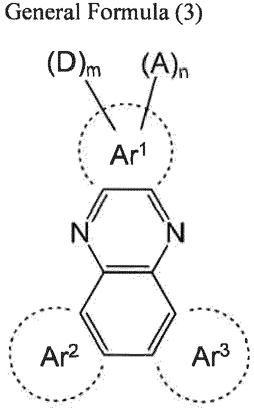 In the general formula (3), Ar¹ represents a cyclic structure, and represents a benzene ring, a naphthalene ring, an anthracene ring, or a phenanthrene ring. D represents a group represented by the above-mentioned general formula (2). A represents one or a combination of two or more groups selected from the group consisting of a cyano group, a phenyl group, a pyrimidyl group, a triazyl group and an alkyl group (except for a substituted alkyl group). m represents 1 or 2, n represents 0, 1 or 2. When m is 2, two D's can be the same or different. When n is 2, two A's can be the same or different. Ar² and Ar³ each can form a cyclic structure selected from the group consisting of a benzene ring, a naphthalene ring and a pyridine ring, and the formed cyclic structure can be substituted with one or a combination of two or more groups selected from the group consisting of an alkyl group, an aryl group, a heteroaryl group and a cyano group.
[3] The compound according to [1], having a skeleton of any of the following: 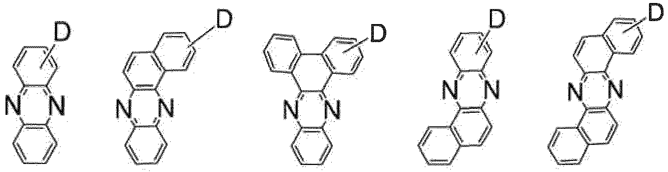 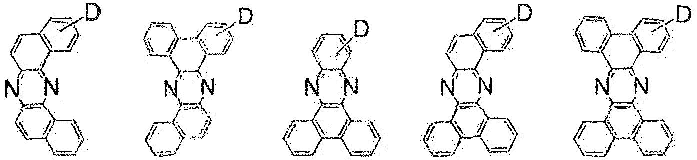 The above skeletons each can have a substituent within the range of the general formula (1), but any further ring is not condensed with the skeletons.
[4] The compound according to [1], represented by any of the following general formulae (4a) to (4f). 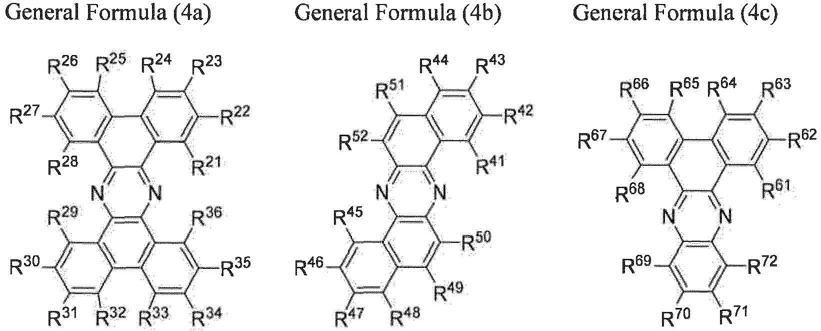 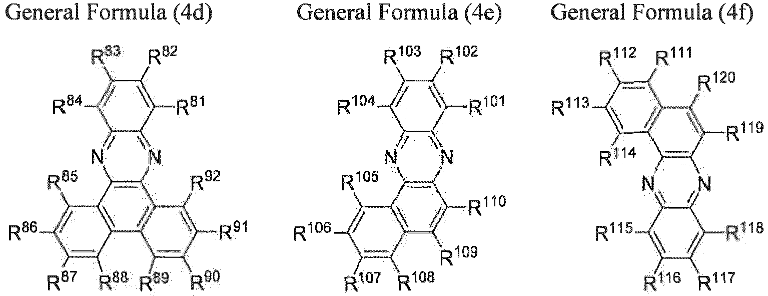 In the general formulae (4a) to (4f), R²¹ to R²⁸, R⁴¹ to R⁴⁴, R⁵¹, R⁵², R⁶¹ to R⁶⁸, R⁸¹ to R⁸⁴, R¹⁰¹ to R¹⁰⁴, R¹¹¹ to R¹¹⁴, R¹¹⁹, and R¹²⁰ each independently represent a hydrogen atom, a deuterium atom, D or A. Provided that 1 or 2 of R²¹ to R²⁸ are D, and 0 to 2 are A; 1 or 2 of R⁴¹ to R⁴⁴, R⁵¹ and R⁵² are D, and 0 to 2 are A; 1 or 2 of R⁶¹ to R⁶⁸ are D, and 0 to 2 are A; 1 or 2 of R⁸¹ to R⁸⁴ are D, and 0 to 2 are A; 1 or 2 of R¹⁰¹ to R¹⁰⁴ are D, and 0 to 2 are A; 1 or 2 of R¹¹¹ to R¹¹⁴, R¹¹⁹ and R¹²⁰ are D, and 0 to 2 are A. R²⁹ to R³⁶, R⁴⁵ to R⁵⁰, R⁶⁹ to R⁷², R⁸⁵ to R⁹², R¹⁰⁵ to R¹¹⁰, and R¹¹⁵ to R¹¹⁸ each independently represent a hydrogen atom, a deuterium atom, or one or a combination of two or more groups selected from the group consisting of an alkyl group, an aryl group and a cyano group.
[5] The compound according to any one of [1] to [4], wherein n is 0.
[6] A light-emitting material containing the compound of any one of [1] to [5].
[7] A film containing the compound of any one of [1] to [5].
[8] An organic semiconductor device containing the compound of any one of [1] to [5].
[9] An organic light-emitting device containing the compound of any one of [1] to [5].
[10] The organic light-emitting device according to [9], wherein the device has a layer containing the compound and the layer also contains a host material.
[11] The organic light-emitting device according to [10], wherein the layer containing the compound also contains a delayed fluorescent material in addition to the host material, and the lowest excited singlet energy of the delayed fluorescent material is lower than that of the host material and higher than that of the compound.
[12] The organic light-emitting device according to [9], wherein the device has a layer containing the compound, and the layer also contains a light-emitting material having a structure different from that of the compound.
[13] The organic light-emitting device according to any one of [9] to [11], wherein, among the materials contained in the device, the amount of light emission from the compound is the maximum.
[14] The organic light-emitting device according to [12], wherein the amount of light emission from the light-emitting material is larger than the amount of light emission from the compound.
[15] The organic light-emitting device according to any one of [9] to [14], which emits delayed fluorescence.

### Advantageous Effects of Invention

The compound of the present invention is a compound useful for light-emitting devices. The compound of the present invention can be used as a light-emitting material, and using the compound of the present invention, an organic light-emitting device can be produced. The organic light-emitting device using the compound of the present invention is excellent in at least one or more characteristics of light emission efficiency (especially light emission efficiency at high concentrations), device durability and color purity improvement.

### Brief Description of Drawing

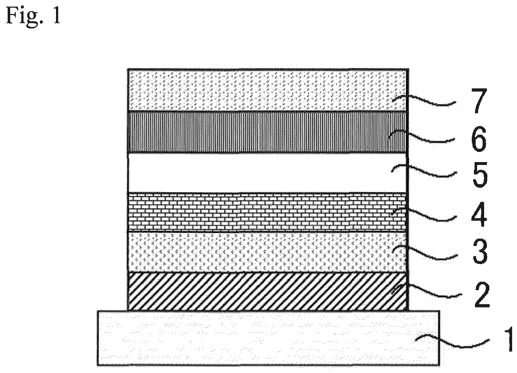

[Fig. 1] This is a schematic cross-sectional view showing an example of a layer configuration of an organic electroluminescent device.

### Description of Embodiments

The contents of the invention will be described in detail below. The constitutional elements may be described below with reference to representative embodiments and specific examples of the invention, but the invention is not limited to the embodiments and the examples. In the description herein, a numerical range expressed as "to" means a range that includes the numerical values described before and after "to" as the upper limit and the lower limit. A part or all of hydrogen atoms existing in the molecule of the compound for use in the present invention can be substituted with deuterium atoms (²H, deuterium D). In the chemical structural formulae in the description herein, the hydrogen atom is expressed as H, or the expression thereof is omitted. For example, when expression of the atoms bonding to the ring skeleton-constituting carbon atoms of a benzene ring is omitted, H is considered to bond to the ring skeleton-constituting carbon atom at the site having the omitted expression. In the chemical structural formulae in the present description, a deuterium atom is expressed as D. In the present specification, the term "substituent" means an atom or an atomic group except a hydrogen atom and a deuterium atom. On the other hand, the term "substituted or unsubstituted" means that a hydrogen atom can be substituted with a deuterium atom or a substituent.

### [Compound Represented by General Formula (1)]

The compound of the present invention is a compound represented by the following general formula (1).

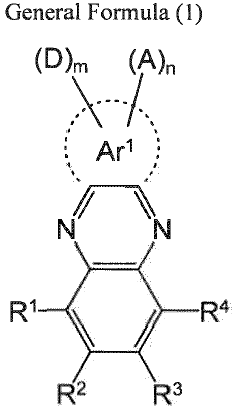

In the general formula (1), Ar¹ represents a cyclic structure, and represents a benzene ring, a naphthalene ring, an anthracene ring, or a phenanthrene ring. For example, when Ar¹ represents a benzene ring, the benzene ring is condensed with the pyrazine ring to be a quinoxaline structure. When Ar¹ represents a naphthalene ring, any of a 1,2-naphtho ring or a 2,3-naphtho ring can be condensed with the pyrazine ring. When a 1,2-naphtho ring is condensed with the pyrazine ring, the 1-positioned and 2-positioned carbon atoms of the naphthalene ring covalently bond to the 2-positioned and 3-positioned carbon atoms, respectively, constituting the pyrazine ring. When Ar¹ represents an anthracene ring, a 2,3-anthracene ring is condensed with the pyrazine ring. When Ar¹ represents a phenanthrene ring, any of a 1,2-phenanthrene ring, a 2,3-phenanthrene ring, a 3,4-phenanthrene ring or a 9,10-phenanthrene ring can be condensed with the pyrazine ring. In a preferred embodiment of the present invention, any of a benzene ring, a 2,3-naphtho ring, or a 9,10-phenanthrene ring is condensed with the pyrazine ring. In a more preferred embodiment of the present invention, any of a 2,3-naphtho ring or a 9,10-phenanthrene ring is condensed with the pyrazine ring. For example, a 2,3-naphtho ring can be condensed, or a 9,10-phenanthrene ring can be condensed.

In the cyclic structure that Ar¹ represents, m D's and n A's bond to the ring skeleton as substituents. When Ar¹ represents a naphthalene ring, an anthracene ring or a phenanthrene ring, D and A can bond to any benzene ring constituting these rings. m D's and n A's can bond to any one benzene ring alone, and neither D nor A cannot bond to the other benzene rings. Or a part of m D's and n A's can bond to one benzene ring, and the rest thereof can bond to the other one benzene ring. In one preferred embodiment of the present invention, n is 0 and m D's bond to one benzene ring alone. In another preferred embodiment of the present invention, n is 0, and a part of m D's bond to one benzene ring and the rest thereof bond to the other one benzene ring. When Ar¹ represents a naphthalene ring, an anthracene ring or a phenanthrene ring, in one preferred embodiment of the present invention, neither D nor A bonds to the benzene ring directly condensed with the pyrazine ring, and m D's and n A's bond to only the remaining benzene ring (that is, the benzene ring not directly condensed with the pyrazine ring). When Ar¹ represents a naphthalene ring, an anthracene ring or a phenanthrene ring, in one preferred embodiment of the present invention, n is 0, and D does not bond to the benzene ring directly condensed with the pyrazine ring, and m D's bond to only the remaining benzene ring (that is, the benzene ring not directly condensed with the pyrazine ring).

In the general formula (1), m is 1 or 2, and n is 0, 1 or 2. When m is 2, two D's can be the same or different. Two D's can bond to the same benzene ring, or to different benzene rings. When n is 2, two A's can be the same or different. Two A's can bond to the same benzene ring, or to different benzene rings. In one preferred embodiment of the present invention, n is 0. For example, m is 1 and n is 0. For example, m is 2 and n is 0. When Ar¹ represents a naphthalene ring, an anthracene ring or a phenanthrene ring, and n is 1 or 2, in one embodiment of the present invention, A does not bond to the benzene ring to which D bonds, and D does not bond to the benzene ring to which A bonds.

In the general formula (1), D represents a group represented by the following general formula (2).

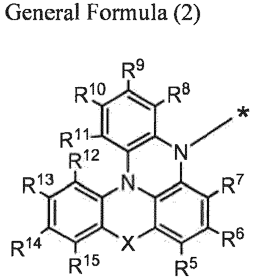

In the general formula (2), X represents a single bond, an oxygen atom or a sulfur atom. In one preferred embodiment of the present invention, X is a single bond. In one preferred embodiment of the present invention, X is an oxygen atom. X can be an oxygen atom or a sulfur atom.

In the general formula (2), * indicates a bonding position.

In the general formula (2), R⁵ to R¹⁵ each independently represent a hydrogen atom, a deuterium atom or a substituent. The substituent can be selected from, for example, Substituent Group A, or can be selected from Substituent Group B, or can be selected from Substituent Group C, or can be selected from Substituent Group D, or can be selected from Substituent Group E. In one preferred embodiment of the present invention, the substituent means one or a combination of two or more groups selected from the group consisting of an alkyl group (for example, having 1 to 20 carbon atoms), an aryl group (for example, having 6 to 22 carbon atoms), and a cyano group. For example, the substituent can be a cyano group, or an aryl group optionally substituted with one or a combination of two or more groups selected from the group consisting of a cyano group and an alkyl group. When two or more of R⁵ to R¹⁵ represent substituents, these two or more substituents can be the same or different. 6 to 11 of R⁵ to R¹⁵ are preferably hydrogen atoms or deuterium atoms, and for example, 8 to 11 can be hydrogen atoms or deuterium atoms. All R⁵ to R¹⁵ can be hydrogen atoms or deuterium atoms. 8 to 10 thereof can be hydrogen atoms or deuterium atoms. For example, 8 can be hydrogen atoms or deuterium atoms, or 9 can be hydrogen atoms or deuterium atoms, or 10 can be hydrogen atoms or deuterium atoms.

R⁵ and R⁶, R⁶ and R⁷, R⁸ and R⁹, R⁹ and R¹⁰, R¹⁰ and R¹¹, R¹¹ and R¹², R¹² and R¹³, R¹³ and R¹⁴, and R¹⁴ and R¹⁵ each can bond to each other to form a cyclic structure. The cyclic structure can be any of an aromatic ring, a heteroaromatic ring, an aliphatic hydrocarbon ring, or an aliphatic heteroring, and can also be a condensed ring thereof. Preferred is an aromatic ring or a heteroaromatic ring. The aromatic ring includes a benzene ring. The heteroaromatic ring means a ring of aromaticity containing a hetero atom as the ring skeleton constituting atom, and is preferably a 5- to 7-membered ring. For example, employable is a 5-membered ring or a 6-membered ring. In one embodiment of the present invention, the heteroaromatic ring includes a furan ring, a thiophene ring and a pyrrole ring. In one preferred embodiment of the present invention, the cyclic structure is a furan ring of a substituted or unsubstituted benzofuran, a thiophene ring of a substituted or unsubstituted benzothiophene, or a pyrrole ring of a substituted or unsubstituted indole. Benzofuran, benzothiophene and indole as referred to herein can be unsubstituted or can be substituted with a substituent selected from Substituent Group A, or can be substituted with a substituent selected from Substituent Group B, or can be substituted with a substituent selected from Substituent Group C, or can be substituted with a substituent selected from Substituent Group D, or can be substituted with a substituent selected from Substituent Group E. Preferably, a substituted or unsubstituted aryl group bonds to the nitrogen atom constituting the pyrrole ring of indole, and examples of the substituent include those selected from the group of any of Substituent Groups A to E. Preferably, 0 to 2 of R⁵ and R⁶, R⁶ and R⁷, R⁸ and R⁹, R⁹ and R¹⁰, R¹⁰ and R¹¹, R¹¹ and R¹², R¹² and R¹³, R¹³ and R¹⁴, and R¹⁴ and R¹⁵ each bond to each other to form a cyclic structure, more preferably 0 or 1 bonds to each other to form a cyclic structure. 1 or 2 each can bond to each other to form a cyclic structure. Or only 1 can bond to each other to form a cyclic structure. Or 0 can bond to each other to form a cyclic structure.

Hereinunder specific examples of D employable in the general formula (1) are shown. D that can be employed in the general formula (1) can be a group containing the following structure. For example, D can be a phenyl group substituted with a group having the following structure, or can be a group of a ring with which the benzene ring of the following structure is condensed (for example, a benzene ring). D that can be employed in the present invention should not be limitatively interpreted by the following specific examples. The waved line in the following specific examples indicates a bonding position.

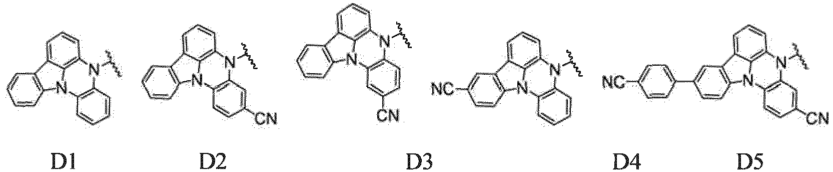

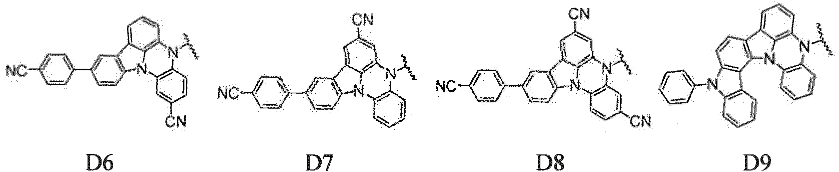

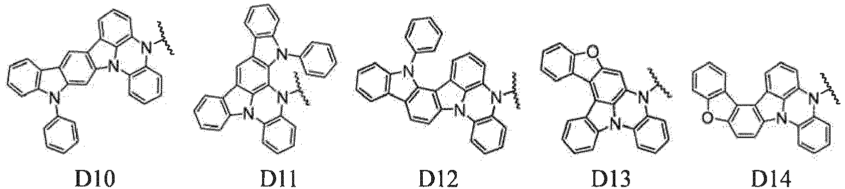

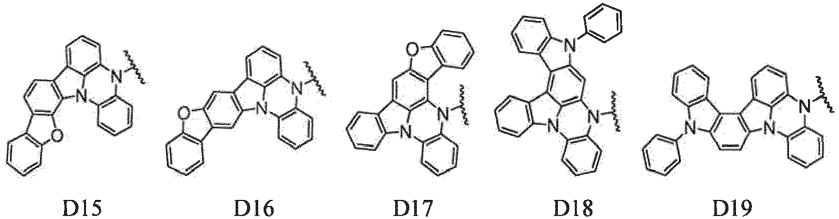

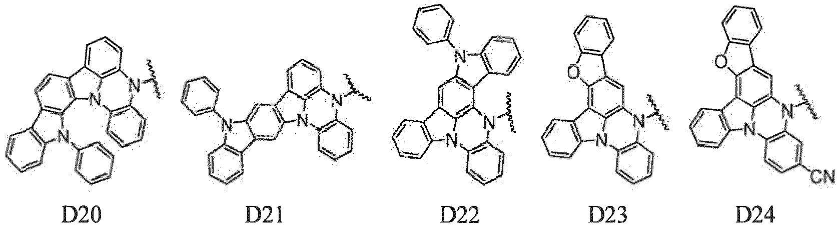

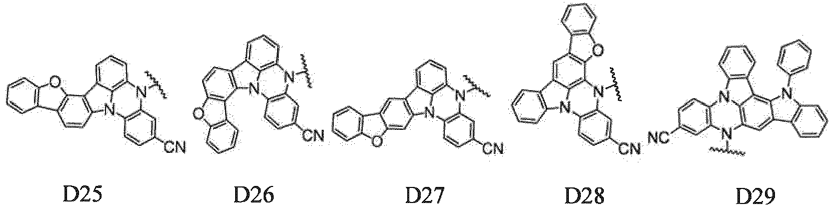

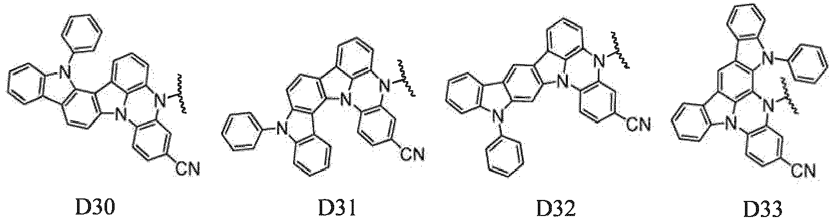

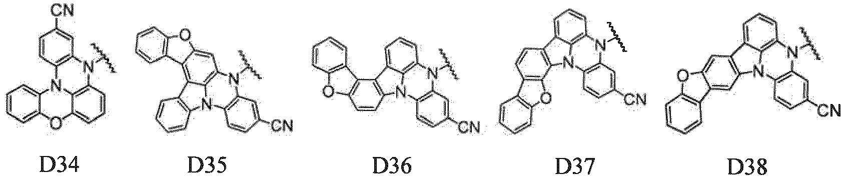

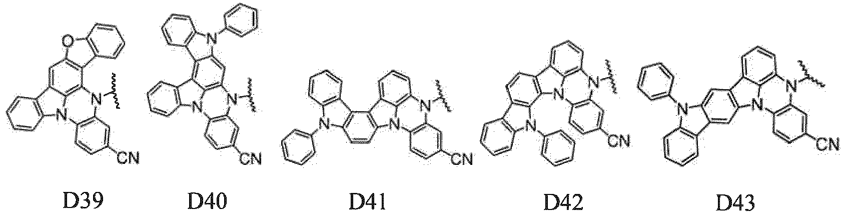

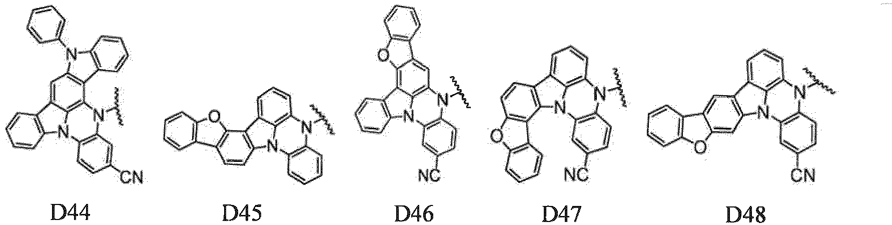

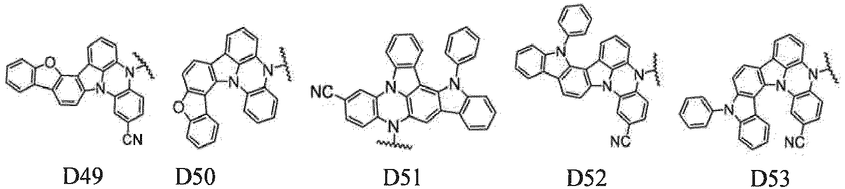

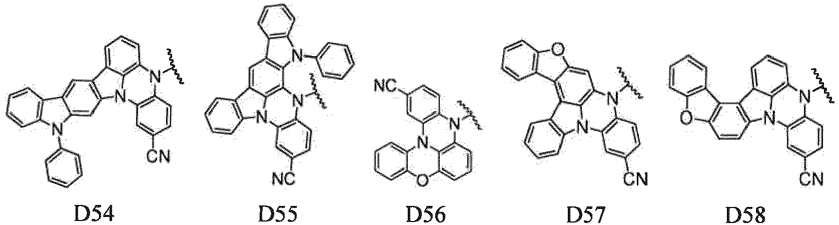

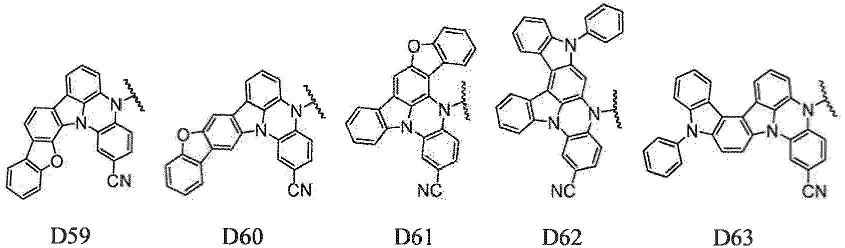

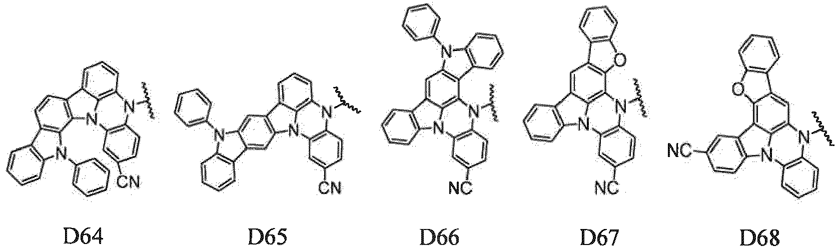

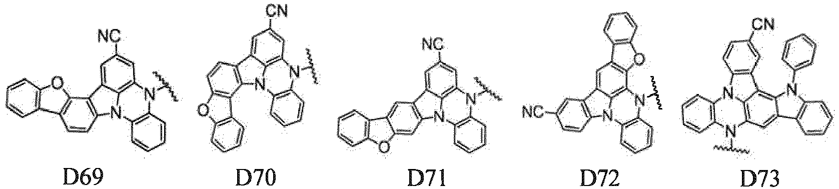

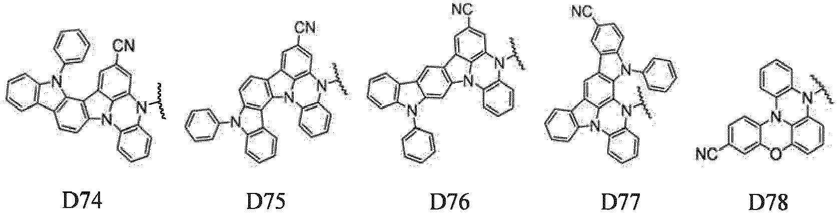

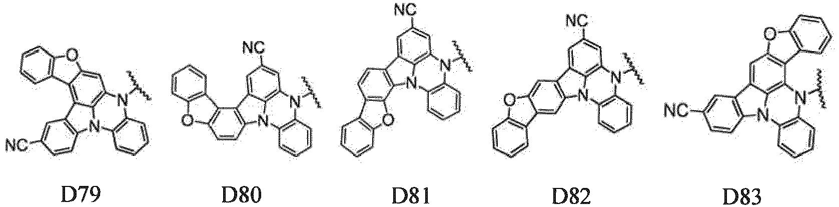

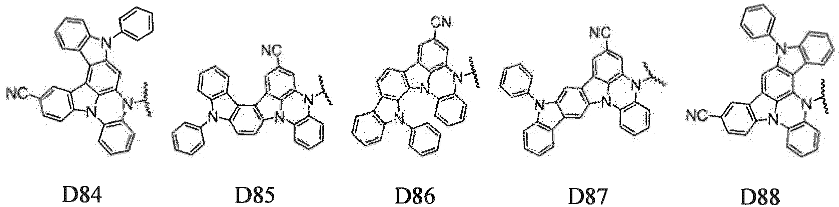

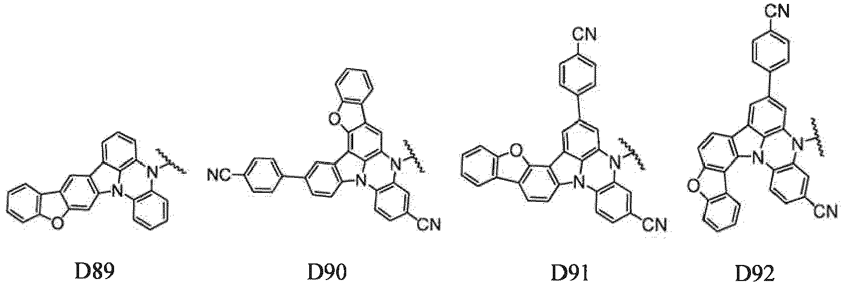

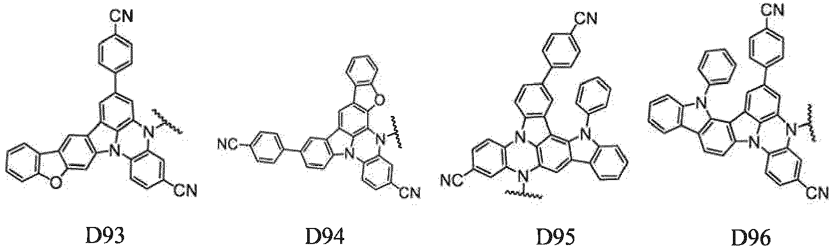

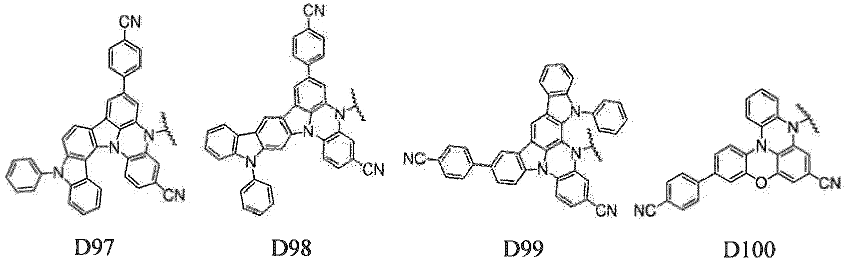

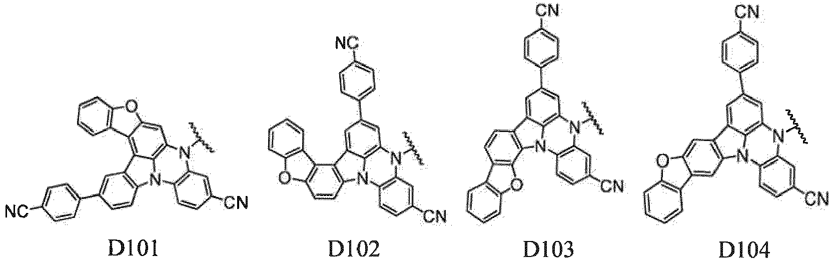

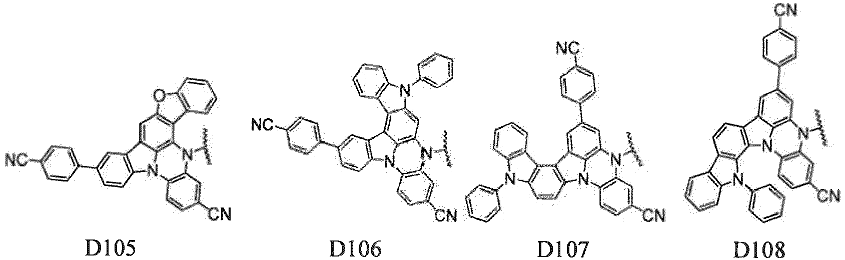

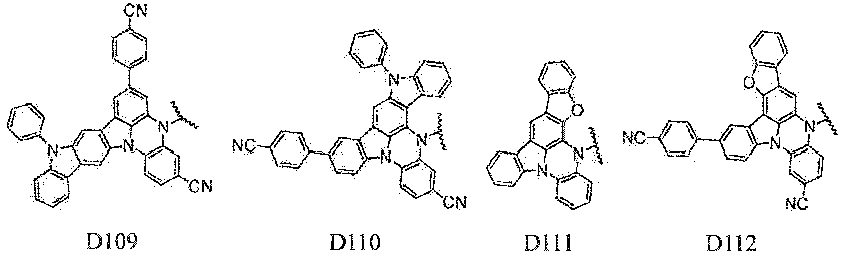

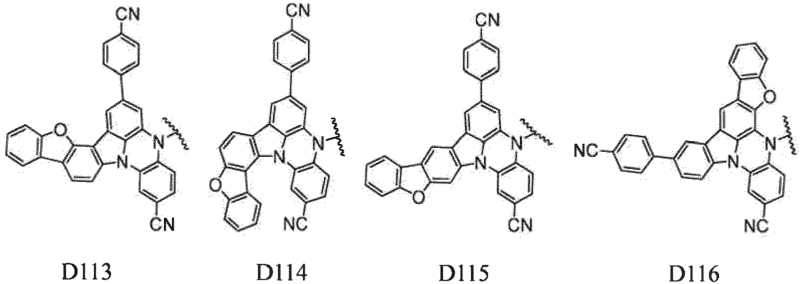

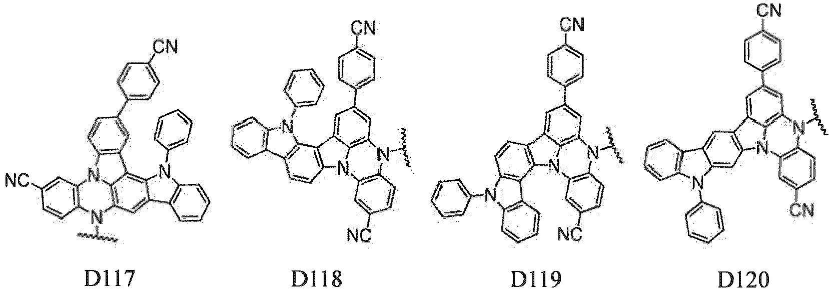

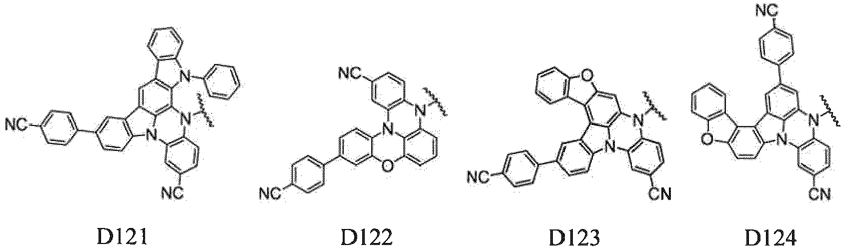

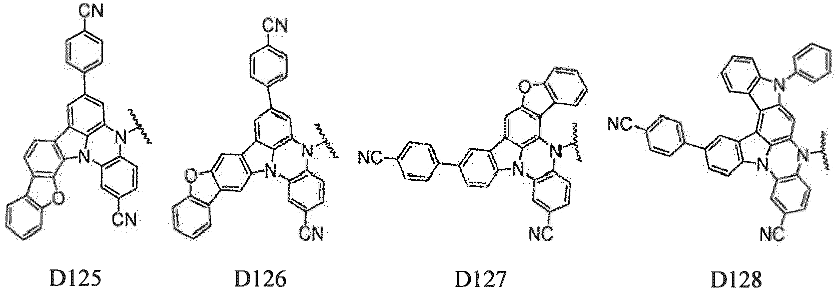

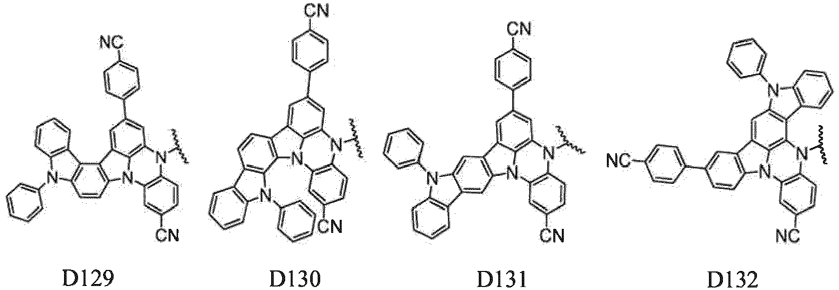

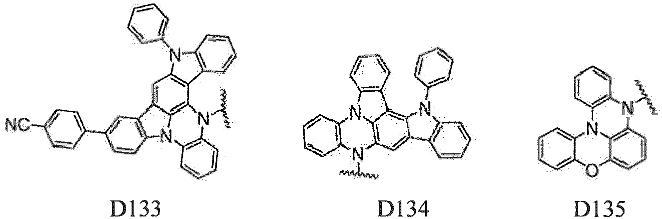

In the general formula (1), A represents one or a combination of two or more groups selected from the group consisting of a cyano group, a phenyl group, a pyrimidyl group, a triazyl group and an alkyl group (except for a substituted alkyl group). Namely, A is a cyano group, a substituted or unsubstituted phenyl group, a substituted or unsubstituted pyrimidyl group, or a substituted or unsubstituted triazyl group, and the substituent for the phenyl group, the pyrimidyl group and the triazyl group includes one or a combination of two or more groups selected from the group consisting of a cyano group, a phenyl group, a pyrimidyl group, a triazyl group and an alkyl group, and the phenyl group and the pyrimidyl group can be condensed with a benzene ring.

In a preferred embodiment of the present invention, A is a cyano group or a phenyl group substituted with a cyano group. In one embodiment of the present invention, A is a substituted or unsubstituted pyrimidyl group, or a substituted or unsubstituted triazyl group, preferably a pyrimidyl group substituted with a substituted or unsubstituted phenyl group, or a triazyl group substituted with a substituted or unsubstituted phenyl group. In one embodiment of the present invention, A is a phenyl group substituted with a substituted or unsubstituted pyrimidyl group, or a phenyl group substituted with a substituted or unsubstituted triazyl group.

Hereinunder specific examples of A that can be employed in the general formula (1) are shown. A employable in the general formula (1) can also be a group containing any of the following structures. For example, A can be a phenyl group substituted with a group having any of the following structures, or a group of the following structure in which the benzene ring is condensed with a ring (for example, a benzene ring). A that can be employed in the present invention should not be limitatively interpreted by the following specific examples. In the following specific examples, * indicates a bonding position. Methyl group is omitted. For example, A15 is a group having two 4-methylphenyl groups.

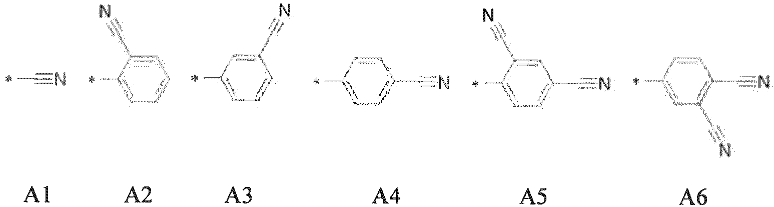

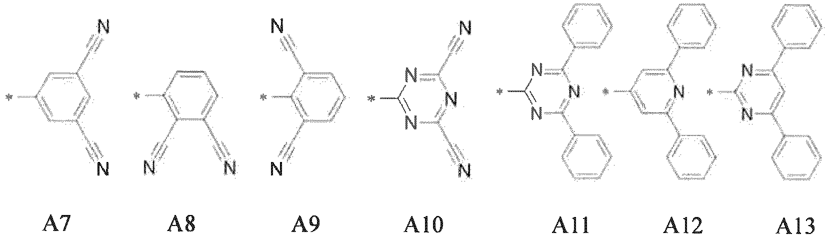

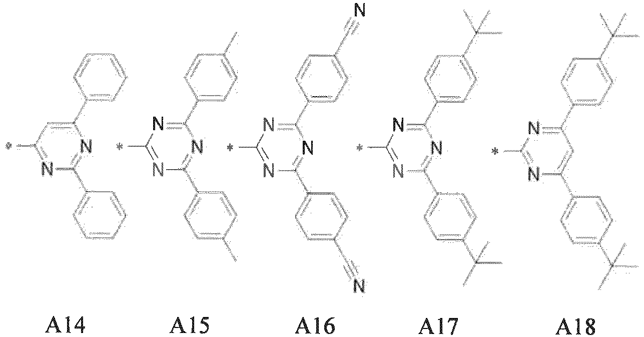

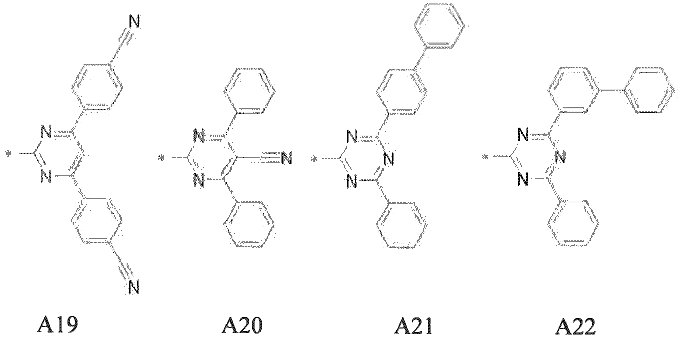

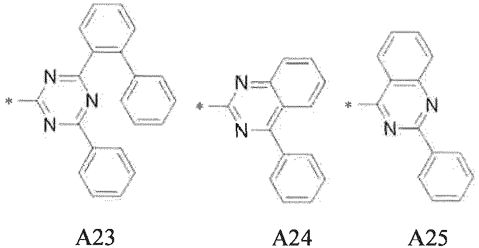

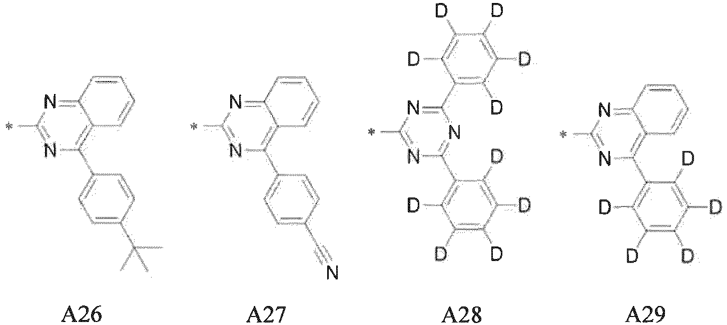

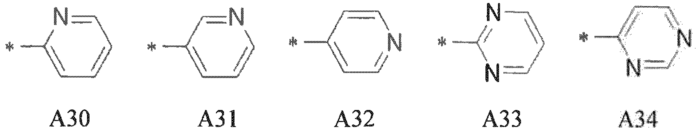

R¹ to R⁴ each independently represent a hydrogen atom, a deuterium atom, or one or a combination of two or more groups selected from the group consisting of an alkyl group, an aryl group, a heteroaryl group and a cyano group. Namely, R¹ to R⁴ each are independently a hydrogen atom, a deuterium atom, a substituted or unsubstituted alkyl group, a substituted or unsubstituted aryl group, a substituted or unsubstituted heteroaryl group, or a cyano group, the substituent for the alkyl group, the aryl group and the heteroaryl group includes one or a combination of two or more groups selected from the group consisting of an alkyl group, an aryl group, a heteroaryl group and a cyano group. In one embodiment of the present invention, the substituent is an alkyl group optionally substituted with an aryl group, or an aryl group optionally substituted with an alkyl group. In one embodiment of the present invention, the substituent is a cyano group, or an aryl group or a heteroaryl group substituted with a cyano group. When 2 or more of R¹ to R⁴ are substituents, these substituents can be the same or different. All R¹ to R⁴ can be hydrogen atoms or deuterium atoms.

In one preferred embodiment of the present invention, R¹ to R⁴ each are independently a hydrogen atom, a deuterium atom, or an alkyl group, an aryl group optionally substituted with a cyano group, or a pyridyl group, preferably a hydrogen atom, a deuterium atom, or an alkyl group, a phenyl group optionally substituted with a cyano group, or a pyridyl group. For example, a hydrogen atom, a deuterium atom, an alkylphenyl group, a cyanophenyl group, a phenyl group or a pyridyl group can be selected, and for example, a hydrogen atom, a deuterium atom, an alkylphenyl group or a phenyl group can be selected.

R¹ and R², and R³ and R⁴ each can bond to each other to form a cyclic structure selected from the group consisting of a benzene ring, a naphthalene ring and a pyridine ring, and the formed cyclic structure can be substituted with one or a combination of two or more groups selected from the group consisting of an alkyl group, an aryl group, and a cyano group. In one embodiment of the present invention, one pair of R¹ and R², and R³ and R⁴ bonds to each other to form a benzene ring, a naphthalene ring or a pyridine ring. In one embodiment of the present invention, both of R¹ and R², and R³ and R⁴ each bond to each other to form a benzene ring, a naphthalene ring or a pyridine ring. At that time, the ring formed by R¹ and R², and the ring formed by R³ and R⁴ can be the same or different. In one embodiment of the present invention, neither R¹ and R², nor R³ and R⁴ bond to each other to form a ring. In one embodiment of the present invention, the cyclic structure to be formed is a benzene ring or a naphthalene ring. In one embodiment of the present invention, the cyclic structure to be formed is a pyridine ring. The hydrogen atom bonding to the benzene ring, the naphthalene ring and the pyridine ring can be substituted with a deuterium atom or a substituent. The substituent as referred to herein includes one or a combination of two or more groups selected from the group consisting of an alkyl group, an aryl group, a heteroaryl group and a cyano group. In one embodiment of the present invention, the substituent is an alkyl group optionally substituted with an aryl group, or an aryl group optionally substituted with an alkyl group. In one embodiment of the present invention, the substituent is a cyano group, or an aryl group substituted with a cyano group. The hydrogen atom bonding to the benzene ring, the naphthalene ring and the pyridine ring can be unsubstituted.

Hereinunder specific examples of the aryl group optionally substituted with an alkyl group are shown. However, the aryl group optionally substituted with an alkyl group, which can be employed in the present invention, should not be limitatively interpreted by the following specific examples. In the following specific examples, * indicates a bonding position. Methyl group is omitted. For example, N4 is a 4-methylphenyl group. Above all, preferred are N5, N8, N10 and N11.

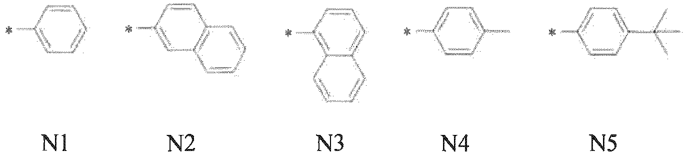

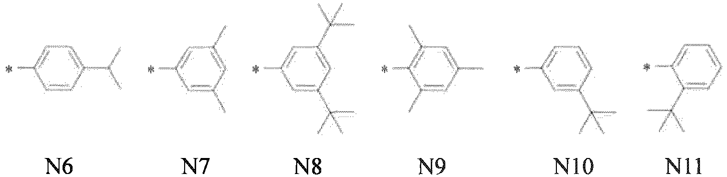

As R¹ to R⁴ in the general formula (1), more preferred are N5 and a tert-butyl group, and even more preferred is N5.

The compound represented by the general formula (1) can be a compound represented by the following general formula (3).

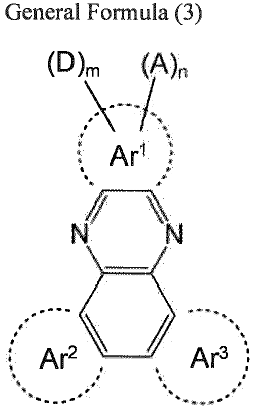

In the general formula (3), Ar¹ represents a cyclic structure, and represents a benzene ring, a naphthalene ring, an anthracene ring, or a phenanthrene ring. D represents a group represented by the above-mentioned general formula (2). A represents one or a combination of two or more groups selected from the group consisting of a cyano group, a phenyl group, a pyrimidyl group, a triazyl group and an alkyl group (except for a substituted alkyl group). m represents 1 or 2, n represents 0, 1 or 2. When m is 2, two D's can be the same or different. When n is 2, two A's can be the same or different. Ar² and Ar³ each can form a cyclic structure selected from the group consisting of a benzene ring, a naphthalene ring and a pyridine ring, and the formed cyclic structure can be substituted with one or a combination of two or more groups selected from the group consisting of an alkyl group, an aryl group, a heteroaryl group and a cyano group.

For the details and the preferred range of Ar¹, D, A, m and n in the general formula (3), reference can be made to the corresponding description of the above-mentioned general formula (1). For the details and the preferred range of the benzene ring, the naphthalene ring and the pyridine ring that Ar² and Ar³ represent, reference can be made to the description of the benzene ring, the naphthalene ring and the pyridine ring to be formed by R¹ and R², and R³ and R⁴ each bonding to each other in the general formula (1).

In one embodiment of the present invention, D in the general formula (3) is a substituted or unsubstituted 5H-indolo[3,2,1-de]phenazin-5-yl group, A is a cyano group, a phenyl group, a pyrimidyl group, a triazyl group or a benzonitrile group, n is 0 or 1, Ar² and Ar³ each are independently a benzene ring, a naphthalene ring, a pyridine ring, or a benzene ring substituted with a cyano group.

The compound represented by the general formula (1) preferably has a ring skeleton of any of the following, for example. At least one hydrogen atom in the following skeletons can be substituted with a deuterium atom or a substituent falling within the range of the general formula (1). However, any other ring is not condensed with the skeletons. The general formula (1) indispensably has D, and therefore one D alone is described in the following ring skeletons.

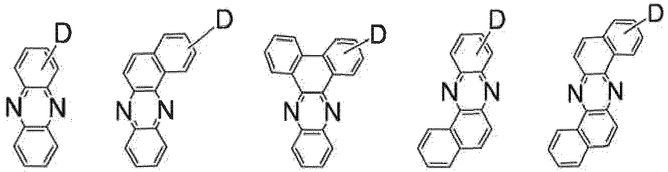

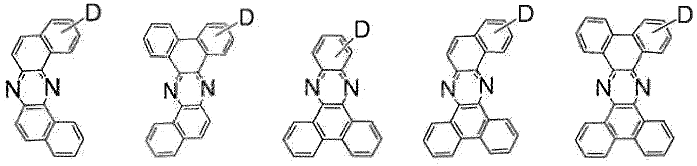

In one preferred embodiment of the present invention, the compound represented by the general formula (1) has a ring skeleton of any of the following Ring Skeleton Group 1.

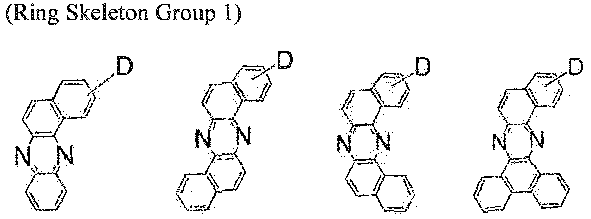

In one preferred embodiment of the present invention, the compound represented by the general formula (1) has a ring skeleton of any of the following Ring Skeleton Group 2.

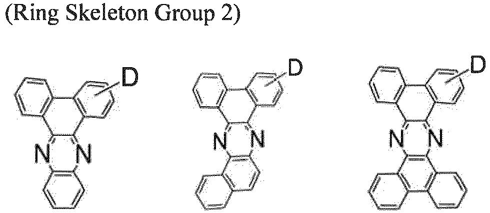

In Ring Skeleton Group 1 and Ring Skeleton Group 2, in one preferred embodiment, A does not exist in the molecule. In one embodiment of the present invention, a hydrogen atom, a deuterium atom, an unsubstituted alkyl group or an aryl group optionally substituted with an alkyl group bonds to the aromatic ring condensed with the lower part of the pyrazine ring in Ring Skeleton Group 1 and Ring Skeleton Group 2. In one preferred embodiment of the present invention, a hydrogen atom, a deuterium atom or an unsubstituted alkyl group bonds to the aromatic ring condensed with the lower part of the pyrazine ring in Ring Skeleton Group 1 and Ring Skeleton Group 2.

The compound represented by the general formula (1) can be a compound represented by any of the following general formulae (4a) to (4f).

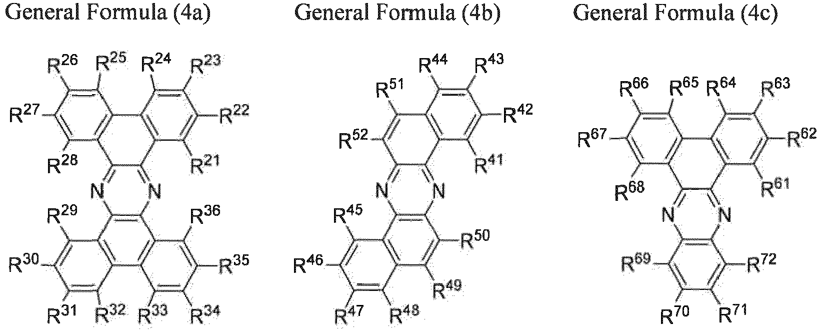

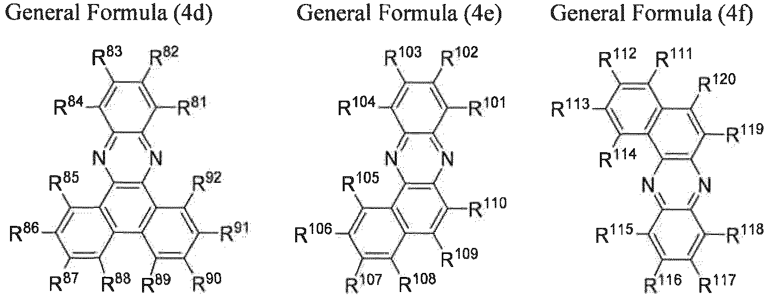

In the general formulae (4a) to (4f), R²¹ to R²⁸, R⁴¹ to R⁴⁴, R⁵¹, R⁵², R⁶¹ to R⁶⁸, R⁸¹ to R⁸⁴, R¹⁰¹ to R¹⁰⁴, R¹¹¹ to R¹¹⁴, R¹¹⁹, and R¹²⁰ each independently represent a hydrogen atom, a deuterium atom, D or A. Provided that 1 or 2 of R²¹ to R²⁸ are D, and 0 to 2 are A; 1 or 2 of R⁴¹ to R⁴⁴, R⁵¹ and R⁵² are D, and 0 to 2 are A; 1 or 2 of R⁶¹ to R⁶⁸ are D, and 0 to 2 are A; 1 or 2 of R⁸¹ to R⁸⁴ are D, and 0 to 2 are A; 1 or 2 of R¹⁰¹ to R¹⁰⁴ are D and 0 to 2 are A; 1 or 2 of R¹¹¹ to R¹¹⁴, R¹¹⁹ and R¹²⁰ are D, and 0 to 2 are A. R²⁹ to R³⁶, R⁴⁵ to R⁵⁰, R⁶⁹ to R⁷², R⁸⁵ to R⁹², R¹⁰⁵ to R¹¹⁰, and R¹¹⁵ to R¹¹⁸ each independently represent a hydrogen atom, a deuterium atom, or one or a combination of two or more groups selected from the group consisting of an alkyl group, an aryl group and a cyano group. In the general formulae (4a) to (4f), the ring skeleton described is not further condensed with any other ring.

For the details and the preferred range of the general formulae (4a) to (4f), reference can be made to the corresponding description of the general formula (1). In one embodiment of the present invention, the compound represented by the general formula (4a) is selected. In one embodiment of the present invention, the compound represented by the general formula (4b) is selected. In one embodiment of the present invention, the compound represented by the general formula (4c) is selected. In one embodiment of the present invention, the compound represented by the general formula (4d) is selected. In one embodiment of the present invention, the compound represented by the general formula (4e) is selected. In one embodiment of the present invention, the compound represented by the general formula (4f) is selected.

Specific examples of the compound represented by the general formula (1) are shown in the following Tables 1 to 12. Specific examples of the compound represented by the general formula (4a') are shown in Table 1 and Table 2; specific examples of the compound represented by the general formula (4b') are shown in Table 3 and Table 4; specific examples of the compound represented by the general formula (4c') are shown in Table 5 and Table 6; specific examples of the compound represented by the general formula (4d') are shown in Table 7 and Table 8; specific examples of the compound represented by the general formula (4e') are shown in Table 9 and Table 10; specific examples of the compound represented by the general formula (4f) are shown in Table 11 and Table 12. However, the compound represented by the general formula (1) employable in the present invention should not be limitatively interpreted by these specific examples.

**[Table 1]**

| | | | |
|---|---|---|---|
| 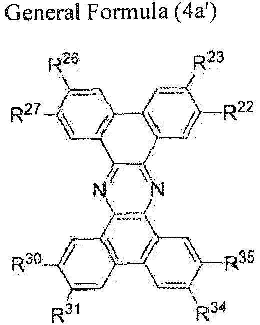 | | | |

| No. | R²² | R²³ | R²⁶,R²⁷,R³⁰, R³¹,R³⁴,R³⁵ |
|---|---|---|---|
| 1 | D1 | H | H |
| 2 | D2 | H | H |
| 3 | D3 | H | H |
| 4 | D4 | H | H |
| 5 | D5 | H | H |
| 6 | D8 | H | H |
| 7 | D7 | H | H |
| 8 | D8 | H | H |
| 9 | D9 | H | H |
| 10 | D10 | H | H |
| 11 | D11 | H | H |
| 12 | D12 | H | H |
| 13 | D13 | H | H |
| 14 | D14 | H | H |
| 15 | D15 | H | H |
| 16 | D16 | H | H |
| 17 | D17 | H | H |
| 18 | D18 | H | H |
| 19 | D19 | H | H |
| 20 | D20 | H | H |
| 21 | D21 | H | H |
| 22 | D22 | H | H |
| 23 | D23 | H | H |
| 24 | D24 | H | H |
| 25 | D25 | H | H |
| 26 | D26 | H | H |
| 27 | D27 | H | H |
| 28 | D28 | H | H |
| 29 | D29 | H | H |
| 30 | D30 | H | H |
| 31 | D31 | H | H |
| 32 | D32 | H | H |
| 33 | D33 | H | H |
| 34 | D34 | H | H |
| 35 | D35 | H | H |
| 36 | D38 | H | H |
| 37 | D37 | H | H |
| 38 | D38 | H | H |
| 39 | D39 | H | H |
| 40 | D40 | H | H |
| 41 | D41 | H | H |
| 42 | D42 | H | H |
| 43 | D43 | H | H |
| 44 | D44 | H | H |
| 45 | D45 | H | H |
| 48 | D46 | H | H |
| 47 | D47 | H | H |
| 48 | D48 | H | H |
| 49 | D49 | H | H |
| 50 | D50 | H | H |
| 51 | H | D1 | H |
| 52 | H | D2 | H |
| 53 | H | D3 | H |
| 54 | H | D4 | H |
| 55 | H | D5 | H |
| 56 | H | D6 | H |
| 57 | H | D7 | H |
| 58 | H | D8 | H |
| 59 | H | D9 | H |
| 60 | H | D10 | H |
| 61 | H | D11 | H |
| 62 | H | D12 | H |
| 63 | H | D13 | H |
| 64 | H | D14 | H |
| 65 | H | D15 | H |
| 66 | H | D18 | H |
| 67 | H | D17 | H |
| 68 | H | D18 | H |
| 69 | H | D19 | H |
| 70 | H | D20 | H |
| 71 | H | D21 | H |
| 72 | H | D22 | H |
| 73 | H | D23 | H |
| 74 | H | D24 | H |
| 75 | H | D25 | H |
| 76 | H | D28 | H |
| 77 | H | D27 | H |
| 78 | H | D28 | H |
| 79 | H | D29 | H |
| 80 | H | D30 | H |
| 81 | H | D31 | H |
| 82 | H | D32 | H |
| 83 | H | D33 | H |
| 84 | H | D34 | H |
| 85 | H | D35 | H |
| 88 | H | D36 | H |
| 87 | H | D37 | H |
| 88 | H | D38 | H |
| 89 | H | D39 | H |
| 90 | H | D40 | H |
| 91 | H | D41 | H |
| 92 | H | D42 | H |
| 93 | H | D43 | H |
| 94 | H | D44 | H |
| 95 | H | D45 | H |
| 96 | H | D46 | H |
| 97 | H | D47 | H |
| 98 | H | D48 | H |
| 99 | H | D49 | H |
| 100 | H | D50 | H |

The following Table 2 further exemplifies the compound represented by the general formula (4a') in a different table form. In Table 2, structures formed by further substituting a part of the structure specified by the compound number are further given compound numbers. For example, in Table 2, Compounds 101 to 150 (expressed as Nos. 101 to 150 in the table) are compounds formed by further substituting R²⁷ (expressed as R27 in the table) of Compounds 1 to 50 with a substituent of R²² (expressed as R22 in the table) of Compounds 1 to 50). Compound 101 is a compound formed by further substituting R²⁷ of Compound 1 with D1 of R²² of Compound 1; Compound 102 is a compound formed by further substituting R²⁷ of Compound 2 with D2 of R²² of Compound 2. The structures of the compounds listed in Table 2 and the compounds listed in Table 4, Table 6, Table 8, Table 10 and Table 12 are identified in such a manner. In Table 2, Table 4, Table 6, Table 8, Table 10 and Table 12, every numbered compound is individually identified for the structure thereof, and is specifically disclosed one by one in the present specification. In the tables, "t-Bu" represents a tertiary butyl group (tert-butyl group).

**[Table 2]**

| Group to be Substituted | | | Substituting Group | | New Compound No. | |
|---|---|---|---|---|---|---|
| To R27 of | Nos. | 1 to 50, | the same group as R22 | is introduced to form | Nos. | 101 to 150 |
| To R26 of | Nos. | 51 to 100, | the same group as R23 | is introduced to form | Nos. | 151 to 200 |
| To R30 of | Nos. | 1 to 200, | N1 | is introduced to form | Nos. | 201 to 400 |
| To R31 of | Nos. | 1 to 200, | N1 | is introduced to form | Nos. | 401 to 600 |
| To R34 of | Nos. | 1 to 100, | N1 | is introduced to form | Nos. | 601 to 700 |
| To R35 of | Nos. | 1 to 100, | N1 | is introduced to form | Nos. | 701 to 800 |
| To R30 and R35 of | Nos. | 1 to 200, | N1 | is introduced to form | Nos. | 801 to 1000 |
| To R31 and R34 of | Nos. | 1 to 200, | N1 | is introduced to form | Nos. | 1001 to 1200 |
| To R30 of | Nos. | 1 to 200, | A4 | is introduced to form | Nos. | 1201 to 1400 |
| To R31 of | Nos. | 1 to 200, | A4 | is introduced to form | Nos. | 1401 to 1600 |
| To R34 of | Nos. | 1 to 100, | A4 | is introduced to form | Nos. | 1601 to 1700 |
| To R35 of | Nos. | 1 to 100, | A4 | is introduced to form | Nos. | 1701 to 1800 |
| To R30 and R35 of | Nos. | 1 to 200, | A4 | is introduced to form | Nos. | 1801 to 2000 |
| To R31 and R34 of | Nos. | 1 to 200, | A4 | is introduced to form | Nos. | 2001 to 2200 |
| To R30 of | Nos. | 1 to 200, | N5 | is introduced to form | Nos. | 2201 to 2400 |
| To R31 of | Nos. | 1 to 200, | N5 | is introduced to form | Nos. | 2401 to 2600 |
| To R34 of | Nos. | 1 to 100, | N5 | is introduced to form | Nos. | 2601 to 2700 |
| To R35 of | Nos. | 1 to 100, | N5 | is introduced to form | Nos. | 2701 to 2800 |
| To R30 and R35 of | Nos. | 1 to 200, | N5 | is introduced to form | Nos. | 2801 to 3000 |
| To R31 and R34 of | Nos. | 1 to 200, | N5 | is introduced to form | Nos. | 3001 to 3200 |
| To R30 and R35 of | Nos. | 1 to 200, | N8 | is introduced to form | Nos. | 16201 to 16400 |
| To R31 and R34 of | Nos. | 1 to 200, | N8 | is introduced to form | Nos. | 16401 to 16600 |
| To R30 and R35 of | Nos. | 1 to 200, | N10 | is introduced to form | Nos. | 16601 to 16800 |
| To R31 and R34 of | Nos. | 1 to 200, | N10 | is introduced to form | Nos. | 16801 to 17000 |
| To R30 and R35 of | Nos. | 1 to 200, | t-Bu | is introduced to form | Nos. | 17001 to 17200 |
| To R31 and R34 of | Nos. | 1 to 200, | t-Bu | is introduced to form | Nos. | 17201 to 17400 |
| To R26 of | Nos. | 1 to 100, | N5 | is introduced to form | Nos. | 17401 to 17500 |
| To R27 of | Nos. | 1 to 100, | N5 | is introduced to form | Nos. | 17501 to 17600 |
| To R26 of | Nos. | 1 to 100, | N8 | is introduced to form | Nos. | 17601 to 17700 |
| To R27 of | Nos. | 1 to 100, | N8 | is introduced to form | Nos. | 17701 to 17800 |
| To R26 of | Nos. | 1 to 100, | N9 | is introduced to form | Nos. | 17801 to 17900 |
| To R27 of | Nos. | 1 to 100. | N9 | is introduced to form | Nos. | 17901 to 18000 |
| To R26 of | Nos. | 1 to 100, | t-Bu | is introduced to form | Nos. | 18001 to 18100 |
| To R27 of | Nos. | 1 to 100, | t-Bu | is introduced to form | Nos. | 18101 to 18200 |

**[Table 3]**

| | | | |
|---|---|---|---|
| 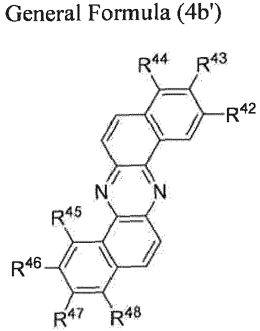 | | | |

| No. | R⁴² | R⁴³ | **R⁴⁴∼R⁴⁸** |
|---|---|---|---|
| 3201 | D1 | H | H |
| 3202 | D2 | H | H |
| 3203 | D3 | H | H |
| 3204 | D4 | H | H |
| 3205 | D5 | H | H |
| 3208 | D6 | H | H |
| 3207 | D7 | H | H |
| 3208 | D8 | H | H |
| 3209 | D9 | H | H |
| 3210 | D10 | H | H |
| 3211 | D11 | H | H |
| 3212 | D12 | H | H |
| 3213 | D13 | H | H |
| 3214 | D14 | H | H |
| 3215 | D15 | H | H |
| 3216 | D18 | H | H |
| 3217 | D17 | H | H |
| 3218 | D18 | H | H |
| 3219 | D19 | H | H |
| 3220 | D20 | H | H |
| 3221 | D21 | H | H |
| 3222 | D22 | H | H |
| 3223 | D23 | H | H |
| 3224 | D24 | H | H |
| 3225 | D25 | H | H |
| 3228 | D28 | H | H |
| 3227 | D27 | H | H |
| 3228 | D28 | H | H |
| 3229 | D29 | H | H |
| 3230 | D30 | H | H |
| 3231 | D31 | H | H |
| 3232 | D32 | H | H |
| 3233 | D33 | H | H |
| 3234 | D34 | H | H |
| 3235 | D35 | H | H |
| 3236 | D36 | H | H |
| 3237 | D37 | H | H |
| 3238 | D38 | H | H |
| **3239** | D39 | H | H |
| 3240 | D40 | H | H |
| 3241 | D41 | H | H |
| 3242 | D42 | H | H |
| 3243 | D43 | H | H |
| 3244 | D44 | H | H |
| 3245 | D45 | H | H |
| 3248 | D46 | H | H |
| 3247 | D47 | H | H |
| 3248 | D48 | H | H |
| 3249 | D49 | H | H |
| 3250 | D50 | H | H |
| 3251 | H | D1 | H |
| 3252 | H | D2 | H |
| 3253 | H | D3 | H |
| 3254 | H | D4 | H |
| 3255 | H | D5 | H |
| 3256 | H | D6 | H |
| 3257 | H | D7 | H |
| 3258 | H | D8 | H |
| 3259 | H | D9 | H |
| 3280 | H | D10 | H |
| 3261 | H | D11 | H |
| 3262 | H | D12 | H |
| 3263 | H | D13 | H |
| 3264 | H | D14 | H |
| 3265 | H | D15 | H |
| 3266 | H | D16 | H |
| 3267 | H | D17 | H |
| 3268 | H | D18 | H |
| 3269 | H | D19 | H |
| 3270 | H | D20 | H |
| 3271 | H | D21 | H |
| 3272 | H | D22 | H |
| 3273 | H | D23 | H |
| 3274 | H | D24 | H |
| 3275 | H | D25 | H |
| 3276 | H | D26 | H |
| 3277 | H | D27 | H |
| 3278 | H | D28 | H |
| 3279 | H | D29 | H |
| 3280 | H | D30 | H |
| 3281 | H | D31 | H |
| 3282 | H | D32 | H |
| 3283 | H | D33 | H |
| 3284 | H | D34 | H |
| 3285 | H | D35 | H |
| 3288 | H | D38 | H |
| 3287 | H | D37 | H |
| 3288 | H | D38 | H |
| 3289 | H | D39 | H |
| 3290 | H | D40 | H |
| 3291 | H | D41 | H |
| 3292 | H | D42 | H |
| 3293 | H | D43 | H |
| 3294 | H | D44 | H |
| 3295 | H | D45 | H |
| 3296 | H | D46 | H |
| 3297 | H | D47 | H |
| 3298 | H | D48 | H |
| 3299 | H | D49 | H |
| 3300 | H | D50 | H |

**[Table 4]**

| Group to be Substituted | | | Substituting Group | | New Compound No. | |
|---|---|---|---|---|---|---|
| To R43 of | Nos. | 3201 to 3250, | the same group as R42 | is introduced to form | Nos. | 3301 to 3350 |
| To R44 of | Nos. | 3251 to 3300, | the same group as R43 | is introduced to form | Nos. | 3351 to 3400 |
| To R45 of | Nos. | 3201 to 3400, | N1 | is introduced to form | Nos. | 3401 to 3600 |
| To R46 of | Nos. | 3201 to 3400, | N1 | is introduced to form | Nos. | 3601 to 3800 |
| To R47 of | Nos. | 3201 to 3400, | N1 | is introduced to form | Nos. | 3801 to 4000 |
| To R48 of | Nos. | 3201 to 3400, | N1 | is introduced to form | Nos. | 4001 to 4200 |
| To R45 of | Nos. | 3201 to 3400, | A4 | is introduced to form | Nos. | 4201 to 4400 |
| To R46 of | Nos. | 3201 to 3400, | A4 | is introduced to form | Nos. | 4401 to 4600 |
| To R47 of | Nos. | 3201 to 3400, | A4 | is introduced to form | Nos. | 4601 to 4800 |
| To R48 of | Nos. | 3201 to 3400, | A4 | is introduced to form | Nos. | 4801 to 5000 |
| To R45 of | Nos. | 3201 to 3400, | N5 | is introduced to form | Nos. | 5001 to 5200 |
| To R46 of | Nos. | 3201 to 3400, | N5 | is introduced to form | Nos. | 5201 to 5400 |
| To R47 of | Nos. | 3201 to 3400, | N5 | is introduced to form | Nos. | 5401 to 5600 |
| To R48 of | Nos. | 3201 to 3400, | N5 | is introduced to form | Nos. | 5601 to 5800 |

**[Table 5]**

| | | | |
|---|---|---|---|
| 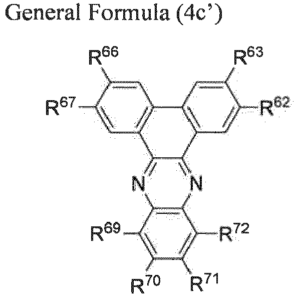 | | | |

| No. | R⁶² | R⁶³ | R⁶⁶,R⁶⁷ R⁶⁹∼R⁷² |
|---|---|---|---|
| 5801 | D1 | H | H |
| 5802 | D2 | H | H |
| 5803 | D3 | H | H |
| 5804 | D4 | H | H |
| 5805 | D5 | H | H |
| 5808 | D6 | H | H |
| 5807 | D7 | H | H |
| 5808 | D8 | H | H |
| 5809 | D9 | H | H |
| 5810 | D10 | H | H |
| 5811 | D11 | H | H |
| 5812 | D12 | H | H |
| 5813 | D13 | H | H |
| 5814 | D14 | H | H |
| 5815 | D15 | H | H |
| 5816 | D16 | H | H |
| 5817 | D17 | H | H |
| 5818 | D18 | H | H |
| 5819 | D19 | H | H |
| 5820 | D20 | H | H |
| 5821 | D21 | H | H |
| 5822 | D22 | H | H |
| 5823 | D23 | H | H |
| 5824 | D24 | H | H |
| 5825 | D25 | H | H |
| 5826 | D26 | H | H |
| 5827 | D27 | H | H |
| 5828 | D28 | H | H |
| 5829 | D29 | H | H |
| 5830 | D30 | H | H |
| 5831 | D31 | H | H |
| 5832 | D32 | H | H |
| 5833 | D33 | H | H |
| 5834 | D34 | H | H |
| 5835 | D35 | H | H |
| 5836 | D38 | H | H |
| 5837 | D37 | H | H |
| 5838 | D38 | H | H |
| 5839 | D39 | H | H |
| 5840 | D40 | H | H |
| 5841 | D41 | H | H |
| 5842 | D42 | H | H |
| 5843 | D43 | H | H |
| 5844 | D44 | H | H |
| 5845 | D45 | H | H |
| 5846 | D46 | H | H |
| 5847 | D47 | H | H |
| 5848 | D48 | H | H |
| 5849 | D49 | H | H |
| 5850 | D50 | H | H |
| 5851 | H | D1 | H |
| 5852 | H | D2 | H |
| 5853 | H | D3 | H |
| 5854 | H | D4 | H |
| 5855 | H | D5 | H |
| 5858 | H | D6 | H |
| 5857 | H | D7 | H |
| 5858 | H | D8 | H |
| 5859 | H | D9 | H |
| 5860 | H | D10 | H |
| 5861 | H | D11 | H |
| 5862 | H | D12 | H |
| 5863 | H | D13 | H |
| 5864 | H | D14 | H |
| 5865 | H | D15 | H |
| 5866 | H | D16 | H |
| 5867 | H | D17 | H |
| 5868 | H | D18 | H |
| 5869 | H | D19 | H |
| 5870 | H | D20 | H |
| 5871 | H | D21 | H |
| 5872 | H | D22 | H |
| 5873 | H | D23 | H |
| 5874 | H | D24 | H |
| 5875 | H | D25 | H |
| 5876 | H | D28 | H |
| 5877 | H | D27 | H |
| 5878 | H | D28 | H |
| 5879 | H | D29 | H |
| 5880 | H | D30 | H |
| 5881 | H | D31 | H |
| 5882 | H | D32 | H |
| 5883 | H | D33 | H |
| 5884 | H | D34 | H |
| 5885 | H | D35 | H |
| 5886 | H | D36 | H |
| 5887 | H | D37 | H |
| 5888 | H | D38 | H |
| 5889 | H | D39 | H |
| 5890 | H | D40 | H |
| 5891 | H | D41 | H |
| 5892 | H | D42 | H |
| 5893 | H | D43 | H |
| 5894 | H | D44 | H |
| 5895 | H | D45 | H |
| 5896 | H | D46 | H |
| 5897 | H | D47 | H |
| 5898 | H | D48 | H |
| 5899 | H | D49 | H |
| 5800 | H | D50 | H |

**[Table 6]**

| Group to be Substituted | | | Substituting Group | | New Compound No. | |
|---|---|---|---|---|---|---|
| To R67 of | Nos. | 5801 to 5850. | the same group as R62, | is introduced to form | Nos. | 5901 to 5950 |
| To R66 of | Nos. | 5851 to 5900. | the same group as R63 | is introduced to form | Nos. | 5951 to 6000 |
| To R69 of | Nos. | 5801 to 6000, | N1 | is introduced to form | Nos. | 6001 to 6200 |
| To R70 of | Nos. | 5801 to 6000, | N1 | is introduced to form | Nos. | 6201 to 6400 |
| To R71 of | Nos. | 5801 to 5900, | N1 | is introduced to form | Nos. | 6401 to 6500 |
| To R72 of | Nos. | 5801 to 5900, | N1 | is introduced to form | Nos. | 6501 to 6600 |
| To R69 of | Nos. | 5801 to 6000, | A4 | is introduced to form | Nos. | 6601 to 6800 |
| To R70 of | Nos. | 5801 to 6000. | A4 | is introduced to form | Nos.' | 6801 to 7000 |
| To R71 of | Nos. | 5801 to 5900. | A4 | is introduced to form | Nos. | 7001 to 7100 |
| To R72 of | Nos. | 5801 to 5900, | A4 | is introduced to form | Nos. | 7101 to 7200 |
| To R69 of | Nos. | 5801 to 6000, | N5 | is introduced to form | Nos. | 7201 to 7400 |
| To R70 of | Nos. | 5801 to 6000, | N5 | is introduced to form | Nos. | 7401 to 7600 |
| To R71 of | Nos. | 5801 to 5900. | N5 | is introduced to form | Nos. | 7601 to 7700 |
| To R72 of | Nos. | 5801 to 5900, | N5 | is introduced to form | Nos. | 7701 to 7800 |
| To R69 and R72 of | Nos. | 5801 to 6000, | N1 | is introduced to form | Nos. | 18201 to 18400 |
| To R70 and R71 of | Nos. | 5801 to 6000, | N1 | is introduced to form | Nos. | 18401 to 18600 |
| To R69 and R72 of | Nos. | 5801 to 6000, | N5 | is introduced to form | Nos. | 18601 to 18800 |
| To R70 and R71 of | Nos. | 5801 to 6000, | N5 | is introduced to form | Nos. | 18801 to 19000 |
| To R69 and R72 of | Nos. | 5801 to 6000. | N7 | is introduced to form | Nos. | 19001 to 19200 |
| To R70 and R71 of | Nos. | 5801 to 6000, | N7 | is introduced to form | Nos. | 19201 to 19400 |
| To R69 and R72 of | Nos. | 5801 to 6000, | N8 | is introduced to form | Nos. | 19401 to 19600 |
| To R70 and R71 of | Nos. | 5801 to 6000, | N8 | is introduced to form | Nos. | 19601 to 19800 |
| To R69 and R72 of | Nos. | 5801 to 6000, | N9 | is introduced to form | Nos. | 19801 to 20000 |
| To R70 and R71 of | Nos. | 5801 to 6000, | N9 | is introduced to form | Nos. | 20001 to 20200 |
| To R69 and R72 of | Nos. | 5801 to 6000, | N10 | is introduced to form | Nos. | 20201 to 20400 |
| To R70 and R71 of | Nos. | 5801 to 6000, | N10 | is introduced to form | Nos. | 20401 to 20600 |
| To R69 and R72 of | Nos. | 5801 to 6000. | N11 | is introduced to form | Nos. | 20601 to 20800 |
| To R70 and R71 of | Nos. | 5801 to 6000. | N11 | is introduced to form | Nos. | 20801 to 21000 |
| To R66, R69, R72 of | Nos. | 5801 to 5900 | N5 | is introduced to form | Nos. | 21001 to 21100 |
| To R66, R70, R71 of | Nos. | 5801 to 5900, | N5 | is introduced to form | Nos. | 21101 to 21200 |
| To R67, R69, R72 of | Nos. | 5801 to 5900, | N5 | is introduced to form | Nos. | 21201 to 21300 |
| To R67, R70, R71 of | Nos. | 5801 to 5900, | N5 | is introduced to form | Nos. | 21301 to 21400 |
| To R66, R69, R72 of | Nos. | 5801 to 5900, | N8 | is introduced to form | Nos. | 21401 to 21500 |
| To R66, R70, R71 of | Nos. | 5801 to 5900, | N8 | is introduced to form | Nos. | 21501 to 21600 |
| To R67, R69, R72 of | Nos. | 5801 to 5900. | N8 | is introduced to form | Nos. | 21601 to 21700 |
| To R67, R70, R71 of | Nos. | 5801 to 5900, | N8 | is introduced to form | Nos. | 21701 to 21800 |
| To R66, R69, R72 of | Nos. | 5801 to 5900, | N10 | is introduced to form | Nos. | 21801 to 21900 |
| To R66, R70, R71 of | Nos. | 5801 to 5900. | N10 | is introduced to form | Nos. | 21901 to 22000 |
| To R67, R69, R72 of | Nos. | 5801 to 5900, | N10 | is introduced to form | Nos. | 22001 to 22100 |
| To R67, R70, R71 of | Nos. | 5801 to 5900, | N10 | is introduced to form | Nos. | 22101 to 22200 |
| To R66 of | Nos. | 5801 to 5900, | introduce t-Bu, and | | | |
| to R69 and R72 thereof | | | introduce N5 | to form | Nos. | 22201 to 22300 |
| To R66 of | Nos. | 5801 to 5900, | introduce t-Bu, and | | | |
| to R70 and R71 thereof | | | introduce N5 | to form | Nos. | 22301 to 22400 |
| To R67 of | Nos. | 5801 to 5900. | introduce t-Bu, and | | | |
| to R69 and R72 thereof | | | introduce N5 | to form | Nos. | 22401 to 22500 |
| To R67 of | Nos. | 5801 to 5900. | introduce t-Bu, and | | | |
| to R70 and R71 thereof | | | introduce N5 | to form | Nos. | 22501 to 22600 |

**[Table 7]**

| | | | |
|---|---|---|---|
| 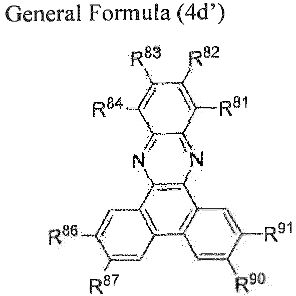 | | | |

| No. | R⁸¹ | R⁸² | R⁸³,R⁸⁴,R⁸⁶ R⁸⁷,R⁹⁰,R⁹¹ |
|---|---|---|---|
| 7801 | D1 | H | H |
| 7802 | D2 | H | H |
| 7803 | D3 | H | H |
| 7804 | D4 | H | H |
| 7805 | D5 | H | H |
| 7808 | D6 | H | H |
| 7807 | D7 | H | H |
| 7808 | D8 | H | H |
| 7809 | D9 | H | H |
| 7810 | D10 | H | H |
| 7811 | D11 | H | H |
| 7812 | D12 | H | H |
| 7813 | D13 | H | H |
| 7814 | D14 | H | H |
| 7815 | D15 | H | H |
| 7816 | D18 | H | H |
| 7817 | D17 | H | H |
| 7818 | D18 | H | H |
| 7819 | D19 | H | H |
| 7820 | D20 | H | H |
| 7821 | D21 | H | H |
| 7822 | D22 | H | H |
| 7823 | D23 | H | H |
| 7824 | D24 | H | H |
| 7825 | D25 | H | H |
| 7826 | D26 | H | H |
| 7827 | D27 | H | H |
| 7828 | D28 | H | H |
| 7829 | D29 | H | H |
| 7830 | D30 | H | H |
| 7831 | D31 | H | H |
| 7832 | D32 | H | H |
| 7833 | D33 | H | H |
| 7834 | D34 | H | H |
| 7835 | D35 | H | H |
| 7836 | D38 | H | H |
| 7837 | D37 | H | H |
| 7838 | D38 | H | H |
| 7839 | D39 | H | H |
| 7840 | D40 | H | H |
| 7841 | D41 | H | H |
| 7842 | D42 | H | H |
| 7843 | D43 | H | H |
| 7844 | D44 | H | H |
| 7845 | D45 | H | H |
| 7848 | D48 | H | H |
| 7847 | D47 | H | H |
| 7848 | D48 | H | H |
| 7849 | D49 | H | H |
| 7850 | D50 | H | H |
| 7851 | H | D1 | H |
| 7852 | H | D2 | H |
| 7853 | H | D3 | H |
| 7854 | H | D4 | H |
| 7855 | H | D5 | H |
| 7856 | H | D6 | H |
| 7857 | H | D7 | H |
| 7858 | H | D8 | H |
| 7859 | H | D9 | H |
| 7860 | H | D10 | H |
| 7881 | H | D11 | H |
| 7862 | H | D12 | H |
| 7863 | H | D13 | H |
| 7864 | H | D14 | H |
| 7865 | H | D15 | H |
| 7888 | H | D16 | H |
| 7867 | H | D17 | H |
| 7868 | H | D18 | H |
| 7869 | H | D19 | H |
| 7870 | H | D20 | H |
| 7871 | H | D21 | H |
| 7872 | H | D22 | H |
| 7873 | H | D23 | H |
| 7874 | H | D24 | H |
| 7875 | H | D25 | H |
| 7876 | H | D28 | H |
| 7877 | H | D27 | H |
| 7878 | H | D28 | H |
| 7879 | H | D29 | H |
| 7880 | H | D30 | H |
| 7881 | H | D31 | H |
| 7882 | H | D32 | H |
| 7883 | H | D33 | H |
| 7884 | H | D34 | H |
| 7885 | H | D35 | H |
| 7886 | H | D36 | H |
| 7887 | H | D37 | H |
| 7888 | H | D38 | H |
| 7889 | H | D39 | H |
| 7890 | H | D40 | H |
| 7891 | H | D41 | H |
| 7892 | H | D42 | H |
| 7893 | H | D43 | H |
| 7894 | H | D44 | H |
| 7895 | H | D45 | H |
| 7896 | H | D46 | H |
| 7897 | H | D47 | H |
| 7898 | H | D48 | H |
| 7899 | H | D49 | H |
| 7900 | H | D50 | H |

**[Table 8]**

| Group to be Substituted | | | Substituting Group | | New Compound No. | |
|---|---|---|---|---|---|---|
| To R84 of | Nos. | 7801 to 7850, | the same group as R81 | is introduced to form | Nos. | 7901 to 7950 |
| To R83 of | Nos. | 7851 to 7900, | the same group as R82 | is introduced to form | Nos. | 7951 to 8000 |
| To R90 of | Nos. | 7801 to 8000, | N1 | is introduced to form | Nos. | 8001 to 8200 |
| To R91 of | Nos. | 7801 to 8000, | N1 | is introduced to form | Nos. | 8201 to 8400 |
| To R87 of | Nos. | 7801 to 7900. | N1 | is introduced to form | Nos. | 8401 to 8500 |
| To R86 of | Nos. | 7801 to 7900, | N1 | is introduced to form | Nos. | 8501 to 8600 |
| To R90 and R87 of | Nos. | 7801 to 8000, | N1 | is introduced to form | Nos. | 8601 to 8800 |
| To R91 and R86 of | Nos. | 7801 to 8000, | N1 | is introduced to form | Nos. | 8801 to 9000 |
| To R90 of | Nos. | 7801 to 8000, | A4 | is introduced to form | Nos. | 9001 to 9200 |
| To R91 of | Nos. | 7801 to 8000, | A4 | is introduced to form | Nos. | 9201 to 9400 |
| To R87 of | Nos. | 7801 to 7900, | A4 | is introduced to form | Nos. | 9401 to 9500 |
| To R86 of | Nos. | 7801 to 7900. | A4 | is introduced to form | Nos. | 9501 to 9600 |
| To R90 and R87 of | Nos. | 7801 to 8000, | A4 | is introduced to form | Nos. | 9601 to 9800 |
| To R91 and R86 of | Nos. | 7801 to 8000, | A4 | is introduced to form | Nos. | 9801 to 10000 |
| To R90 of | Nos. | 7801 to 8000, | N5 | is introduced to form | Nos. | 10001 to 10200 |
| To R91 of | Nos. | 7801 to 8000, | N5 | is introduced to form | Nos. | 10201 to 10400 |
| To R87 of | Nos. | 7801 to 7900, | N5 | is introduced to form | Nos. | 10401 to 10500 |
| To R86 of | Nos. | 7801 to 7900, | N5 | is introduced to form | Nos. | 10501 to 10600 |
| To R90 and R87 of | Nos. | 7801 to 8000, | N5 | is introduced to form | Nos. | 10601 to 10800 |
| To R91 and R86 of | Nos. | 7801 to 8000, | N5 | is introduced to form | Nos. | 10801 to 11000 |

**[Table 9]**

| | | | |
|---|---|---|---|
| 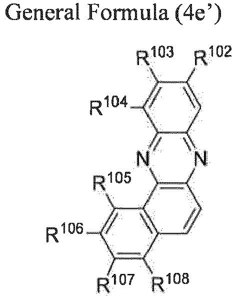 | | | |

| No. | R¹⁰² | R¹⁰³ | R¹⁰⁴∼R¹⁰⁸ |
|---|---|---|---|
| 11001 | D1 | H | H |
| 11002 | D2 | H | H |
| 11003 | D3 | H | H |
| 11004 | D4 | H | H |
| 11005 | D5 | H | H |
| 11006 | D6 | H | H |
| 11007 | D7 | H | H |
| 11008 | D8 | H | H |
| 11009 | D9 | H | H |
| 11010 | D10 | H | H |
| 11011 | D11 | H | H |
| 11012 | D12 | H | H |
| 11013 | D13 | H | H |
| 11014 | D14 | H | H |
| 11015 | D15 | H | H |
| 11016 | D16 | H | H |
| 11017 | D17 | H | H |
| 11018 | D18 | H | H |
| 11019 | D19 | H | H |
| 11020 | D20 | H | H |
| 11021 | D21 | H | H |
| 11022 | D22 | H | H |
| 11023 | D23 | H | H |
| 11024 | D24 | H | H |
| 11025 | D25 | H | H |
| 11026 | D26 | H | H |
| 11027 | D27 | H | H |
| 11028 | D28 | H | H |
| 11029 | D29 | H | H |
| 11030 | D30 | H | H |
| 11031 | D31 | H | H |
| 11032 | D32 | H | H |
| 11033 | D33 | H | H |
| 11034 | D34 | H | H |
| 11035 | D35 | H | H |
| 11036 | D38 | H | H |
| 11037 | D37 | H | H |
| 11038 | D38 | H | H |
| 11039 | D39 | H | H |
| 11040 | D40 | H | H |
| 11041 | D41 | H | H |
| 11042 | D42 | H | H |
| 11043 | D43 | H | H |
| 11044 | D44 | H | H |
| 11045 | D45 | H | H |
| 11048 | D46 | H | H |
| 11047 | D47 | H | H |
| 11048 | D48 | H | H |
| 11049 | D49 | H | H |
| 11050 | D50 | H | H |
| 11051 | H | D1 | H |
| 11052 | H | D2 | H |
| 11053 | H | D3 | H |
| 11054 | H | D4 | H |
| 11055 | H | D5 | H |
| 11056 | H | D6 | H |
| 11057 | H | D7 | H |
| 11058 | H | D8 | H |
| 11059 | H | D9 | H |
| 11080 | H | D10 | H |
| 11081 | H | D11 | H |
| 11082 | H | D12 | H |
| 11083 | H | D13 | H |
| 11084 | H | D14 | H |
| 11085 | H | D15 | H |
| 11066 | H | D16 | H |
| 11087 | H | D17 | H |
| 11088 | H | D18 | H |
| 11089 | H | D19 | H |
| 11070 | H | D20 | H |
| 11071 | H | D21 | H |
| 11072 | H | D22 | H |
| 11073 | H | D23 | H |
| 11074 | H | D24 | H |
| 11075 | H | D25 | H |
| 11078 | H | D26 | H |
| 11077 | H | D27 | H |
| 11078 | H | D28 | H |
| 11079 | H | D29 | H |
| 11080 | H | D30 | H |
| 11081 | H | D31 | H |
| 11082 | H | D32 | H |
| 11083 | H | D33 | H |
| 11084 | H | D34 | H |
| 11085 | H | D35 | H |
| 11088 | H | D38 | H |
| 11087 | H | D37 | H |
| 11088 | H | D38 | H |
| 11089 | H | D39 | H |
| 11090 | H | D40 | H |
| 11091 | H | D41 | H |
| 11092 | H | D42 | H |
| 11093 | H | D43 | H |
| 11094 | H | D44 | H |
| 11095 | H | D45 | H |
| 11098 | H | D46 | H |
| 11097 | H | D47 | H |
| 11098 | H | D48 | H |
| **11099** | H | D49 | H |
| 11100 | H | D50 | H |

**[Table 10]**

| Group to be Substituted | | | Substituting Group | | New Compound No. | |
|---|---|---|---|---|---|---|
| To R103 of | Nos. | 11001 to 11050, | the same group as R102 | is introduced to form | Nos. | 11101 to 11150 |
| To R104 of | Nos. | 11051 to 11100, | the same group as R103 | is introduced to form | Nos. | 11151 to 11200 |
| To R105 of | Nos. | 11001 to 11200, | N1 | is introduced to form | Nos. | 11201 to 11400 |
| To R106 of | Nos. | 11001 to 11200, | N1 | is introduced to form | Nos. | 11401 to 11600 |
| To R107 of | Nos. | 11001 to 11200, | N1 | is introduced to form | Nos. | 11601 to 11800 |
| To R108 of | Nos. | 11001 to 11200, | N1 | is introduced to form | Nos. | 11801 to 12000 |
| To R105 of | Nos. | 11001 to 11200, | A4 | is introduced to form | Nos. | 12001 to 12200 |
| To R106 of | Nos. | 11001 to 11200, | A4 | is introduced to form | Nos. | 12201 to 12400 |
| To R107 of | Nos. | 11001 to 11200, | A4 | is introduced to form | Nos. | 12401 to 12600 |
| To R108 of | Nos. | 11001 to 11200, | A4 | is introduced to form | Nos. | 12601 to 12800 |
| To R105 of | Nos. | 11001 to 11200, | N5 | is introduced to form | Nos. | 12801 to 13000 |
| To R106 of | Nos. | 11001 to 11200, | N5 | is introduced to form | Nos. | 13001 to 13200 |
| To R107 of | Nos. | 11001 to 11200, | N5 | is introduced to form | Nos. | 13201 to 13400 |
| To R108 of | Nos. | 11001 to 11200, | N5 | is introduced to form | Nos. | 13401 to 13600 |

**[Table 11]**

| | | | |
|---|---|---|---|
| 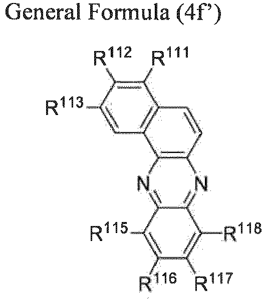 | | | |

| No. | R¹¹² | R¹¹³ | R¹¹¹. R¹¹⁵~R¹¹⁸ |
|---|---|---|---|
| 13601 | D1 | H | H |
| 13602 | D2 | H | H |
| 13603 | D3 | H | H |
| 13604 | D4 | H | H |
| 13605 | D5 | H | H |
| 13606 | D6 | H | H |
| 13607 | D7 | H | H |
| 13608 | D8 | H | H |
| 13609 | D9 | H | H |
| 13610 | D10 | H | H |
| 13611 | D11 | H | H |
| 13612 | D12 | H | H |
| 13813 | D13 | H | H |
| 13814 | D14 | H | H |
| 13815 | D15 | H | H |
| 13616 | D18 | H | H |
| 13817 | D17 | H | H |
| 13618 | D18 | H | H |
| 13619 | D19 | H | H |
| 13620 | D20 | H | H |
| 13621 | D21 | H | H |
| 13622 | D22 | H | H |
| 13823 | D23 | H | H |
| 13624 | D24 | H | H |
| 13825 | D25 | H | H |
| 13828 | D26 | H | H |
| 13827 | D27 | H | H |
| 13828 | D28 | H | H |
| 13829 | D29 | H | H |
| 13630 | D30 | H | H |
| 13631 | D31 | H | H |
| 13632 | D32 | H | H |
| 13833 | D33 | H | H |
| 13634 | D34 | H | H |
| 13635 | D35 | H | H |
| 13838 | D36 | H | H |
| 13637 | D37 | H | H |
| 13838 | D38 | H | H |
| 13838 | D39 | H | H |
| 13640 | D40 | H | H |
| 13641 | D41 | H | H |
| 13842 | D42 | H | H |
| 13643 | D43 | H | H |
| 13644 | D44 | H | H |
| 13645 | D45 | H | H |
| 13646 | D46 | H | H |
| 13847 | D47 | H | H |
| 13648 | D48 | H | H |
| 13649 | D49 | H | H |
| 13650 | D50 | H | H |
| 13651 | H | D1 | H |
| 13652 | H | D2 | H |
| 13653 | H | D3 | H |
| 13654 | H | D4 | H |
| 13655 | H | D5 | H |
| 13656 | H | D8 | H |
| 13657 | H | D7 | H |
| 13658 | H | D8 | H |
| 13659 | H | D9 | H |
| 13660 | H | D10 | H |
| 13661 | H | D11 | H |
| 13662 | H | D12 | H |
| 13663 | H | D13 | H |
| 13664 | H | D14 | H |
| 13665 | H | D15 | H |
| 13888 | H | D16 | H |
| 13667 | H | D17 | H |
| 13668 | H | D18 | H |
| 13669 | H | D19 | H |
| 13670 | H | D20 | H |
| 13671 | H | D21 | H |
| 13672 | H | D22 | H |
| 13673 | H | D23 | H |
| 13674 | H | D24 | H |
| 13675 | H | D25 | H |
| 13676 | H | D26 | H |
| 13677 | H | D27 | H |
| 13678 | H | D28 | H |
| 13679 | H | D29 | H |
| 13680 | H | D30 | H |
| 13681 | H | D31 | H |
| 13682 | H | D32 | H |
| 13683 | H | D33 | H |
| 13684 | H | D34 | H |
| 13685 | H | D35 | H |
| 13888 | H | D36 | H |
| 13687 | H | D37 | H |
| 13888 | H | D38 | H |
| 13689 | H | D39 | H |
| 13690 | H | D40 | H |
| 13691 | H | D41 | H |
| 13692 | H | D42 | H |
| 13693 | H | D43 | H |
| 13694 | H | D44 | H |
| 13695 | H | D45 | H |
| 13696 | H | D46 | H |
| 13697 | H | D47 | H |
| 13698 | H | D48 | H |
| 13699 | H | D49 | H |
| 13700 | H | D50 | H |

**[Table 12]**

| Group to be Substituted | | | Substituting Group | | New Compound No. | |
|---|---|---|---|---|---|---|
| To R113 of | Nos. | 13601 to 13650, | the same group as R112 | is introduced to form | Nos. | 13701 to 13750 |
| To R111 of | Nos. | 13651 to 13700, | the same group as R113 | is introduced to form | Nos. | 13751 to 13800 |
| To R115 of | Nos. | 13601 to 13800, | N1 | is introduced to form | Nos. | 13801 to 14000 |
| To R116 of | Nos. | 13601 to 13800, | N1 | is introduced to form | Nos. | 14001 to 14200 |
| To R117 of | Nos. | 13601 to 13800, | N1 | is introduced to form | Nos. | 14201 to 14400 |
| To R118 of | Nos. | 13601 to 13800, | N1 | is introduced to form | Nos. | 14401 to 14600 |
| To R115 of | Nos. | 13601 to 13800, | A4 | is introduced to form | Nos. | 14601 to 14800 |
| To R116 of | Nos. | 13601 to 13800, | A4 | is introduced to form | Nos. | 14801 to 15000 |
| To R117 of | Nos. | 13601 to 13800. | A4 | is introduced to form | Nos. | 15001 to 15200 |
| To R118 of | Nos. | 13601 to 13800, | A4 | is introduced to form | Nos. | 15201 to 15400 |
| To R115 of | Nos. | 13601 to 13800, | N5 | I is introduced to form | Nos. | 15401 to 15600 |
| To R116 of | Nos. | 13601 to 13800. | N5 | is introduced to form | Nos. | 15601 to 15800 |
| To R117 of | Nos. | 13601 to 13800, | N5 | is introduced to form | Nos. | 15801 to 16000 |
| To R118 of | Nos. | 13601 to 13800, | N5 | is introduced to form | Nos. | 16001 to 16200 |

Compounds formed by substituting all the hydrogen atoms existing in the molecule of Compounds 1 to 22600 with a deuterium atoms are disclosed as Compound 1d to 22600d. In the case where the above-exemplified compounds have rotational isomers, mixtures of rotational isomers and each separated rotational isomer are considered to be disclosed in the present specification.

In one embodiment of the present invention, a compound having an axisymmetric structure is selected as the compound represented by the general formula (1). In one embodiment of the present invention, a compound having an asymmetric structure is selected as the compound represented by the general formula (1).

In one embodiment of the present invention, Compounds 1 to 3200 and 1d to 3200d are selected as the compound represented by the general formula (1). In one embodiment of the present invention, Compounds 3201 to 5800 and 3201d to 5800d are selected as the compound represented by the general formula (1). In one embodiment of the present invention, Compounds 5801 to 7800 and 5801d to 7800d are selected as the compound represented by the general formula (1). In one embodiment of the present invention, Compounds 7801 to 11000 and 7801d to 11000d are selected as the compound represented by the general formula (1). In one embodiment of the present invention, Compounds 11001 to 13600 and 11001d to 13600d are selected as the compound represented by the general formula (1). In one embodiment of the present invention, Compounds 13601 to 16200 and 13601d to 16200d are selected as the compound represented by the general formula (1). In one embodiment of the present invention, Compounds 16201 to 18200 and 16201d to 18200d are selected as the compound represented by the general formula (1). In one embodiment of the present invention, Compounds 18201 to 22600 and 18201d to 22600d are selected as the compound represented by the general formula (1).

In the compound represented by the general formula (1), an acceptor group may not bond to the skeleton of the general formula (1). The acceptor group as referred to herein is a group having a positive Hammett's σp value. The compound represented by the general formula (1) may be one not having a Hammett's σp value of 0.2 or more.

The molecular weight of the compound represented by the general formula (1) is, for example, when an organic layer containing the compound represented by the general formula (1) is intended to be formed by an evaporation method and used, preferably 1500 or less, more preferably 1200 or less, even more preferably 1000 or less, further more preferably 900 or less. The lower limit of the molecular weight is a molecular weight of the smallest compound of the compound group represented by the general formula (1).

The compound represented by the general formula (1) can be formed into a layer by a coating method, irrespective of the molecular weight thereof. According to a coating method, the compound having a relatively large molecular weight can be formed into a layer. The compound represented by the general formula (1) has an advantage that the compound is readily soluble in an organic solvent. Consequently, a coating method is readily applicable to the compound represented by the general formula (1) and, in addition, the compound can be purified to have an increased purity.

By applying the present invention, it is considered that a compound containing plural number of structures represented by the general formula (1) in the molecule can be used as a light-emitting material.

For example, it is considered that a polymerizable group is previously introduced into the structure represented by the general formula (1), and the polymer formed by polymerizing the polymerizable group is used as a light-emitting material. Specifically, it is considered that a monomer containing a polymerizable functional group in any structure represented by the general formula (1) (for example, in any of Ar¹, D, A, and R¹ to R⁴) is prepared, and this is homo-polymerized, or is copolymerized with any other monomer to give a polymer having a repeating unit, and the polymer is used as a light-emitting material. Or it is also considered that compounds of the general formula (1) are coupled to give a dimer or a trimer, and these are used as a light-emitting material.

Examples of the polymer having a repeating unit that contains the structure represented by the general formula (1) include polymers having a structure represented by any of the following two general formulae.

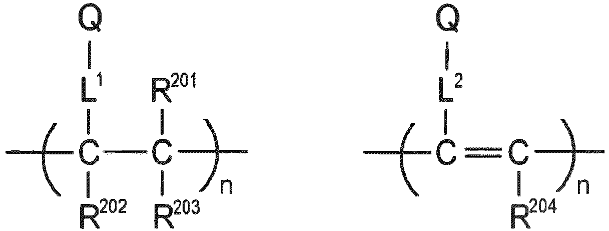

In the above general formulae, Q represents a group containing the structure represented by the general formula (1), L¹ and L² each represent a linking group. The carbon number of the linking group is preferably 0 to 20, more preferably 1 to 15, even more preferably 2 to 10. The linking group preferably has a structure represented by - X¹¹-L¹¹-. Here, X¹¹ represents an oxygen atom or a sulfur atom, and is preferably an oxygen atom. L¹¹ represents a linking group, and is preferably a substituted or unsubstituted alkylene group, or a substituted or unsubstituted arylene group, more preferably a substituted or unsubstituted alkylene group having 1 to 10 carbon atoms, or a substituted or unsubstituted phenylene group.

R²⁰¹, R²⁰², R²⁰³ and R²⁰⁴ each independently represent a substituent. Preferably, they each are a substituted or unsubstituted alkyl group having 1 to 6 carbon atoms, a substituted or unsubstituted alkoxy group having 1 to 6 carbon atoms, or a halogen atom, more preferably an unsubstituted alkyl group having 1 to 3 carbon atoms, an unsubstituted alkoxy group having 1 to 3 carbon atoms, a fluorine atom or a chlorine atom, even more preferably an unsubstituted alkyl group having 1 to 3 carbon atoms or an unsubstituted alkoxy group having 1 to 3 carbon atoms.

The linking group represented by L¹ and L² can bond to any position (for example, any of Ar¹, D, A, and R¹ to R⁴ of the structure represented by the general formula (1) that constitutes Q. Two or more linking groups can bond to one Q to form a crosslinked structure or a network structure.

Examples of specific structures of the repeating unit include structures represented by the following formulae.

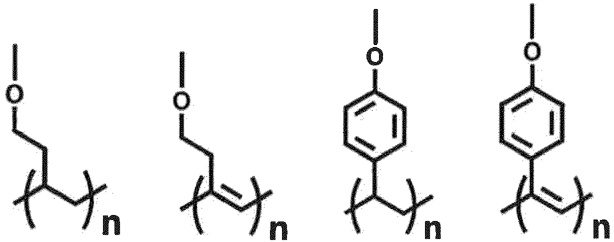

Polymers having a repeating unit that contains any of these formulae can be synthesized by previously introducing a hydroxy group into any structure represented by the general formula (1) (for example, into any of Ar¹, D, A, and R¹ to R⁴), then reacting the group serving as a linker with the following compound to thereby introduce a polymerizable group, and polymerizing the polymerizable group.

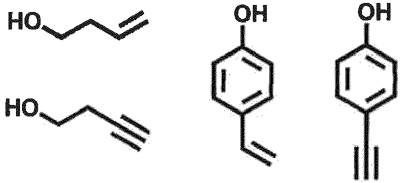

The polymer having a structure represented by the general formula (1) in the molecule can be a polymer having only a repeating unit that has the structure represented by the general formula (1), or can be a polymer containing a repeating unit that has any other structure. The repeating unit having the structure represented by the general formula (1) to be contained in the polymer may be a single kind or two or more kinds. The repeating unit not having the structure of the general formula (1) includes those derived from monomers used in general copolymerization. For example, it includes repeating units derived from monomers having an ethylenically unsaturated bond, such as ethylene or styrene.

Preferably, the compound represented by the general formula (1) does not contain a metal atom. For example, as the compound represented by the general formula (1), a compound formed of atoms selected from the group consisting of a carbon atom, a hydrogen atom, a deuterium atom, a nitrogen atom, an oxygen atom and a sulfur atom can be selected. For example, as the compound represented by the general formula (1), a compound formed of atoms selected from the group consisting of a carbon atom, a hydrogen atom, a deuterium atom, a nitrogen atom, and an oxygen atom can be selected. For example, as the compound represented by the general formula (1), a compound formed of atoms selected from the group consisting of a carbon atom, a hydrogen atom, a deuterium atom, a nitrogen atom, and a sulfur atom can be selected. For example, as the compound represented by the general formula (1), a compound formed of atoms selected from the group consisting of a carbon atom, a hydrogen atom, a deuterium atom, and a nitrogen atom can be selected. For example, as the compound represented by the general formula (1), a compound formed of atoms selected from the group consisting of a carbon atom, a hydrogen atom and a nitrogen atom can be selected.

In the present specification, the "alkyl group" can be linear, branched or cyclic. Two or more of a linear moiety, a cyclic moiety and a branched moiety can exist therein as combined. The carbon number of the alkyl group can be, for example 1 or more, 2 or more, or 4 or more. The carbon number can also be 30 or less, 20 or less, 10 or less, 6 or less, or 4 or less. Specific examples of the alkyl group include a methyl group, an ethyl group, an n-propyl group, an isopropyl group, an n-butyl group, an isobutyl group, a tert-butyl group, an n-pentyl group, an isopentyl group, an n-hexyl group, an isohexyl group, a 2-ethylhexyl group, an n-heptyl group, an isoheptyl group, an n-octyl group, an isooctyl group, an n-nonyl group, an isononyl group, an n-decanyl group, an isodecanyl group, a cyclopentyl group, a cyclohexyl group, and a cycloheptyl group. The alkyl group as a substituent can be further substituted with an aryl group.

The "alkenyl group" can be linear, branched or cyclic. Two or more of a linear moiety, a cyclic moiety and a branched moiety can exist therein as combined. The carbon number of the alkenyl group can be, for example 2 or more, or 4 or more. The carbon number can also be 30 or less, 20 or less, 10 or less, 6 or less, or 4 or less. Specific examples of the alkenyl group include an ethenyl group, an n-propenyl group, an isopropenyl group, an n-butenyl group, an isobutenyl group, an n-pentenyl group, an isopentenyl group, an n-hexenyl group, an isohexenyl group, and a 2-ethylhexenyl group. The alkenyl group as a substituent can be further substituted.

The "aryl group" and the "heteroaryl group" each may be a single ring or may be a condensed ring of two or more kinds of rings. In the case of a condensed ring, the number of the rings that are condensed is preferably 2 to 6, and, for example, can be selected from 2 to 4. Specific examples of the ring include a benzene ring, a pyridine ring, a pyrimidine ring, a triazine ring, a naphthalene ring, an anthracene ring, a phenanthrene ring, a triphenylene ring, a quinoline ring, a pyrazine ring, a quinoxaline ring, and a naphthyridine ring, and the ring can be a condensed ring of these rings. Specific examples of the aryl group or the heteroaryl group include a phenyl group, a 1-naphthyl group, a 2-naphthyl group, a 1-anthracenyl group, a 2-anthracenyl group, a 9-anthracenyl group, a 2-pyridyl group, a 3-pyridyl group, and a 4-pyridyl group. The ring skeleton-constituting atom number of the aryl group is preferably 6 to 40, more preferably 6 to 20, and can be selected within a range of 6 to 14, or can be selected within a range of 6 to 10. The ring skeleton-constituting atom number of the heteroaryl group is preferably 4 to 40, more preferably 5 to 20, and can be selected within a range of 5 to 14, or can be selected within a range of 5 to 10. For the "arylene group" and the "heteroaryl group", the valency number in the description of the aryl group and the heteroaryl group is changed from 1 to 2.

In the present specification, "Substituent Group A" means one or a combination of two or more groups selected from the group consisting of a hydroxy group, a halogen atom (for example, a fluorine atom, a chlorine atom, a bromine atom, an iodine atom), an alkyl group (for example, having 1 to 40 carbon atoms), an alkoxy group (for example, having 1 to 40 carbon atoms), an alkylthio group (for example, having 1 to 40 carbon atoms), an aryl group (for example, having 6 to 30 carbon atoms), an aryloxy group (for example, having 6 to 30 carbon atoms), an arylthio group (for example, having 6 to 30 carbon atoms), a heteroaryl group (for example, having a ring skeleton-constituting atom number of 5 to 30), a heteroaryloxy group (for example, having a ring skeleton-constituting atom number of 5 to 30), a heteroarylthio group (for example, having a ring skeleton-constituting atom number of 5 to 30), an acyl group (for example, having 2 to 40 carbon atoms), an alkenyl group (for example, having 1 to 40 carbon atoms), an alkynyl group (for example, having 1 to 40 carbon atoms), an alkoxycarbonyl group (for example, having 1 to 40 carbon atoms), an aryloxycarbonyl group (for example, having 7 to 40 carbon atoms), a heteroaryloxycarbonyl group (for example, having 6 to 40 carbon atoms), a silyl group (for example, a trialkylsilyl group having 1 to 40 carbon atoms) and a nitro group.

In the present specification, "Substituent Group B" means one or a combination of two or more groups selected from the group consisting of an alkyl group (for example, having 1 to 40 carbon atoms), an alkoxy group (for example, having 1 to 40 carbon atoms), an aryl group (for example, having 6 to 30 carbon atoms), an aryloxy group (for example, having 6 to 30 carbon atoms), a heteroaryl group (for example, having a ring skeleton-constituting atom number of 5 to 30), a heteroaryloxy group (for example, having a ring skeleton-constituting atom number of 5 to 30), a diarylamino group (for example, having 0 to 20 carbon atoms).

In the present specification, "Substituent Group C" means one or a combination of two or more groups selected from the group consisting of an alkyl group (for example, having 1 to 20 carbon atoms), an aryl group (for example, having 6 to 22 carbon atoms), a heteroaryl group (for example, having a ring skeleton-constituting atom number of 5 to 20), and a diarylamino group (for example, having 12 to 20 carbon atoms).

In the present specification, "Substituent Group D" means one or a combination of two or more groups selected from the group consisting of an alkyl group (for example, having 1 to 20 carbon atoms), an aryl group (for example, having 6 to 22 carbon atoms), and a heteroaryl group (for example, having a ring skeleton-constituting atom number of 5 to 20).

In the present specification, "Substituent Group E" means one or a combination of two or more groups selected from the group consisting of an alkyl group (for example, having 1 to 20 carbon atoms), and an aryl group (for example, having 6 to 22 carbon atoms).

In the present specification, the substituent in the case of a description "substituent" or "substituted or unsubstituted" can be selected, for example, from Substituent Group A, or from Substituent Group B, or from Substituent Group C, or from Substituent Group D, or from Substituent Group E.

In some embodiments, the compound represented by the general formula (1) is a light-emitting material.

In some embodiments of the present disclosure, the compound represented by the general formula (1) is a compound capable of emitting delayed fluorescence.

In some embodiments, the compound represented by the general formula (1) is, when excited thermally or by an electronic means, able to emit light in a UV region, emit light of blue, green, yellow or orange in a visible region, or emit light in a red region (e.g., about 420 nm to about 500 nm, about 500 nm to about 600 nm, or about 600 nm to about 700 nm) or in a near IR region.

In some embodiments of the present disclosure, the compound represented by the general formula (1) is, when excited thermally or by an electronic means, able to emit light of red or orange in a visible region (e.g., about 620 nm to about 780 nm, about 650 nm).

In some embodiments of the present disclosure, the compound represented by the general formula (1) is, when excited thermally or by an electronic means, able to emit light of orange or yellow in a visible region (e.g., about 570 nm to about 620 nm, about 590 nm, about 570 nm).

In some embodiments of the present disclosure, the compound represented by the general formula (1) is, when excited thermally or by an electronic means, able to emit light of green in a visible region (e.g., about 490 nm to about 575 nm, about 510 nm).

In some embodiments of the present disclosure, the compound represented by the general formula (1) is, when excited thermally or by an electronic means, able to emit light of blue in a visible region (e.g., about 400 nm to about 490 nm, about 475 nm).

In some embodiments of the present disclosure, the compound represented by the general formula (1) is, when excited thermally or by an electronic means, able to emit light in a UV region (e.g., about 280 to 400 nm).

In some embodiments of the present disclosure, the compound represented by the general formula (1) is, when excited thermally or by an electronic means, able to emit light in an IR region (e.g., about 780 nm to 2 µm).

In some embodiments of the present disclosure, an organic semiconductor device using the compound represented by the general formula (1) can be produced. For example, CMOS (complementary metal-oxide semiconductor) using the compound represented by the general formula (1) can be produced. In some embodiments of the present disclosure, an organic photonic device such as an organic electroluminescent device and a solid-state image sensing device (for example, CMOS image sensor) using the compound represented by the general formula (1) can be produced.

Electronic characteristics of small-molecule chemical substance libraries can be calculated by known *ab initio* quantum chemistry calculation. For example, according to time-dependent density functional theory calculation using 6-31G* as a basis, and a functional group known as Becke's three parameters, Lee-Yang-Parr hybrid functionals, the Hartree-Fock equation (TD-DFT/B3LYP/6-31G*) is analyzed and molecular fractions (parts) having HOMO not lower than a specific threshold value and LUMO not higher than a specific threshold value can be screened, and the calculated triplet state of the parts is more than 2.75 eV.

With that, for example, in the presence of a HOMO energy (for example, ionizing potential) of -6.5 eV or more, a donor part ("D") can be selected. On the other hand, for example, in the presence of a LUMO energy (for example, electron affinity) of-0.5 eV or less, an acceptor part ("A") can be selected. A bridge part ("B") is a strong conjugated system, for example, capable of strictly limiting the acceptor part and the donor part in a specific three-dimensional configuration, and therefore prevents the donor part and the acceptor part from overlapping in the pai-conjugated system.

In some embodiments, a compound library is screened using at least one of the following characteristics.
1. Light emission around a specific wavelength.
2. A triplet state over a calculated specific energy level.
3. ΔE_{ST} value lower than a specific value.
4. Quantum yield more than a specific value.
5. HOMO level.
6. LUMO level.

In some embodiments, the difference (ΔE_{ST}) between the lowest singlet excited state and the lowest triplet excited state at 77 K is less than about 0.5 eV, less than about 0.4 eV, less than about 0.3 eV, less than about 0.2 eV, or less than about 0.1 eV. In some embodiments, ΔE_{ST} value is less than about 0.09 eV, less than about 0.08 eV, less than about 0.07 eV, less than about 0.06 eV, less than about 0.05 eV, less than about 0.04 eV, less than about 0.03 eV, less than about 0.02 eV, or less than about 0.01 eV.

In some embodiments, the compound represented by the general formula (1) shows a quantum yield of more than 25%, for example, about 30%, about 35%, about 40%, about 45%, about 50%, about 55%, about 60%, about 65%, about 70%, about 75%, about 80%, about 85%, about 90%, about 95% or more.

### [Synthesis Method for Compound represented by general formula (1)]

The compound represented by the general formula (1) is a novel compound.

The compound represented by the general formula (1) can be synthesized by combining known reactions. For example, the compound can be synthesized by utilizing ring-closing reaction or by utilizing substitution reaction. Regarding the specific condition for synthesis, reference can be made to Synthesis Examples given hereinunder.

### [Structure Using the Compound Represented by the General Formula (1)]

In some embodiments, the compound represented by the general formula (1) is used along with one or more materials (e.g., small molecules, polymers, metals, metal complexes), by combining them, or by dispersing the compound, or by covalent-bonding with the compound, or by coating with the compound, or by carrying the compound, or by associating with the compound, and solid films or layers are formed. For example, by combining the compound represented by the general formula (1) with an electroactive material, a film can be formed. In some cases, the compound represented by the general formula (1) can be combined with a hole transporting polymer. In some cases, the compound represented by the general formula (1) can be combined with an electron transporting polymer. In some cases, the compound represented by the general formula (1) can be combined with a hole transporting polymer and an electron transporting polymer. In some cases, the compound represented by the general formula (1) can be combined with a copolymer having both a hole transporting moiety and an electron transporting moiety. In the embodiments mentioned above, the electrons and/or the holes formed in a solid film or layer can be interacted with the compound represented by the general formula (1).

### [Film Formation]

In some embodiments, a film containing the compound represented by the general formula (1) can be formed in a wet process. In a wet process, a solution prepared by dissolving a composition containing the compound of the present invention is applied onto a surface, and then the solvent is removed to form a film. The wet process includes a spin coating method, a slit coating method, an ink jet method (a spraying method), a gravure printing method, an offset printing method and flexographic printing method, which, however are not limitative. In the wet process, an appropriate organic solvent capable of dissolving a composition containing the compound of the present invention is selected and used. In some embodiments, a substituent (e.g., an alkyl group) capable of increasing the solubility in an organic solvent can be introduced into the compound contained in the composition.

In some embodiments, a film containing the compound of the present invention can be formed in a dry process. In some embodiments, a vacuum evaporation method is employable as a dry process, which, however, is not limitative. In the case where a vacuum evaporation method is employed, compounds to constitute a film can be co-evaporated from individual evaporation sources, or can be co-evaporated from a single evaporation source formed by mixing the compounds. In the case where a single evaporation source is used, a mixed powder prepared by mixing compound powders can be used, or a compression molded body prepared by compression-molding the mixed powder can be used, or a mixture prepared by heating and melting the constituent compounds and cooling the resulting melt can be used. In some embodiments, by coevaporation under the condition where the evaporation rate (weight reduction rate) of the plural compounds contained in a single evaporation source is the same or is nearly the same, a film having a compositional ratio corresponding to the compositional ratio of the plural compounds contained in the evaporation source can be formed. When plural compounds are mixed in the same compositional ratio as the compositional ratio of the film to be formed to prepare an evaporation source, a film having a desired compositional ratio can be formed in a simplified manner. In some embodiments, the temperature at which the compounds to be co-evaporated have the same weight reduction ratio is specifically defined, and the temperature can be employed as the temperature of coevaporation.

### [Use Examples of Compound of the Present Disclosure]

The compound represented by the general formula (1) is useful as a material for organic light-emitting devices. In particular, the compound is favorably used for organic light-emitting diodes and the like.

### Organic Light-emitting Diode:

One embodiment of the present invention relates to use of the compound represented by the general formula (1) of the present invention as a light-emitting material for organic light-emitting devices. In some embodiments, the compound represented by the general formula (1) of the present invention can be effectively used as a light-emitting material in a light-emitting layer in an organic light-emitting device. In some embodiments, the compound represented by the general formula (1) of the present invention includes delayed fluorescence (delayed fluorescent material) that emits delayed fluorescence. In some embodiments, the present invention provides a delayed fluorescent material having a structure represented by the general formula (1) of the present invention. In some embodiments, the present invention relates to use of the compound represented by the general formula (1) of the present invention as a delayed fluorescent material. In some embodiments, the compound represented by the general formula (1) of the present invention can be used as a host material, and can be used along with one or more light-emitting materials, and the light-emitting material can be a fluorescent material, a phosphorescent material or a TADF. In some embodiments, the compound represented by the general formula (1) can be used as a hole transporting material. In some embodiments, the compound represented by the general formula (1) can be used as an electron transporting material. In some embodiments, the present invention relates to a method of generating delayed fluorescence from the compound represented by the general formula (1). In some embodiments, the organic light-emitting device containing the compound as a light-emitting material emits delayed fluorescence and shows a high light emission efficiency.

In some embodiments, the light-emitting layer contains the compound represented by the general formula (1), and the compound represented by the general formula (1) is aligned in parallel to the substrate. In some embodiments, the substrate is a film-forming surface. In some embodiment, the alignment of the compound represented by the general formula (1) relative to the film-forming surface can have some influence on the propagation direction of light emitted by the aligned compounds, or can determine the direction. In some embodiments, by aligning the propagation direction of light emitted by the compound represented by the general formula (1), the light extraction efficiency from the light-emitting layer can be improved.

One embodiment of the present invention relates to an organic light-emitting device. In some embodiments, the organic light-emitting device includes a light-emitting layer. In some embodiments, the light-emitting layer contains, as a light-emitting material therein, the compound represented by the general formula (1). In some embodiments, the organic light-emitting device is an organic photoluminescent device (organic PL device). In some embodiments, the organic light-emitting device is an organic electroluminescent device (organic EL device). In some embodiments, the compound represented by the general formula (1) assists light irradiation from the other light-emitting materials contained in the light-emitting layer (as a so-called assist dopant). In some embodiments, the compound represented by the general formula (1) contained in the light-emitting layer is in a lowest excited energy level, and is contained between the lowest excited single energy level of the host material contained in the light-emitting layer and the lowest excited singlet energy level of the other light-emitting materials contained in the light-emitting layer.

In some embodiments, the organic photoluminescent device comprises at least one light-emitting layer. In some embodiments, the organic electroluminescent device comprises at least an anode, a cathode, and an organic layer between the anode and the cathode. In some embodiments, the organic layer comprises at least a light-emitting layer. In some embodiments, the organic layer comprises only a light-emitting layer. In some embodiments, the organic layer comprises one or more organic layers in addition to the light-emitting layer. Examples of the organic layer include a hole transporting layer, a hole injection layer, an electron barrier layer, a hole barrier layer, an electron injection layer, an electron transporting layer and an exciton barrier layer. In some embodiments, the hole transporting layer may be a hole injection and transporting layer having a hole injection function, and the electron transporting layer may be an electron injection and transporting layer having an electron injection function. An example of an organic electroluminescent device is shown in Fig. 1.

### Light-emitting Layer:

In some embodiments, the light-emitting layer is a layer where holes and electrons injected from the anode and the cathode, respectively, are recombined to form excitons. In some embodiments, the layer emits light.

In some embodiments, only a light-emitting material is used as the light-emitting layer. In some embodiments, the light-emitting layer contains a light-emitting material and a host material. In some embodiments, the light-emitting material is one or more compounds of the general formula (1). In some embodiments, for improving luminous radiation efficiency of an organic electroluminescent device and an organic photoluminescence device, the singlet exciton and the triplet exciton generated in a light-emitting material is confined inside the light-emitting material. In some embodiments, a host material is used in the light-emitting layer in addition to a light-emitting material therein. In some embodiments, the host material is an organic compound. In some embodiments, the organic compound has an excited singlet energy and an excited triplet energy, and at least one of them is higher than those in the light-emitting material of the present invention. In some embodiments, the singlet exciton and the triplet exciton generated in the light-emitting material of the present invention are confined in the molecules of the light-emitting material of the present invention. In some embodiments, the singlet and triplet excitons are fully confined for improving luminous radiation efficiency. In some embodiments, although high luminous radiation efficiency is still attained, singlet excitons and triplet excitons are not fully confined, that is, a host material capable of attaining high luminous radiation efficiency can be used in the present invention with no specific limitation. In some embodiments, in the light-emitting material in the light-emitting layer of the device of the present invention, luminous radiation occurs. In some embodiments, radiated light includes both fluorescence and delayed fluorescence. In some embodiments, radiated light includes radiated light from a host material. In some embodiments, radiated light is composed of radiated light from a host material. In some embodiments, radiated light includes radiated light from the compound represented by the general formula (1) and radiated light from a host material. In some embodiment, a TADF molecule and a host material are used. In some embodiments, TADF is an assist dopant, of which the excited singlet energy is lower than that of the host material in the light-emitting layer and is higher than that of the light-emitting material in the light-emitting layer.

In the case where the compound of the general formula (1) is used as an assist dopant, various compounds can be employed as a light-emitting material (preferably a fluorescent material). As such light-emitting materials, employable are an anthracene derivative, a tetracene derivative, a naphthacene derivative, a pyrene derivative, a perylene derivative, a chrysene derivative, a rubrene derivative, a coumarin derivative, a pyran derivative, a stilbene derivative, a fluorenone derivative, an anthryl derivative, a pyrromethene derivative, a terphenyl derivative, a terphenylene derivative, a fluoranthene derivative, an amine derivative, a quinacridone derivative, an oxadiazole derivative, a malononitrile derivative, a pyran derivative, a carbazole derivative, a julolidine derivative, a thiazole derivative, and a metal (Al, Zn)-having derivative. These exemplified skeletons can have a substituent, or may not have a substituent. These exemplified skeletons can be combined.

Light-emitting materials that can be used in combination with the assist dopant represented by the general formula (1) are shown below.

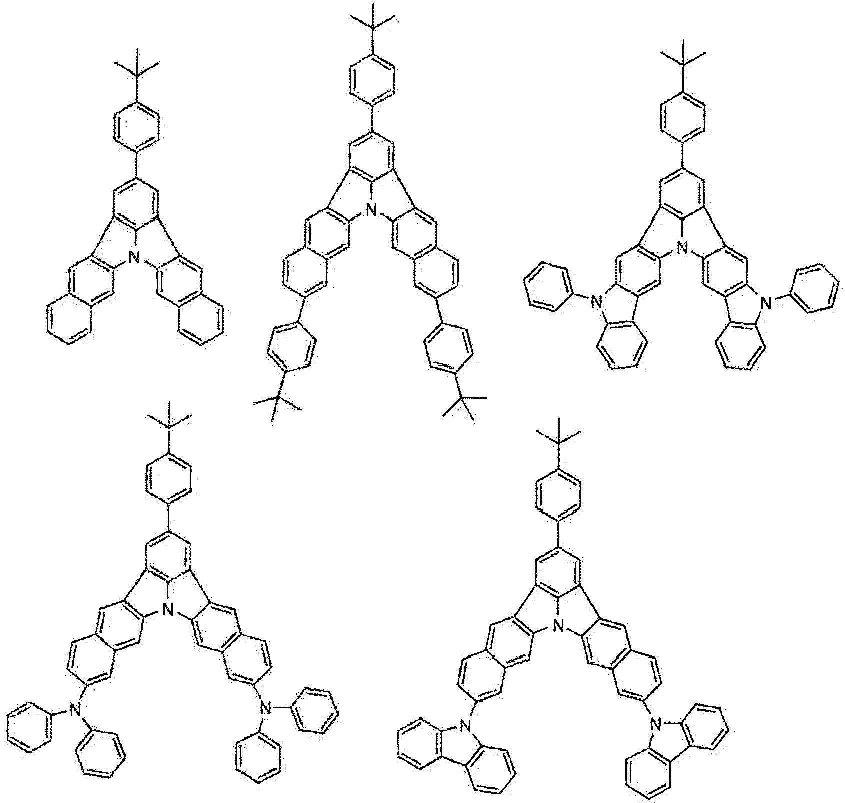

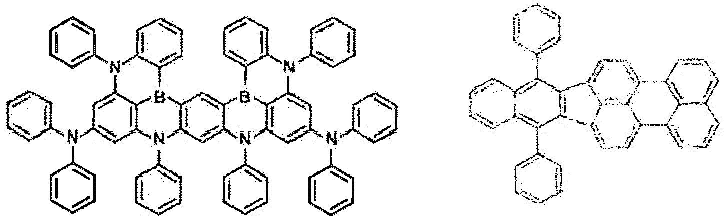

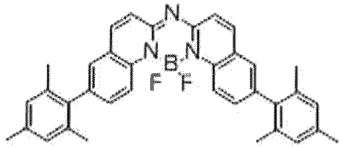

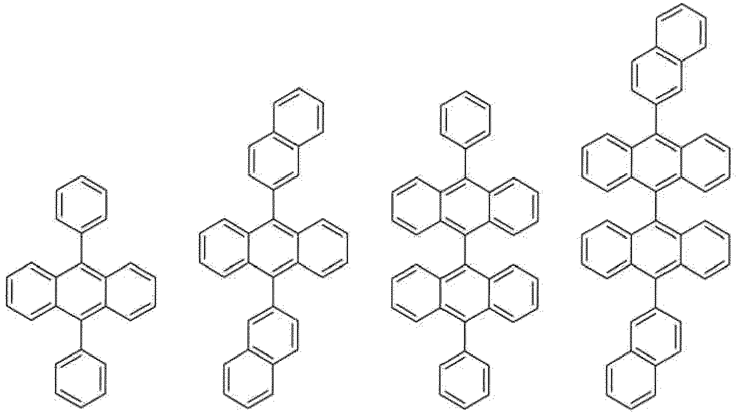

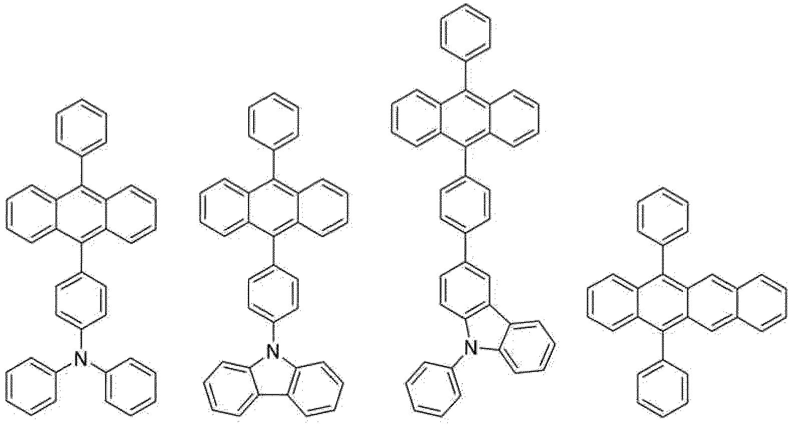

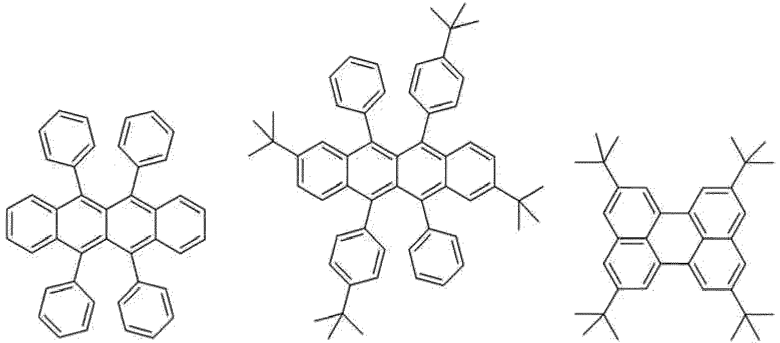

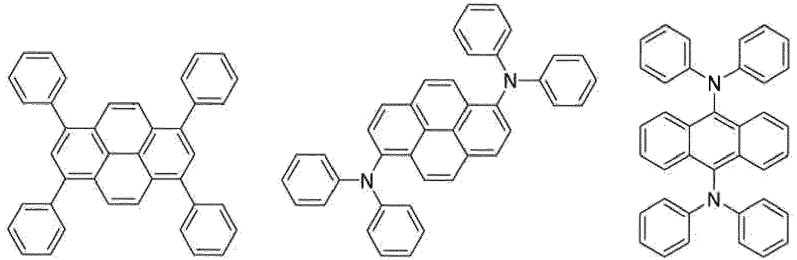

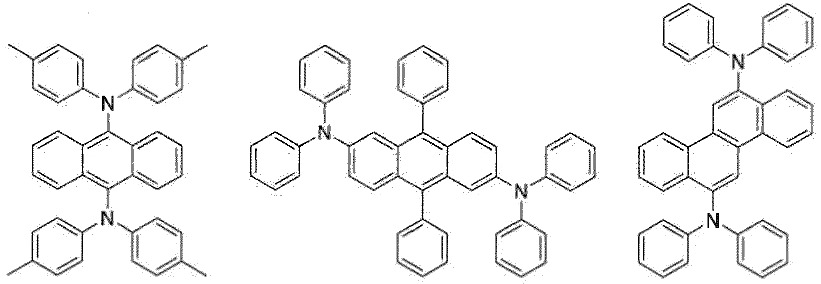

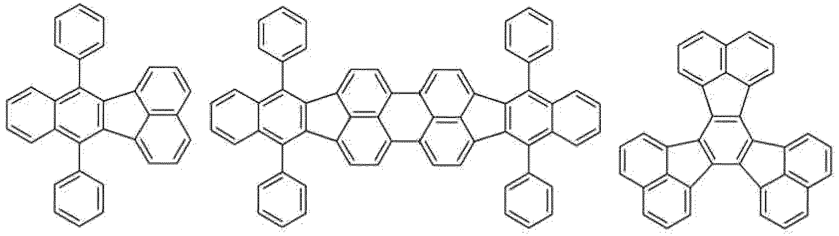

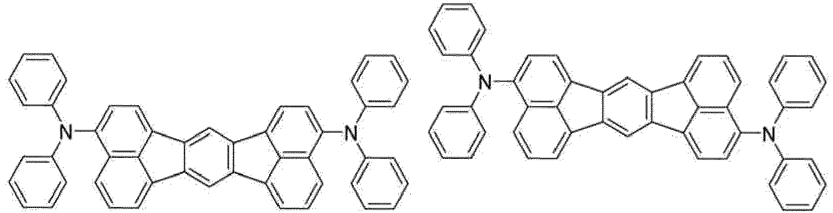

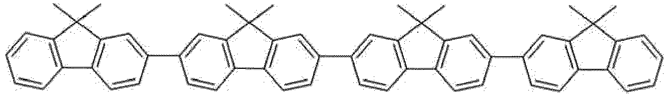

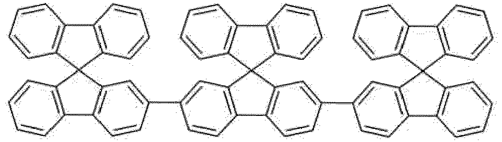

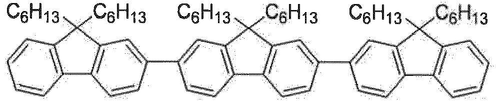

The compounds described in paragraphs 0220 to 0239 in WO2105/022974, and the pyrromethene-boron skeleton-having compounds described in WO2021/015177 are also especially favorably employed as light-emitting materials to be used along with the assist dopant having a structure represented by the general formula (1).

In some embodiments where a host material is used, the amount of the compound of the present invention contained in a light-emitting layer as a light-emitting material is 0.1% by weight or more. In some embodiments where a host material is used, the amount of the compound of the present invention contained in a light-emitting layer as a light-emitting material is 1% by weight or more. In some embodiments where a host material is used, the amount of the compound of the present invention contained in a light-emitting layer as a light-emitting material is 50% by weight or less. In some embodiments where a host material is used, the amount of the compound of the present invention contained in a light-emitting layer as a light-emitting material is 20% by weight or less. In some embodiments where a host material is used, the amount of the compound of the present invention contained in a light-emitting layer as a light-emitting material is 10% by weight or less.

In some embodiments, the host material in a light-emitting layer is an organic compound having a hole transporting capability and an electron transporting capability. In some embodiments, the host material in a light-emitting layer is an organic compound that prevents increase in the wavelength of emitted light. In some embodiments, the host material in a light-emitting layer is an organic compound having a high glass transition temperature.

In some embodiments, the host material is selected from the following group:

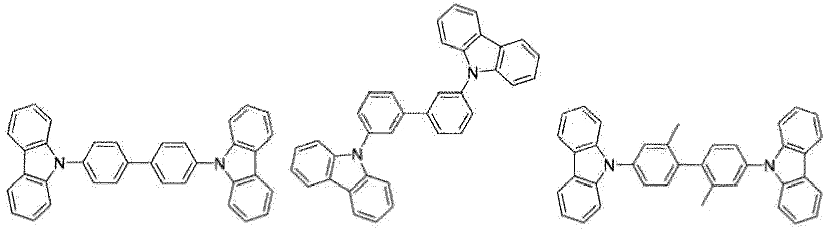

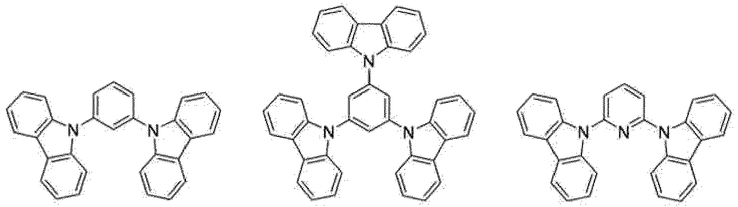

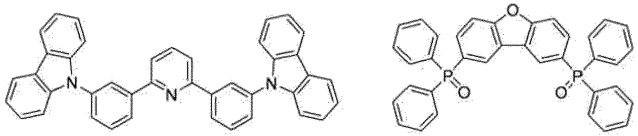

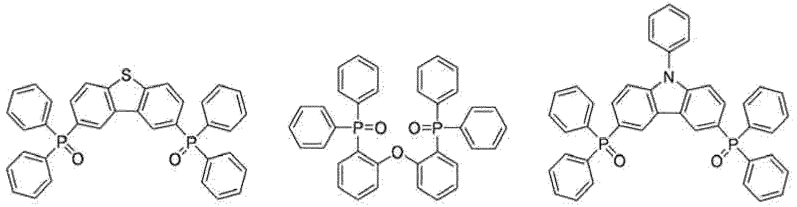

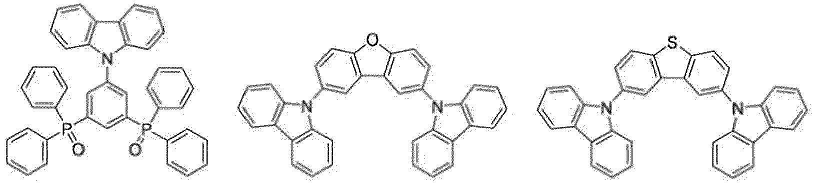

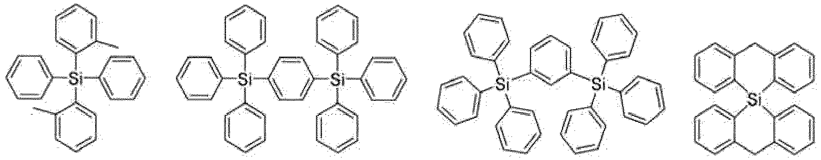

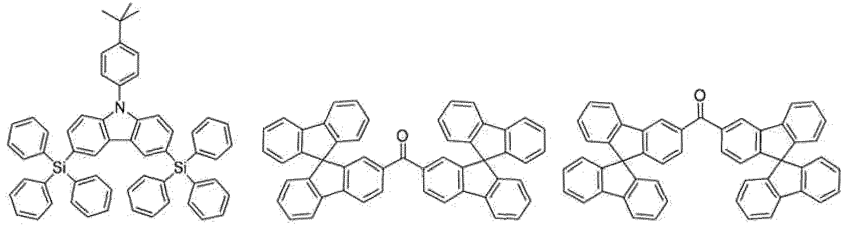

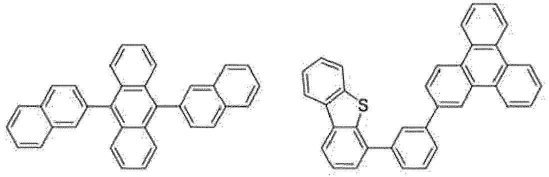

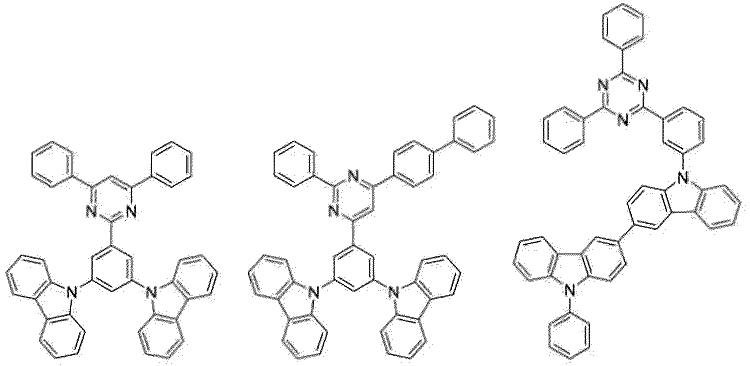

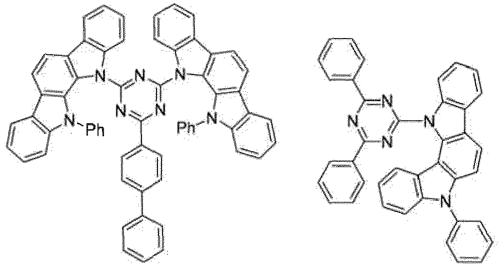

In some embodiments, the light-emitting layer contains two or more kinds of TADF molecules differing in the structure. For example, the light-emitting layer can contain three kinds of materials of a host material, a first TADF molecule and a second TADF molecule whose excited singlet energy level is higher in that order. In that case, both the first TADF molecule and the second TADF molecule are preferably such that the difference ΔE_{ST} between the lowest excited single energy level and the lowest excited triplet energy level at 77 K is 0.3 eV or less, more preferably 0.25 eV or less, even more preferably 0.2 eV or less, further more preferably 0.15 eV or less, further more preferably 0.1 eV or less, further more preferably 0.07 eV or less, further more preferably 0.05 eV or less, further more preferably 0.03 eV or less, further more preferably 0.01 eV or less. The content of the first TADF molecule in the light-emitting layer is preferably larger than the content of the second TADF molecule therein. The content of the host material in the light-emitting layer is preferably larger than the content of the second TADF molecule therein. The content of the first TADF molecule in the light-emitting layer can be larger than or can be smaller than or can be the same as the content of the host material therein. In some embodiments, the composition in the light-emitting layer can be 10 to 70% by weight of a host material, 10 to 80% by weight of a first TADF molecule, and 0.1 to 30% by weighty of a second TADF molecule. In some embodiments, the composition in the light-emitting layer can be 20 to 45% by weight of a host material, 50 go 75% by weight of a first TADF molecule, and 5 to 20% by weighty of a second TADF molecule. In some embodiments, the emission quantum yield ϕPL1(A) by photo-excitation of a co-deposited film of a first TADF molecule and a host material (the content of the first TADF molecule in the co-deposited film = A% by weight) and the emission quantum yield ϕPL2(A) by photo-excitation of a co-deposited film of a second TADF molecule and a host material (the content of the second TADF molecule in the co-deposited film = A% by weight) satisfy a relational formula ϕPL1(A)>ϕPL2(A). In some embodiments, the emission quantum yield ϕPL2(B) by photo-excitation of a co-deposited film of a second TADF molecule and a host material (the content of the second TADF molecule in the co-deposited film = B% by weight) and the emission quantum yield ϕPL2(100) by photo-excitation of a single film of a second TADF molecule satisfy a relational formula ϕPL2(B)>ϕPL2(100). In some embodiments, the light-emitting layer can contain three kinds of TADF molecules differing in the structure. The compound of the present invention can be any of the plural TADF compounds contained in the light-emitting layer.

In some embodiments, the light-emitting layer can be composed of materials selected from the group consisting of a host material an assist dopant and a light-emitting material. In some embodiments, the light-emitting layer does not contain a metal element. In some embodiments, the light-emitting layer can be formed of a material composed of atoms alone selected from the group consisting of a carbon atom, a hydrogen atom, a deuterium atom, a nitrogen atom, an oxygen atom and a sulfur atom. Or the light-emitting layer can be formed of a material composed of atoms alone selected from the group consisting of a carbon atom, a hydrogen atom, a deuterium atom, a nitrogen atom and an oxygen atom. Or the light-emitting layer can be formed of a material composed of atoms alone selected from the group consisting of a carbon atom, a hydrogen atom, a nitrogen atom and an oxygen atom.

In the case where the light-emitting layer contain any other TADF material than the compound of the present invention, the TADF material can be a known delayed fluorescent material. As preferred delayed fluorescent materials, there can be mentioned compounds included in the general formulae described in WO2013/154064, paragraphs 0008 to 0048 and 0095 to 0133; WO2013/011954, paragraphs 0007 to 0047 and 0073-0085; WO2013/011955, paragraphs 0007 to 0033 and 0059 to 0066; WO2013/081088, paragraphs 0008 to 0071 and 0118 to 0133; JP 2013-256490 A, paragraphs 0009 to 0046 and 0093 to 0134; JP 2013-116975 A, paragraphs 0008 to 0020 and 0038 to 0040; WO2013/133359, paragraphs 0007 to 0032 and 0079 to 0084; WO2013/161437, paragraphs 0008 to 0054 and 0101-0121; JP 2014-9352 A, paragraphs 0007 to 0041 and 0060 to 0069; and JP 2014-9224 A, paragraphs 0008 to 0048 and 0067 to 0076; JP 2017-119663 A, paragraphs 0013 to 0025; JP 2017-119664 A, paragraphs 0013 to 0026; JP 2017-222623 A, paragraphs 0012 to 0025; JP 2017-226838 A, paragraphs 0010 to 0050; JP2018-100411 A, paragraphs 0012 to 0043; WO2018/047853, paragraphs 0016 to 0044; and especially, exemplary compounds therein capable of emitting delayed fluorescence. In addition, also preferably employable here are light-emitting materials capable of emitting delayed fluorescence, as described in JP 2013-253121 A, WO2013/133359, WO2014/034535, WO2014/115743, WO2014/122895, WO2014/126200, WO2014/136758, WO2014/133121, WO2014/136860, WO2014/196585, WO2014/189122, WO2014/168101, WO2015/008580, WO2014/203840, WO2015/002213, WO2015/016200, WO2015/019725, WO2015/072470, WO2015/108049, WO2015/080182, WO2015/072537, WO2015/080183, JP 2015-129240 A, WO2015/129714, WO2015/129715, WO2015/133501, WO2015/136880, WO2015/137244, WO2015/137202, WO2015/137136, WO2015/146541 and WO2015/159541. These patent publications described in this paragraph are hereby incorporated as a part of this description by reference.

In the following, the constituent members and the other layers than the light-emitting layer of the organic electroluminescent device are described.

### Substrate:

In some embodiments, the organic electroluminescent device of the invention is supported by a substrate, wherein the substrate is not particularly limited and may be any of those that have been commonly used in an organic electroluminescent device, for example those formed of glass, transparent plastics, quartz and silicon.

### Anode:

In some embodiments, the anode of the organic electroluminescent device is made of a metal, an alloy, an electroconductive compound, or a combination thereof. In some embodiments, the metal, alloy, or electroconductive compound has a large work function (4 eV or more). In some embodiments, the metal is Au. In some embodiments, the electroconductive transparent material is selected from CuI, indium tin oxide (ITO), SnO₂, and ZnO. In some embodiments, an amorphous material capable of forming a transparent electroconductive film, such as IDIXO (In₂O₃-ZnO), is be used. In some embodiments, the anode is a thin film. In some embodiments the thin film is made by vapor deposition or sputtering. In some embodiments, the film is patterned by a photolithography method. In some embodiments, where the pattern may not require high accuracy (for example, approximately 100 µm or more), the pattern may be formed with a mask having a desired shape on vapor deposition or sputtering of the electrode material. In some embodiments, when a material can be applied as a coating, such as an organic electroconductive compound, a wet film forming method, such as a printing method and a coating method is used. In some embodiments, when the emitted light goes through the anode, the anode has a transmittance of more than 10%, and the anode has a sheet resistance of several hundred Ohm per square or less. In some embodiments, the thickness of the anode is from 10 to 1,000 nm. In some embodiments, the thickness of the anode is from 10 to 200 nm. In some embodiments, the thickness of the anode varies depending on the material used.

### Cathode

In some embodiments, the cathode is made of an electrode material a metal having a small work function (4 eV or less) (referred to as an electron injection metal), an alloy, an electroconductive compound, or a combination thereof. In some embodiments, the electrode material is selected from sodium, a sodium-potassium alloy, magnesium, lithium, a magnesium-cupper mixture, a magnesium-silver mixture, a magnesium-aluminum mixture, a magnesium-indium mixture, an aluminum-aluminum oxide (Al₂O₃) mixture, indium, a lithium-aluminum mixture, and a rare earth metal. In some embodiments, a mixture of an electron injection metal and a second metal that is a stable metal having a larger work function than the electron injection metal is used. In some embodiments, the mixture is selected from a magnesium-silver mixture, a magnesium-aluminum mixture, a magnesium-indium mixture, an aluminum-aluminum oxide (Al₂O₃) mixture, a lithium-aluminum mixture, and aluminum. In some embodiments, the mixture increases the electron injection property and the durability against oxidation. In some embodiments, the cathode is produced by forming the electrode material into a thin film by vapor deposition or sputtering. In some embodiments, the cathode has a sheet resistance of several hundred Ohm per square or less. In some embodiments, the thickness of the cathode ranges from 10 nm to 5 µm. In some embodiments, the thickness of the cathode ranges from 50 to 200 nm. In some embodiments, for transmitting the emitted light, any one of the anode and the cathode of the organic electroluminescent device is transparent or translucent. In some embodiments, the transparent or translucent electroluminescent devices enhances the light emission luminance.

In some embodiments, the cathode is formed with an electroconductive transparent material, as described for the anode, to form a transparent or translucent cathode. In some embodiments, a device comprises an anode and a cathode, both being transparent or translucent.

### Injection Layer

An injection layer is a layer between the electrode and the organic layer. In some embodiments, the injection layer decreases the driving voltage and enhances the light emission luminance. In some embodiments the injection layer includes a hole injection layer and an electron injection layer. The injection layer can be positioned between the anode and the light-emitting layer or the hole transporting layer, and between the cathode and the light-emitting layer or the electron transporting layer. In some embodiments, an injection layer is present. In some embodiments, no injection layer is present.

Preferred compound examples for use as a hole injection material are shown below.

### MoO₃,

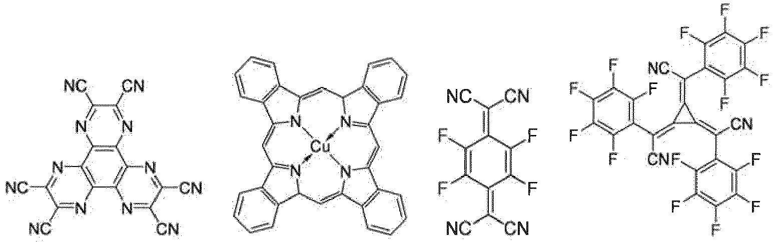

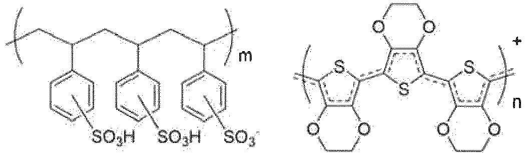

Next, preferred compound examples for use as an electron injection material are shown below.

LiF, CsF,

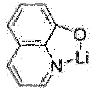

### Barrier Layer

A barrier layer is a layer capable of inhibiting charges (electrons or holes) and/or excitons present in the light-emitting layer from being diffused outside the light-emitting layer. In some embodiments, the electron barrier layer is between the light-emitting layer and the hole transporting layer, and inhibits electrons from passing through the light-emitting layer toward the hole transporting layer. In some embodiments, the hole barrier layer is between the light-emitting layer and the electron transporting layer, and inhibits holes from passing through the light-emitting layer toward the electron transporting layer. In some embodiments, the barrier layer inhibits excitons from being diffused outside the light-emitting layer. In some embodiments, the electron barrier layer and the hole barrier layer are exciton barrier layers. As used herein, the term "electron barrier layer" or "exciton barrier layer" includes a layer that has the functions of both electron barrier layer and of an exciton barrier layer.

### Hole Barrier Layer

A hole barrier layer acts as an electron transporting layer. In some embodiments, the hole barrier layer inhibits holes from reaching the electron transporting layer while transporting electrons. In some embodiments, the hole barrier layer enhances the recombination probability of electrons and holes in the light-emitting layer. The material for the hole barrier layer may be the same materials as the ones described for the electron transporting layer.

Preferred compound examples for use for the hole barrier layer are shown below.

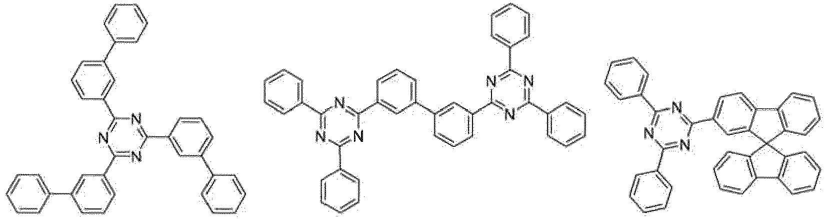

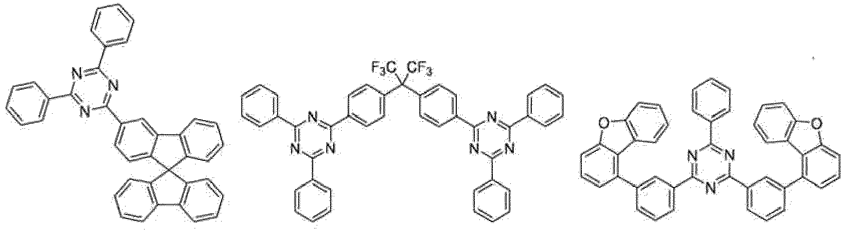

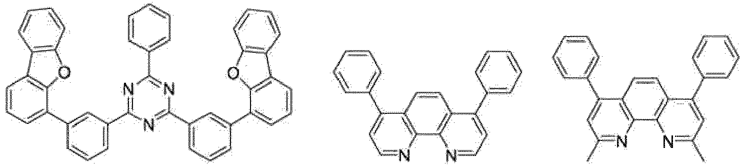

### Electron Barrier Layer

As electron barrier layer transports holes. In some embodiments, the electron barrier layer inhibits electrons from reaching the hole transporting layer while transporting holes. In some embodiments, the electron barrier layer enhances the recombination probability of electrons and holes in the light-emitting layer.

Preferred compound examples for use as the electron barrier material are shown below.

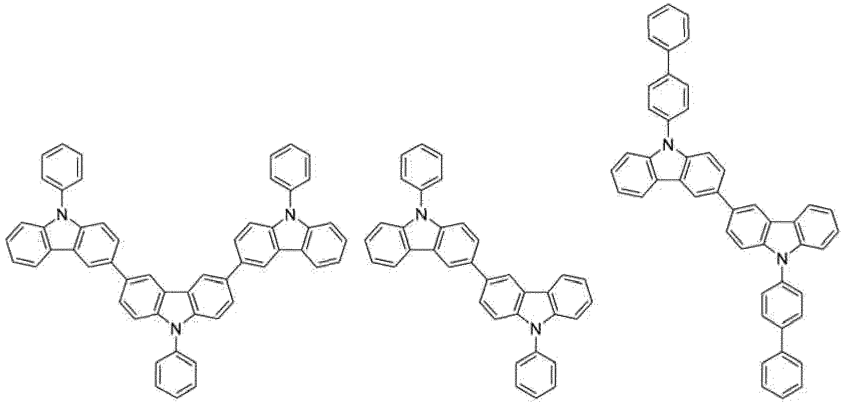

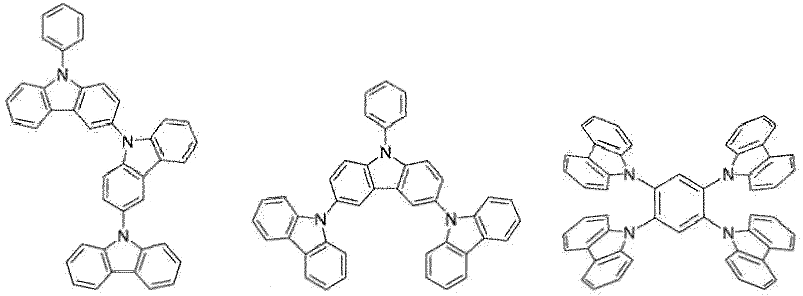

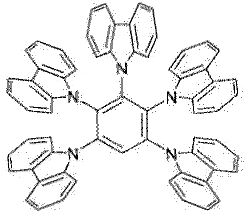

### Exciton Barrier Layer

An exciton barrier layer inhibits excitons generated through recombination of holes and electrons in the light-emitting layer from being diffused to the charge transporting layer. In some embodiments, the exciton barrier layer enables effective confinement of excitons in the light-emitting layer. In some embodiments, the light emission efficiency of the device is enhanced. In some embodiments, the exciton barrier layer is adjacent to the light-emitting layer on any of the side of the anode and the side of the cathode, and on both the sides. In some embodiments, where the exciton barrier layer is on the side of the anode, the layer can be between the hole transporting layer and the light-emitting layer and adjacent to the light-emitting layer. In some embodiments, where the exciton barrier layer is on the side of the cathode, the layer can be between the light-emitting layer and the cathode and adjacent to the light-emitting layer. In some embodiments, a hole injection layer, an electron barrier layer, or a similar layer is between the anode and the exciton barrier layer that is adjacent to the light-emitting layer on the side of the anode. In some embodiments, a hole injection layer, an electron barrier layer, a hole barrier layer, or a similar layer is between the cathode and the exciton barrier layer that is adjacent to the light-emitting layer on the side of the cathode. In some embodiments, the exciton barrier layer comprises excited singlet energy and excited triplet energy, at least one of which is higher than the excited singlet energy and the excited triplet energy of the light-emitting material, respectively.

### Hole Transporting Layer

The hole transporting layer comprises a hole transporting material. In some embodiments, the hole transporting layer is a single layer. In some embodiments, the hole transporting layer comprises a plurality layers.

In some embodiments, the hole transporting material has one of injection or transporting property of holes and barrier property of electrons. In some embodiments, the hole transporting material is an organic material. In some embodiments, the hole transporting material is an inorganic material. Examples of known hole transporting materials that may be used herein include but are not limited to a triazole derivative, an oxadiazole derivative, an imidazole derivative, a carbazole derivative, an indolocarbazole derivative, a polyarylalkane derivative, a pyrazoline derivative, a pyrazolone derivative, a phenylenediamine derivative, an arylamine derivative, an amino-substituted chalcone derivative, an oxazole derivative, a styrylanthracene derivative, a fluorenone derivative, a hydrazone derivative, a stilbene derivative, a silazane derivative, an aniline copolymer and an electroconductive polymer oligomer, particularly a thiophene oligomer, or a combination thereof. In some embodiments, the hole transporting material is selected from a porphyrin compound, an aromatic tertiary amine compound, and a styrylamine compound. In some embodiments, the hole transporting material is an aromatic tertiary amine compound. Preferred compound examples for use as the hole transporting material are shown below.

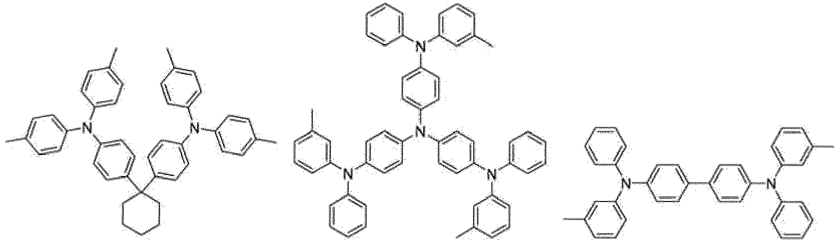

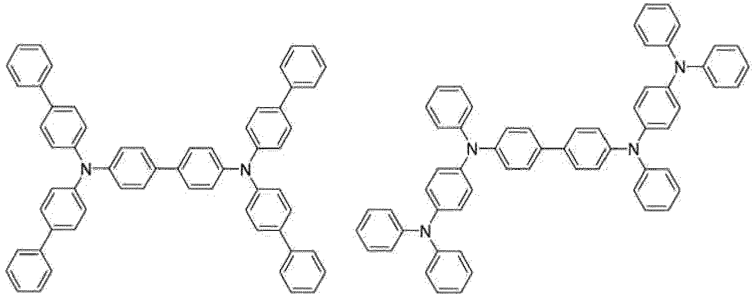

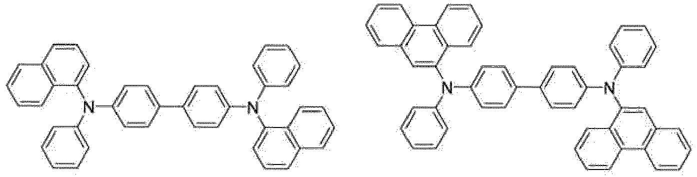

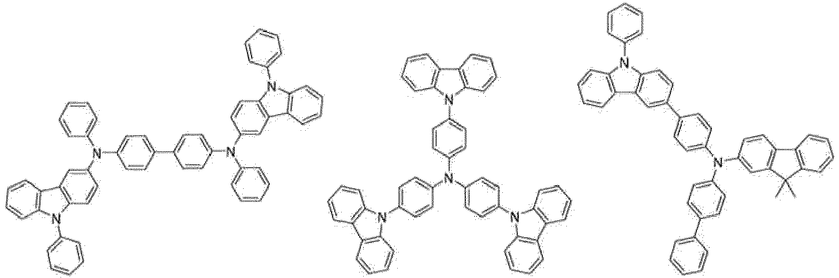

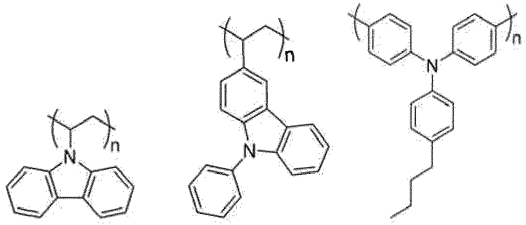

### Electron Transporting Layer

The electron transporting layer comprises an electron transporting material. In some embodiments, the electron transporting layer is a single layer. In some embodiments, the electron transporting layer comprises a plurality of layer.

In some embodiments, the electron transporting material needs only to have a function of transporting electrons, which are injected from the cathode, to the light-emitting layer. In some embodiments, the electron transporting material also function as a hole barrier material. Examples of the electron transporting layer that may be used herein include but are not limited to a nitro-substituted fluorene derivative, a diphenylquinone derivative, a thiopyran dioxide derivative, carbodiimide, a fluorenylidene methane derivative, anthraquinodimethane, an anthrone derivatives, an azole derivative, an azine derivative, an oxadiazole derivative, or a combination thereof, or a polymer thereof. In some embodiments, the electron transporting material is a thiadiazole derivative, or a quinoxaline derivative. In some embodiments, the electron transporting material is a polymer material. Preferred compound examples for use as the electron transporting material are shown below.

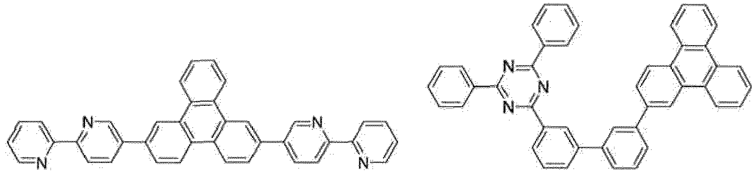

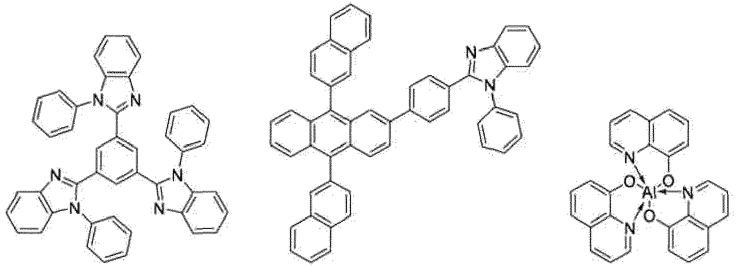

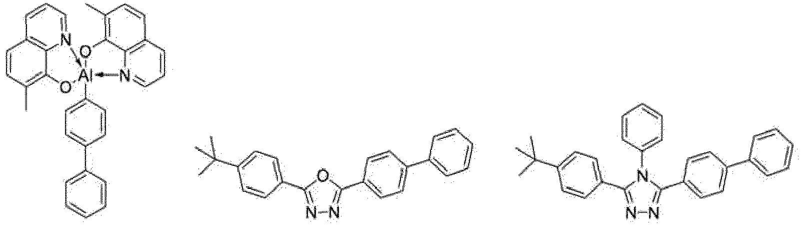

Hereinunder compound examples preferred as a material that can be added to the organic layers are shown. For example, these can be added as a stabilization material.

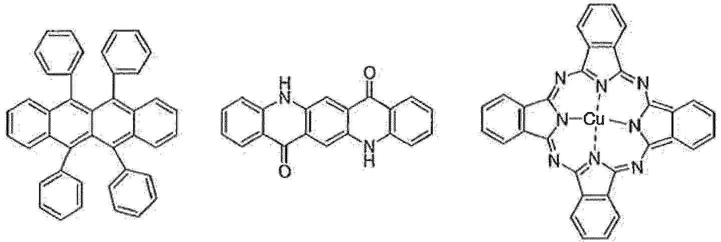

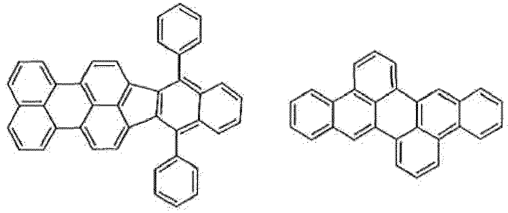

Preferred materials for use in the organic electroluminescent device are specifically shown. However, the materials usable in the invention should not be limitatively interpreted by the following exemplary compounds. Compounds that are exemplified as materials having a specific function can also be used as materials having any other function.

### Devices

In some embodiments, an light-emitting layer is incorporated into a device. For example, the device includes, but is not limited to an OLED bulb, an OLED lamp, a television screen, a computer monitor, a mobile phone, and a tablet.

In some embodiments, an electronic device comprises an OLED comprising an anode, a cathode, and at least one organic layer comprising a light-emitting layer between the anode and the cathode.

In some embodiments, compositions described herein may be incorporated into various light-sensitive or light-activated devices, such as a OLEDs or photovoltaic devices. In some embodiments, the composition may be useful in facilitating charge transfer or energy transfer within a device and/or as a hole-transport material. The device may be, for example, an organic light-emitting diode (OLED), an organic integrated circuit (O-IC), an organic field-effect transistor (O-FET), an organic thin-film transistor (O-TFT), an organic light-emitting transistor (O-LET), an organic solar cell (O-SC), an organic optical detector, an organic photoreceptor, an organic field-quench device (O-FQD), a light-emitting electrochemical cell (LEC) or an organic laser diode (O-laser).

### Bulbs or Lamps

In some embodiments, an electronic device comprises an OLED comprising an anode, a cathode, and at least one organic layer comprising a light-emitting layer between the anode and the cathode.

In some embodiments, a device comprises OLEDs that differ in color. In some embodiments, a device comprises an array comprising a combination of OLEDs. In some embodiments, the combination of OLEDs is a combination of three colors (e.g., RGB). In some embodiments, the combination of OLEDs is a combination of colors that are not red, green, or blue (for example, orange and yellow green). In some embodiments, the combination of OLEDs is a combination of two, four, or more colors.

In some embodiments, a device is an OLED light comprising:
a circuit board having a first side with a mounting surface and an opposing second side, and defining at least one aperture;
at least one OLED on the mounting surface, the at least one OLED configured to emanate light, comprising:
   an anode, a cathode, and at least one organic layer comprising a light-emitting layer between the anode and the cathode;
a housing for the circuit board; and
at least one connector arranged at an end of the housing, the housing and the connector defining a package adapted for installation in a light fixture.

In some embodiments, the OLED light comprises a plurality of OLEDs mounted on a circuit board such that light emanates in a plurality of directions. In some embodiments, a portion of the light emanated in a first direction is deflected to emanate in a second direction. In some embodiments, a reflector is used to deflect the light emanated in a first direction.

### Displays or Screens

In some embodiments, the compounds of the invention can be used in a screen or a display. In some embodiments, the compounds of the invention are deposited onto a substrate using a process including, but not limited to, vacuum evaporation, deposition, vapor deposition, or chemical vapor deposition (CVD). In some embodiments, the substrate is a photoplate structure useful in a two-sided etch provides a unique aspect ratio pixel. The screen (which may also be referred to as a mask) is used in a process in the manufacturing of OLED displays. The corresponding artwork pattern design facilitates a very steep and narrow tie-bar between the pixels in the vertical direction and a large, sweeping bevel opening in the horizontal direction. This allows the close patterning of pixels needed for high definition displays while optimizing the chemical deposition onto a TFT backplane.

The internal patterning of the pixel allows the construction of a 3-dimensional pixel opening with varying aspect ratios in the horizontal and vertical directions. Additionally, the use of imaged "stripes" or halftone circles within the pixel area inhibits etching in specific areas until these specific patterns are undercut and fall off the substrate. At that point the entire pixel area is subjected to a similar etch rate but the depths are varying depending on the halftone pattern. Varying the size and spacing of the halftone pattern allows etching to be inhibited at different rates within the pixel allowing for a localized deeper etch needed to create steep vertical bevels.

A preferred material for the deposition mask is invar. Invar is a metal alloy that is cold rolled into long thin sheet in a steel mill. Invar cannot be electrodeposited onto a rotating mandrel as the nickel mask. A preferred and more cost feasible method for forming the open areas in the mask used for deposition is through a wet chemical etching.

In some embodiments, a screen or display pattern is a pixel matrix on a substrate. In some embodiments, a screen or display pattern is fabricated using lithography (e.g., photolithography and e-beam lithography). In some embodiments, a screen or display pattern is fabricated using a wet chemical etch. In further embodiments, a screen or display pattern is fabricated using plasma etching.

### Methods of Manufacturing Devices

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

An OLED display is generally manufactured by forming a large mother panel and then cutting the mother panel in units of cell panels. In general, each of the cell panels on the mother panel is formed by forming a thin film transistor (TFT) including an active layer and a source/drain electrode on a base substrate, applying a planarization film to the TFT, and sequentially forming a pixel electrode, a light-emitting layer, a counter electrode, and an encapsulation layer, and then is cut from the mother panel.

In another aspect, provided herein is a method of manufacturing an organic light-emitting diode (OLED) display, the method comprising:
forming a barrier layer on a base substrate of a mother panel;
forming a plurality of display units in units of cell panels on the barrier layer;
forming an encapsulation layer on each of the display units of the cell panels;
applying an organic film to an interface portion between the cell panels.

In some embodiments, the barrier layer is an inorganic film formed of, for example, SiNx, and an edge portion of the barrier layer is covered with an organic film formed of polyimide or acryl. In some embodiments, the organic film helps the mother panel to be softly cut in units of the cell panel.

In some embodiments, the thin film transistor (TFT) layer includes a light-emitting layer, a gate electrode, and a source/drain electrode. Each of the plurality of display units may include a thin film transistor (TFT) layer, a planarization film formed on the TFT layer, and a light-emitting unit formed on the planarization film, wherein the organic film applied to the interface portion is formed of a same material as a material of the planarization film and is formed at a same time as the planarization film is formed. In some embodiments, a light-emitting unit is connected to the TFT layer with a passivation layer and a planarization film therebetween and an encapsulation layer that covers and protects the light-emitting unit. In some embodiments of the method of manufacturing, the organic film contacts neither the display units nor the encapsulation layer.

Each of the organic film and the planarization film may include any one of polyimide and acryl. In some embodiments, the barrier layer may be an inorganic film. In some embodiments, the base substrate may be formed of polyimide. The method may further include, before the forming of the barrier layer on one surface of the base substrate formed of polyimide, attaching a carrier substrate formed of a glass material to another surface of the base substrate, and before the cutting along the interface portion, separating the carrier substrate from the base substrate. In some embodiments, the OLED display is a flexible display.

In some embodiments, the passivation layer is an organic film disposed on the TFT layer to cover the TFT layer. In some embodiments, the planarization film is an organic film formed on the passivation layer. In some embodiments, the planarization film is formed of polyimide or acryl, like the organic film formed on the edge portion of the barrier layer. In some embodiments, the planarization film and the organic film are simultaneously formed when the OLED display is manufactured. In some embodiments, the organic film may be formed on the edge portion of the barrier layer such that a portion of the organic film directly contacts the base substrate and a remaining portion of the organic film contacts the barrier layer while surrounding the edge portion of the barrier layer.

In some embodiments, the light-emitting layer includes a pixel electrode, a counter electrode, and an organic light-emitting layer disposed between the pixel electrode and the counter electrode. In some embodiments, the pixel electrode is connected to the source/drain electrode of the TFT layer.

In some embodiments, when a voltage is applied to the pixel electrode through the TFT layer, an appropriate voltage is formed between the pixel electrode and the counter electrode, and thus the organic light-emitting layer emits light, thereby forming an image. Hereinafter, an image forming unit including the TFT layer and the light-emitting unit is referred to as a display unit.

In some embodiments, the encapsulation layer that covers the display unit and prevents penetration of external moisture may be formed to have a thin film encapsulation structure in which an organic film and an inorganic film are alternately stacked. In some embodiments, the encapsulation layer has a thin film encapsulation structure in which a plurality of thin films are stacked. In some embodiments, the organic film applied to the interface portion is spaced apart from each of the plurality of display units. In some embodiments, the organic film is formed such that a portion of the organic film directly contacts the base substrate and a remaining portion of the organic film contacts the barrier layer while surrounding an edge portion of the barrier layer.

In one embodiment, the OLED display is flexible and uses the soft base substrate formed of polyimide. In some embodiments, the base substrate is formed on a carrier substrate formed of a glass material, and then the carrier substrate is separated.

In some embodiments, the barrier layer is formed on a surface of the base substrate opposite to the carrier substrate. In one embodiment, the barrier layer is patterned according to a size of each of the cell panels. For example, while the base substrate is formed over the entire surface of a mother panel, the barrier layer is formed according to a size of each of the cell panels, and thus a groove is formed at an interface portion between the barrier layers of the cell panels. Each of the cell panels can be cut along the groove.

In some embodiments, the method of manufacture further comprises cutting along the interface portion, wherein a groove is formed in the barrier layer, wherein at least a portion of the organic film is formed in the groove, and wherein the groove does not penetrate into the base substrate. In some embodiments, the TFT layer of each of the cell panels is formed, and the passivation layer which is an inorganic film and the planarization film which is an organic film are disposed on the TFT layer to cover the TFT layer. At the same time as the planarization film formed of, for example, polyimide or acryl is formed, the groove at the interface portion is covered with the organic film formed of, for example, polyimide or acryl. This is to prevent cracks from occurring by allowing the organic film to absorb an impact generated when each of the cell panels is cut along the groove at the interface portion. That is, if the entire barrier layer is entirely exposed without the organic film, an impact generated when each of the cell panels is cut along the groove at the interface portion is transferred to the barrier layer, thereby increasing the risk of cracks. However, in one embodiment, since the groove at the interface portion between the barrier layers is covered with the organic film and the organic film absorbs an impact that would otherwise be transferred to the barrier layer, each of the cell panels may be softly cut and cracks may be prevented from occurring in the barrier layer. In one embodiment, the organic film covering the groove at the interface portion and the planarization film are spaced apart from each other. For example, if the organic film and the planarization film are connected to each other as one layer, since external moisture may penetrate into the display unit through the planarization film and a portion where the organic film remains, the organic film and the planarization film are spaced apart from each other such that the organic film is spaced apart from the display unit.

In some embodiments, the display unit is formed by forming the light-emitting unit, and the encapsulation layer is disposed on the display unit to cover the display unit. As such, once the mother panel is completely manufactured, the carrier substrate that supports the base substrate is separated from the base substrate. In some embodiments, when a laser beam is emitted toward the carrier substrate, the carrier substrate is separated from the base substrate due to a difference in a thermal expansion coefficient between the carrier substrate and the base substrate.

In some embodiments, the mother panel is cut in units of the cell panels. In some embodiments, the mother panel is cut along an interface portion between the cell panels by using a cutter. In some embodiments, since the groove at the interface portion along which the mother panel is cut is covered with the organic film, the organic film absorbs an impact during the cutting. In some embodiments, cracks may be prevented from occurring in the barrier layer during the cutting.

In some embodiments, the methods reduce a defect rate of a product and stabilize its quality.

Another aspect is an OLED display including: a barrier layer that is formed on a base substrate; a display unit that is formed on the barrier layer; an encapsulation layer that is formed on the display unit; and an organic film that is applied to an edge portion of the barrier layer.

### Examples

The features of the present invention will be described more specifically with reference to Synthesis Examples and Examples given below. The materials, processes, procedures and the like shown below may be appropriately modified unless they deviate from the substance of the invention. Accordingly, the scope of the invention is not construed as being limited to the specific examples shown below. Hereinunder the light emission characteristics were evaluated using a source meter (available from Keithley Corporation, Keithley 2400), a semiconductor parameter analyzer (available from Agilent Technology Corporation, E5273A), a light power meter apparatus (available from Newport Corporation, 1930C), an optical spectroscope (available from Ocean Optics Corporation, USB 2000), a spectroradiometer (available from Topcon Corporation, SR-3) and a streak camera (available from Hamamatsu Photonics K.K., C4334).

### (Synthesis Example 1) Synthesis of Compound 53

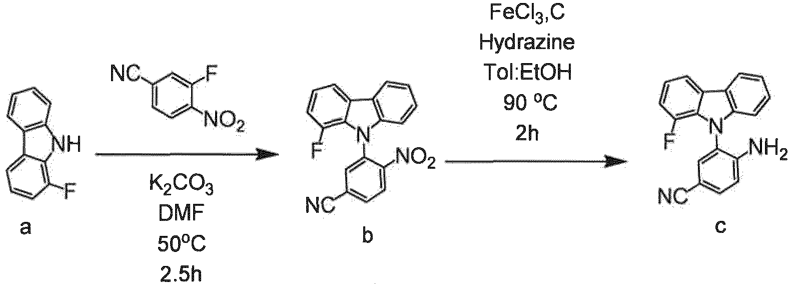

In a nitrogen stream atmosphere, 3-fluoro-4-benzonitrile (1.38 g, 10.0 mmol), and 1-fluorocarbazole (0.93 g, 5.0 mmol) were reacted in dimethylformamide (30 mL) in the presence of potassium carbonate at 50°C for 2.5 hours. Subsequently, this was restored to room temperature, and the reaction was stopped with water. The precipitated solid was filtered out, and the filtered solid was dissolved in ethyl acetate, dried with magnesium sulfate, and the solvent was evaporated away under reduced pressure. The resultant reaction mixture was purified by silica gel column chromatography (ethyl acetate/hexane = 4/1) and reprecipitation (ethyl acetate/hexane) to give a yellow solid of the compound b (1.67 g, yield 99%).
¹H NMR (400 MHz, CDCl₃, δ): 8.29 (d, J = 10 Hz, 1H), 8.11 (d, J = 10 Hz, 1H), 8.0 (s, 1H), 7.95 (d, J = 10 Hz, 1H), 7.89 (d, J = 10 Hz, 1H), 7.44 (t, J = 10 Hz, 1H), 7.35 (t, J = 10 Hz, 1H), 7.24 (m, 1H), 7.12 (t, J = 10.0 Hz, 1H), 7.07 (t, J = 10.0 Hz, 1H). MS (ASAP): 331.08 (M+H⁺). Calcd for C₅₀H₃₇N₅: 332.17.

In a nitrogen stream atmosphere, the compound b (1.38 g, 10.0 mmol), active carbon (0.18 g, 15.0 mmol) and iron chloride (0.135 g, 0.5 mmol) were dissolved in a mixed solvent of toluene (150 mL) and ethanol (150 mL), and hydrazine aqueous solution (4 ml, 125 mmol) was added thereto. The reaction mixture was reacted for 2 hours at 90°C, restored to room temperature and filtered through Celite. The solvent was evaporated away under reduced pressure, then subjected to extraction with water and ethyl acetate added thereto, and thereafter the organic layer was separated. This was dried with magnesium sulfate, and the solvent was evaporated away under reduced pressure. The filtered product was dissolved in ethyl acetate, dried with magnesium sulfate, and then the solvent was evaporated away under reduced pressure. The resultant reaction mixture was purified by silica gel column chromatography (ethyl acetate/hexane = 1/1) and reprecipitation (ethyl acetate/hexane) to give a white solid of the compound c (1.35 g, yield 90%).
¹H NMR (400 MHz, CDCl₃, δ): 8.21 (d, J = 10 Hz, 1H), 8.02 (m, 1H), 7.40 (t, J = 10 Hz, 1H), 7.26 (d, J = 10 Hz, 2H), 7.18 (m, 3H), 6.97 (d, J = 10 Hz, 1H), 7.93 (d, J = 10.0 Hz, 1H), 5.48 (s, 1H).
MS (ASAP): 301.10 (M+H⁺). Calcd for C₅₀H₃₇N₅: 302.17.

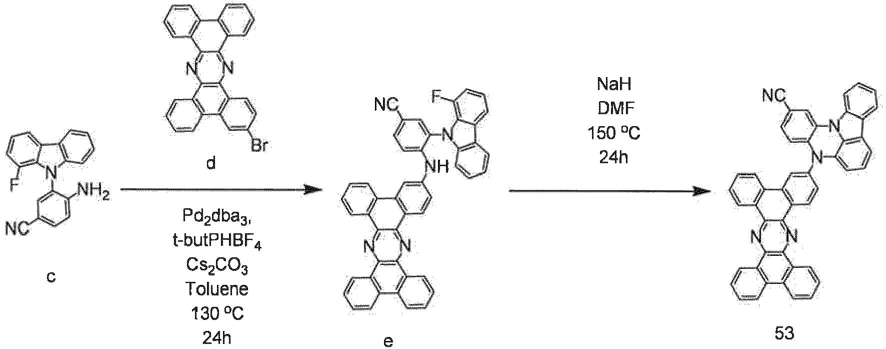

In a nitrogen stream atmosphere, the compound c (0.75 g, 2.5 mmol), the compound d (1.0 g, 2.17 mmol), tri(tert-butyl)phosphonium tetrafluoroborate (95 mg, 0.325 mmol), and cesium carbonate (1.41 g, 4.34 mmol) and trisdibenzylideneacetone bispalladium (100 mg, 0.168 mmol) were reacted in toluene (200 mL) at 130°C for 24 hours. The reaction solution was poured into water at room temperature to stop the reaction. Dichloromethane was added and the organic layer was separated by extraction, and thereafter this was dried with magnesium sulfate and the solvent was evaporated away under reduced pressure. The resultant residue was purified by column chromatography (toluene) to give a yellow solid of the compound e (0.7 g, 63%).
¹H NMR (400 MHz, CDCl₃, δ): 9.46 (m, 3H), 9.32 (d, J = 10 Hz, 1H), 8.87 (t, J = 10 Hz, 3H), 8.54 (m, 1H), 8.46 (s, 1H), 8.25 (d, J = 10 Hz, 1H), 8.10 (d, J = 10.0 Hz, 1H), 7.97 (s, 1H), 7.88 (m, 8H), 7.70 (d, J = 10.0 Hz, 1H), 7.46 (t, J = 10.0 Hz, 1H), 7.28 (m, 3H). MS (ASAP): 679.22(M+H⁺). Calcd for C₅₀H₃₇N₅: 680.35.

The compound e (0.7 g, 1.02 mmol) and sodium hydride (45 mg, 1.1 mmol) were reacted in dimethylformamide (50 mL) at 150°C for 24 hours. The reaction solution was restored to room temperature, and the solid formed by adding water thereto was filtered out, then washed, dissolved in toluene, dried with magnesium sulfate, and the solvent was condensed by being evaporated away under reduced pressure. The resultant residue was purified by column chromatography (toluene) and reprecipitation (chloroform/methanol) to give a yellow solid of the compound 53 (0.27 g, 57%).
MS (ASAP): 659.21(M+H⁺). Calcd for C₅₀H₃₇N₅: 660.09.

### (Synthesis Example 2) Synthesis of Compound 3054

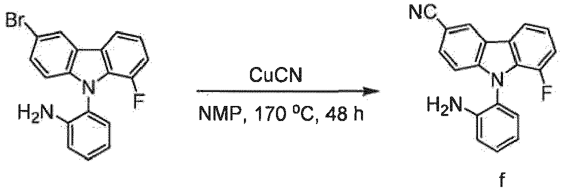

In a nitrogen stream atmosphere, N-methyl-2-pyrrolidone (NMP, 900 mL) was added to a mixture of 2-(6-bromo-1-fluoro-9H-carbazol-9-yl)aminobenzene (19.0 g, 53.5 mmol), and copper(I) cyanide (14.4 g, 161 mmol), and stirred at 170°C for 48 hours. The reaction solution was restored to room temperature, and water was added, and filtered. The crude product was purified by silica gel column chromatography (hexane/ethyl acetate = 4/1) to give a white solid of 12.0 g of the compound f(39.8 mmol, yield 74%). ¹H-NMR (400 MHz, DMSO-*d₆*): *δ* 8.87 (s, 1H), 8.21-8.17 (m, 1H), 7.33-7.28 (m, 2H), 7.25 (t, J = 8 Hz, 1H), 7.14 (d, J = 8 Hz, 1H), 6.93 (d, J = 8 Hz, 1H), 6.67 (t, J = 8 Hz, 1H), 5.05 (s, 1H).
MS (ASAP): 302.48(M+H⁺). Calcd for C₁₉H₁₂FN₃: 301.10.

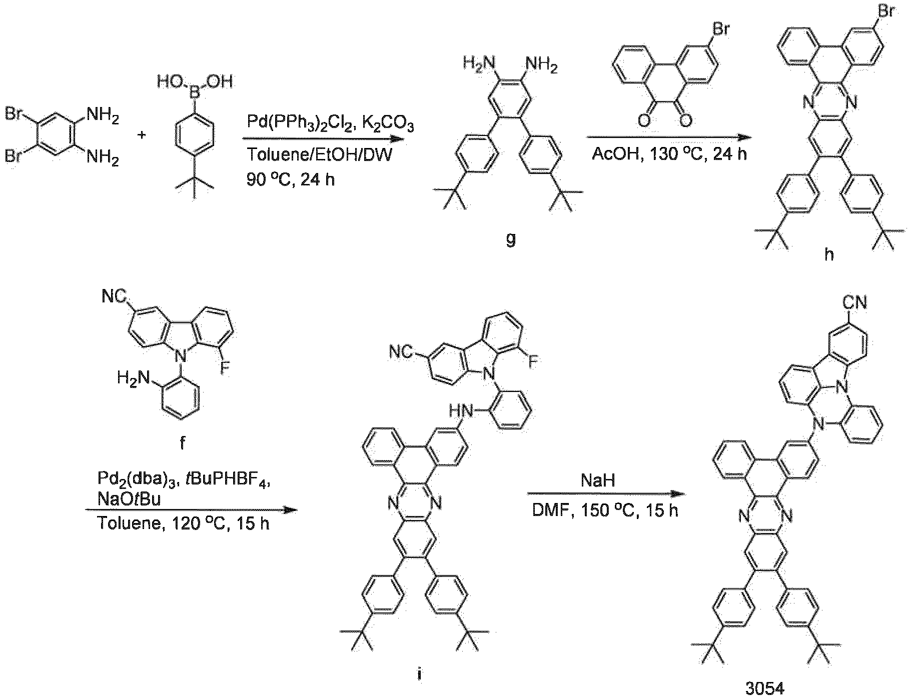

In a nitrogen stream atmosphere, potassium carbonate (8.3 g, 60 mmol), and bis(triphenylphosphine)palladium(II) dichloride (0.26 g, 0.4 mmol) were added to a solution of 4,5-dibromo-1,2-phenylenediamine (4.0 g, 15 mmol), and 4-tert-butylphenylboronic acid (6.7 g, 38 mmol) in toluene (130 mL), ethanol (10 mL) and water (20 mL), and stirred at 90°C for 24 hours. The reaction solution was restored to room temperature, extracted with chloroform, and dried with anhydrous magnesium sulfate. The solvent was evaporated away, and purified by silica gel column chromatography to give a white solid of 4.5 g of the compound g (12 mmol, yield 80%).
MS (ASAP): 373(M+H⁺). Calcd for C₂₆H₃₂N₂: 372.

In a nitrogen stream atmosphere, acetic acid (200 mL) was added to a mixture of the compound g (5.00 g, 13.4 mmol), 3-bromophenanthrene-9,10-dione (3.85 g, 13.4 mmol), and stirred at 130°C for 24 hours. The reaction solution was restored to room temperature, methanol was added and filtered. The crude product was washed with methanol and chloroform to give a white yellow solid of the compound h (7.5 g, 12 mmol, yield 90%).
MS (ASAP): 623 (M+H⁺). Calcd for C₄₀H₃₅BrN₂: 622.

In a nitrogen stream atmosphere, tri-tert-butylphosphonium tetrafluoroborate (0.56 g, 1.2 mmol), cesium carbonate (4.0 g, 12 mmol) and tris(dibenzylideneacetone)dipalladium(0) (0.56 g, 0.61 mmol) were added to a toluene (160 mL) solution of the compound h (3.8 g, 6.1 mmol), and the compound f(2.0 g, 6.7 mmol), and stirred at 120°C for 15 hours. The reaction solution was restored to room temperature, extracted with chloroform, and dried with anhydrous magnesium sulfate. The solvent was evaporated away, and purified by silica gel column chromatography (hexane/toluene = 3/7) to give a yellow solid of the compound i (1.0 g, 1.2 mmol, yield 19%).
¹H-NMR (400 MHz, CDCl₃): *δ* 9.390 (dd, J = 7.2 Hz, 3.2 Hz, 2H), 9.187 (d, J = 8.8 Hz, 1H), 8.418 (s, 1H), 8.335-8.300 (m, 3H), 8.030 (s, 1H), 7.903 (dd, J = 8.0 Hz, 1.2 Hz, 1H), 7.760-7.718 (m, 3H), 7.695 (dd, J = 8.0 Hz, 1.2 Hz, 1H), 7.557 (td, J = 8.8 Hz, 1.6 Hz, 1H), 7.450 (d, J = 7.6 Hz, 1H), 7.331-7.212 (m, 13 H), 5.705 (s, 1H), 1.334 (s, 18H). MS (ASAP): 844.30 (M+H⁺). Calcd for C₅₉H₄₆FN₅: 843.37.
ASAP MS spectrometry: C₅₉H₄₆FN₅: theoretical 843.37, calculated 844.30 [M+H⁺]

A mixture of NaH (28 mg, 0.69 mmol) and the compound i (0.53 g, 0.63 mmol) in 50 mL of N,N-dimethylformamide was stirred at 150°C for 15 hours. The mixture was restored to room temperature, quenched with water added, and the precipitated solid was filtered out and washed with methanol. The resultant solid was purified by silica gel column chromatography to give the compound 3054 (0.45 g, 0.55 mmol, yield 87%). ¹H-NMR (400 MHz, CDCl₃): *δ* 9.725 (d, J = 8.0 Hz, 1H), 9.484 (d, J = 8.8 Hz, 1H), 8.635 (s, 1H), 8.524 (d, J = 9.2 Hz, 1H), 8.418 (d, J = 3.2 Hz, 2H), 8.353 (s, 1H), 8.084 (d, J = 9.2 Hz, 1H), 7.833-7.755 (m, 4H), 7.722 (d, J = 8.4 Hz, 1H), 7.336 (d, J = 8.4 Hz, 4H), 7.275-7.259 (m, 5H), 6.902-6.746 (m, 3H), 6.304 (d, J = 8.4 Hz, 1H), 5.946 (d, J = 7.6 Hz, 1H), 1.352 (s, 18H).
MS (ASAP): 824.52 (M+H⁺). Calcd for C₅₉H₄₅N₅: 823.37.

### (Synthesis Example 3) Synthesis of Compound 17254

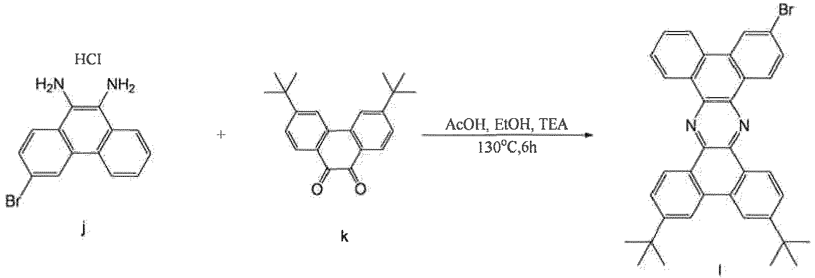

In a nitrogen stream atmosphere, the compound j (1.83 g, 5.74 mmol), the compound k (1.85 g, 5.74 mmol) and triethylamine (3.4 mL) in acetic acid (100 mL) and ethanol (25 mL) were stirred at 130°C for 6 hours. The mixture was restored to room temperature, and the precipitated solid was filtered out. The residue was washed with methanol to give the compound 1 (2.25 g, 3.95 mmol, yield 70%).
MS (ASAP): 570. Calcd for C₃₆H₃₁BrN₂: 570.

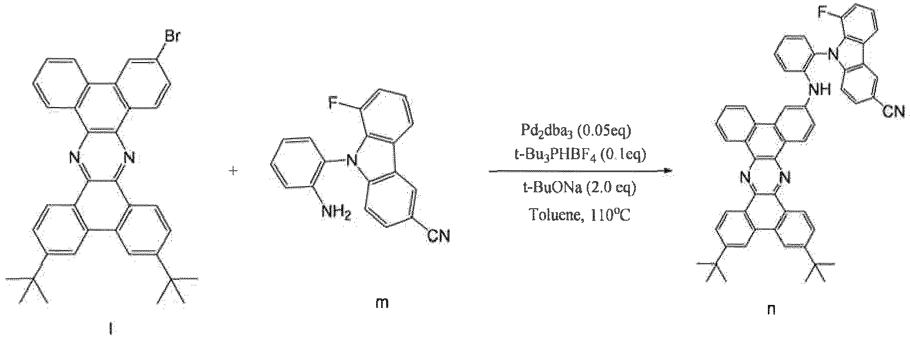

In a nitrogen stream atmosphere, Pd₂dba₃ (0.16 g, 0.17 mmol) was added to a toluene (300 mL) solution of the compound 1 (2.0 g, 3.4 mmol), the compound m (1.2 g, 3.8 mmol), tBu₃PHBF₄ (0.1 g, 0.34 mmol), and tBuONa (0.8 g, 6.8 mmol), and refluxed overnight under heat. The reaction solution was restored to room temperature, then quenched with water added, and extracted with dichloromethane. The solvent was evaporated away with an evaporator and purified by silica gel column chromatography to give the compound n (2.2 g, 2.8 mmol, yield 82%).
MS (ASAP): 791. Calcd for C₅₅H₄₂FN₅: 791.

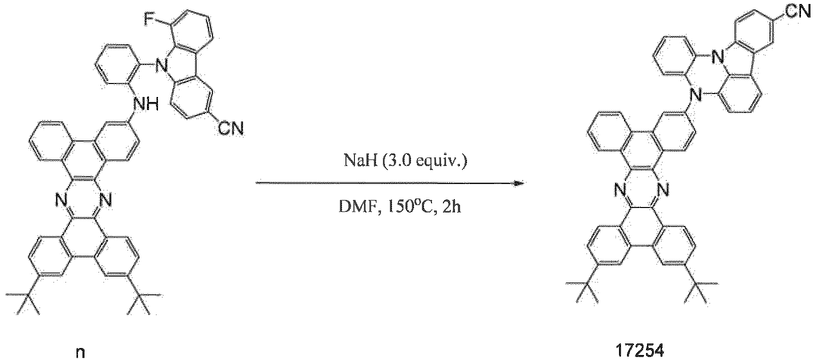

In a nitrogen stream atmosphere, NaH (0.3 g, 7.6 mmol) was added to an N,N-dimethylformamide (200 mL) solution of the compound n (2.0 g, 2.5 mmol), and stirred at 150°C for 2 hours. The reaction solution was restored to room temperature, and then quenched with water added, and the precipitated solid was filtered out. The residue was washed with methanol, and purified by silica gel column chromatography to give the compound 17254 (0.8 g, 1.03 mmol, yield 42%).
MS (ASAP): 771. Calcd for C₅₅H₄₁N₅: 771.

### (Example 1)

### Production and Evaluation of Thin Film

On a quartz substrate according to a vacuum evaporation method, the compound 53 was deposited under the condition of a vacuum degree of lower than 1×10⁻³ Pa to form a thin film of the compound 53 alone having a thickness of 100 nm, and this is a neat thin film of Example 1. Apart from this, on a quartz substrate according to a vacuum evaporation method, the compound 53 and mCBP were evaporated from different evaporation sources under the condition of a vacuum degree of lower than 1×10⁻³ Pa to form a thin film having a thickness of 100 nm in which the concentration of the compound 53 was 20% by weight, and this is a doped thin film of Example 1. The doped thin film of Example 1 provided emission of instantaneous fluorescence and delayed fluorescence later than the former at an emission peak wavelength of 594 nm. The lifetime τ₂ of the delayed fluorescence was 5.1 msec, from which excellent characteristics were confirmed.

Using the compound 3054, the compound 17254 and other compounds represented by the general formula (1) in place of the compound 53, neat thin films and doped thin films can be formed, and excellent characteristics thereof can be confirmed. Containing the compound represented by the general formula (1), high-concentration doped thin films attain high PLQY. Accordingly, organic light-emitting devices using the compound represented by the general formula (1) realize high light emission efficiency and good durability.

### (Example 2)

### Production and Evaluation of Organic Electroluminescent Device

On a glass substrate having, as formed thereon, an indium-tin oxide (ITO) anode film having a thickness of 100 nm, thin films were layered according to a vacuum evaporation method under a vacuum degree of 1 × 10⁻⁶ Pa. First, on ITO, HATCN was formed at a thickness of 10 nm, and then NPD was formed thereon at a thickness of 30 nm. Next, TrisPCz was formed on it at a thickness of 10 nm, and further thereon, formed Host1 at a thickness of 5 nm. Next, the compound 53 and Host1 were co-evaporated from different evaporation sources to form a light-emitting layer at a thickness of 30 nm. At that time, the concentration of the compound 53 was 35% by weight. Further on this, SF3TRZ was formed at a thickness of 10 nm, and further thereon SF3TRZ and Liq were co-evaporated from different evaporation sources to form a layer at a thickness of 30 nm. At that time, SF3TRZ/Liq (by weight) was 7/3. Further, Liq was formed at a thickness of 2 nm, and then aluminum (Al) was evaporated at a thickness of 100 nm to form a cathode. According to the process, an organic electroluminescent device of Example 1 was produced.

Using the compound 3054, the compound 17254 and other compounds represented by the general formula (1) in place of the compound 53, organic electroluminescent devices can be produced according to the same process, and the effects thereof can be confirmed.

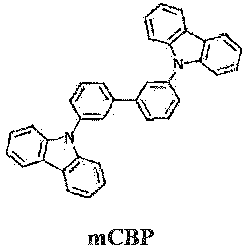

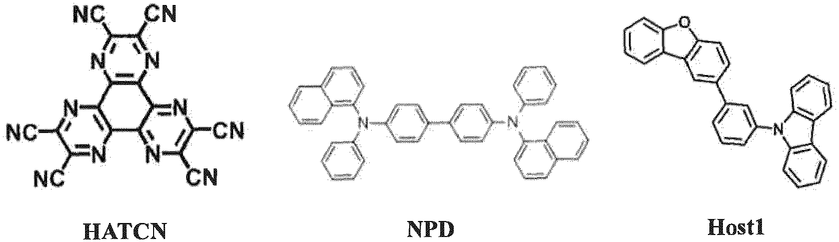

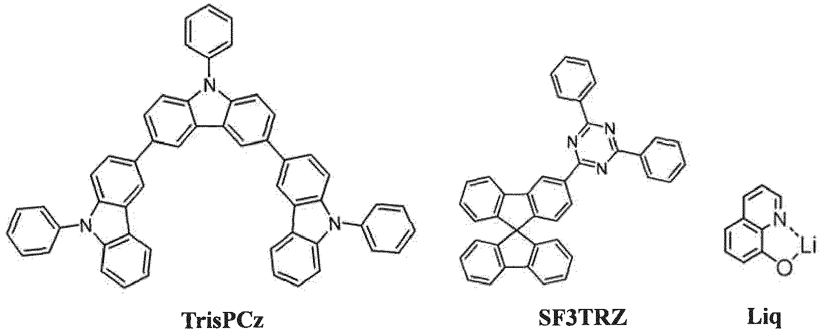

### Reference Signs List

- 1: Substrate
- 2: Anode
- 3: Hole Injection Layer
- 4: Hole Transporting Layer
- 5: Light-emitting Layer
- 6: Electron Transporting Layer
- 7: Cathode

## Claims

1. A compound represented by the following general formula (1). 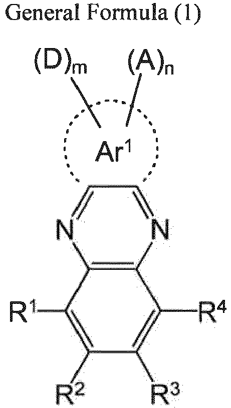 wherein Ar¹ represents a cyclic structure, and represents a benzene ring, a naphthalene ring, an anthracene ring, or a phenanthrene ring, D represents a group represented by the following general formula (2), A represents one or a combination of two or more groups selected from the group consisting of a cyano group, a phenyl group, a pyrimidyl group, a triazyl group and an alkyl group except for a substituted alkyl group, m represents 1 or 2, n represents 0, 1 or 2, when m is 2, two D's can be the same or different, when n is 2, two A's can be the same or different, R¹ to R⁴ each independently represent a hydrogen atom, a deuterium atom, or one or a combination of two or more groups selected from the group consisting of an alkyl group, an aryl group, a heteroaryl group and a cyano group, R¹ and R², and R³ and R⁴ each can bond to each other to form a cyclic structure selected from the group consisting of a benzene ring, a naphthalene ring and a pyridine ring, and the formed cyclic structure can be substituted with one or a combination of two or more groups selected from the group consisting of an alkyl group, an aryl group, a heteroaryl group and a cyano group; 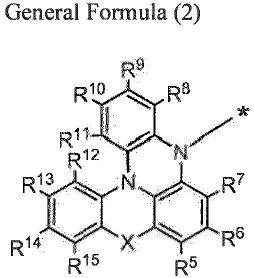 wherein R⁵ to R¹⁵ each independently represent a hydrogen atom, a deuterium atom or a substituent, R⁵ and R⁶, R⁶ and R⁷, R⁸ and R⁹, R⁹ and R¹⁰, R¹⁰ and R¹¹, R¹¹ and R¹², R¹² and R¹³, R¹³ and R¹⁴, and R¹⁴ and R¹⁵ each can bond to each other to form a cyclic structure, X represents a single bond, an oxygen atom or a sulfur atom, * indicates a bonding position.

2. The compound according to claim 1, represented by the following general formula (3). 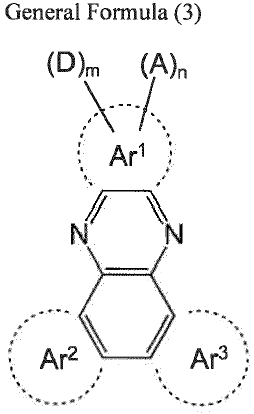 wherein Ar¹ represents a cyclic structure, and represents a benzene ring, a naphthalene ring, an anthracene ring, or a phenanthrene ring, D represents a group represented by the above-mentioned general formula (2), A represents one or a combination of two or more groups selected from the group consisting of a cyano group, a phenyl group, a pyrimidyl group, a triazyl group and an alkyl group except for a substituted alkyl group, m represents 1 or 2, n represents 0, 1 or 2, when m is 2, two D's can be the same or different, when n is 2, two A's can be the same or different, Ar² and Ar³ can each independently form a cyclic structure selected from the group consisting of a benzene ring, a naphthalene ring and a pyridine ring, and the formed cyclic structure can be substituted with one or a combination of two or more groups selected from the group consisting of an alkyl group, an aryl group, a heteroaryl group and a cyano group.

3. The compound according to claim 1, having a skeleton of any of the following: 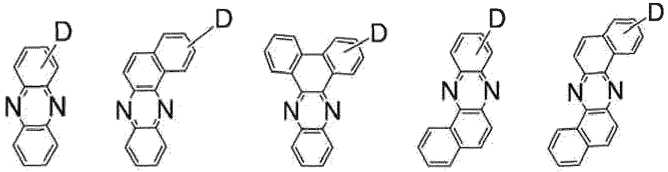 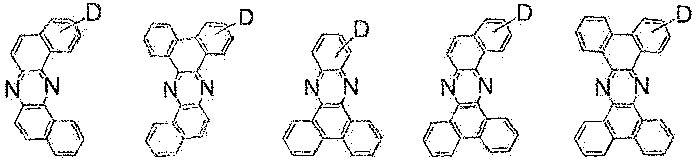 wherein the skeletons each can have a substituent within the range of the general formula (1), but any further ring is not condensed with the skeletons.

4. The compound according to claim 1, represented by any of the following general formulae (4a) to (4f). 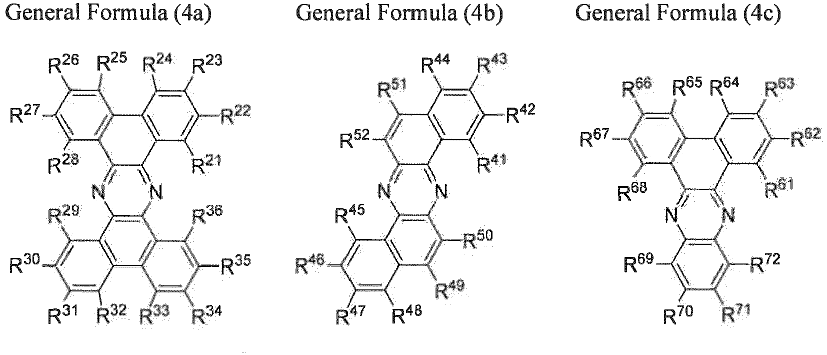 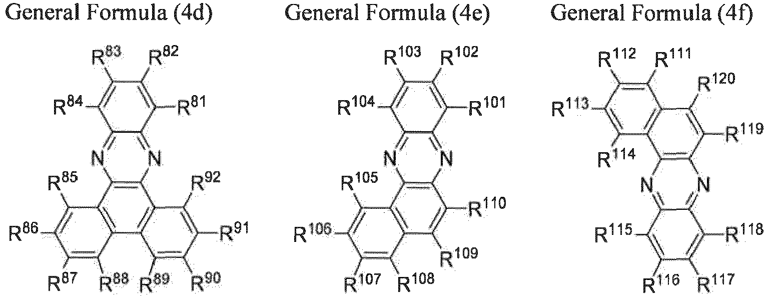 wherein R²¹ to R²⁸, R⁴¹ to R⁴⁴, R⁵¹, R⁵², R⁶¹ to R⁶⁸, R⁸¹ to R⁸⁴, R¹⁰¹ to R¹⁰⁴, R¹¹¹ to R¹¹⁴, R¹¹⁹, and R¹²⁰ each independently represent a hydrogen atom, a deuterium atom, D or A, provided that 1 or 2 of R²¹ to R²⁸ are D, and 0 to 2 are A; 1 or 2 of R⁴¹ to R⁴⁴, R⁵¹ and R⁵² are D, and 0 to 2 are A; 1 or 2 of R⁶¹ to R⁶⁸ are D, and 0 to 2 are A; 1 or 2 of R⁸¹ to R⁸⁴ are D, and 0 to 2 are A; 1 or 2 of R¹⁰¹ to R¹⁰⁴ are D, and 0 to 2 are A; 1 or 2 of R¹¹¹ to R¹¹⁴, R¹¹⁹ and R¹²⁰ are D, and 0 to 2 are A, and R²⁹ to R³⁶, R⁴⁵ to R⁵⁰, R⁶⁹ to R⁷², R⁸⁵ to R⁹², R¹⁰⁵ to R¹¹⁰, and R¹¹⁵ to R¹¹⁸ each independently represent a hydrogen atom, a deuterium atom, or one or a combination of two or more groups selected from the group consisting of an alkyl group, an aryl group and a cyano group.

5. The compound according to any one of claims 1 to 4, wherein n is 0.

6. A light-emitting material comprising the compound of any one of claims 1 to 5.

7. A film comprising the compound of any one of claims 1 to 5.

8. An organic semiconductor device comprising the compound of any one of claims 1 to 5.

9. An organic light-emitting device comprising the compound of any one of claims 1 to 5.

10. The organic light-emitting device according to claim 9, wherein the device has a layer containing the compound and the layer also contains a host material.

11. The organic light-emitting device according to claim 10, wherein the layer containing the compound also contains a delayed fluorescent material in addition to the host material, and the lowest excited singlet energy of the delayed fluorescent material is lower than that of the host material and higher than that of the compound.

12. The organic light-emitting device according to claim 9, wherein the device has a layer containing the compound, and the layer also contains a light-emitting material having a structure different from that of the compound.

13. The organic light-emitting device according to any one of claims 9 to 11, wherein, among the materials contained in the device, the amount of light emission from the compound is the maximum.

14. The organic light-emitting device according to claim 12, wherein the amount of light emission from the light-emitting material is larger than the amount of light emission from the compound.

15. The organic light-emitting device according to any one of claims 9 to 14, which emits delayed fluorescence.
